# EUROPEAN PATENT SPECIFICATION

(11) **EP 1 362 894 B1**
(45) Date of publication and mention of the grant of the patent: **19.12.2007**
(21) Application number: 03003488.8
(22) Date of filing: 14.02.2003
(51) Int. Cl.: C09B 23/00, C12Q 1/68

(54) **Method for optical measurement of multi-stranded nucleic acid using cyanine dyes**
Verfahren zur optischen Bestimmung von mehrsträngigen Nucleinsäuren mit Hilfe von Cyaninfarbstoffen
Procede de mesure optique de acides nucleiques multi-filamentaires utilisant colorants cyaniniques

(30) Priority: 14.02.2002 JP 2002036473; 14.02.2002 JP 2002036474
(43) Date of publication of application: 19.11.2003
(73) Proprietor: FUJIFILM Corporation, Minato-ku Tokyo (JP)
(72) Inventor: Nakamura, Kouki, Fuji Photo Film Co., Ltd., Minami-ashigara-shi, Kanagawa 250-0193 (JP); Takeuchi, Kazuya, Fuji Photo Film Co., Ltd., Minami-ashigara-shi, Kanagawa 250-0193 (JP)
(74) Representative: HOFFMANN EITLE

(56) References cited:
- WO-A-00/66664
- GB-A- 870 753
- US-A- 5 321 130
- US-A- 5 410 030
- US-A- 5 656 449
- US-A- 5 658 751
- FICKEN G E ET AL: "diazaindene and their quaternary salts. Part I" JOURNAL OF THE CHEMICAL SOCIETY, CHEMICAL SOCIETY. LETCHWORTH, GB, 1959, pages 3202-3212, XP002145341 ISSN: 0368-1769

## Description

### Field of Invention

The present invention relates to a novel method for optical measurement of a multi-stranded nucleic acid. More specifically, the present invention relates to a technique for optical measurement of a hybrid multi-stranded nucleic acid utilizing a fluorescence measurement method, which is useful in the field of gene analysis. The present invention also relates to a group of novel class of compounds, classified as dye bases, having an electrically neutral chromophore.

### Related Art

Applications of dyes for methods for detection of nucleic acids is one of the fields in which researches are most actively conducted in recent years. Wide variety of dyes have been developed for various applications, including ethidium bromide used for a purpose of analyzing a position at which a nucleic acid exists during separation of nucleic acids by electrophoresis. Examples are described in detail in Handbook of Fluorescent Probes and Research Chemicals, 8th Edition, Molecular Probes, Inc. (CD-ROM pressed by Molecular Probes, Inc., 2001). For a purposes of obtaining genetic information, or obtaining information of gene expression in pathological tissues and the like, a method has been developed in which presence or absence or an amount of a nucleic acid of interest is detected by suitably treating a nucleic acid extracted from a living body for labeling with a fluorescent dye and then hybridizing the resulting nucleic acid with a probe nucleic acid.

As means for obtaining information of a large number of genes or gene expression at the same time, utilizing the aforementioned method, detection techniques referred to as DNA chips or DNA arrays (generically referred to as "DNA array" hereinafter in the specification) have been developed and noticeably focused. In these techniques, a large number of nucleic acids, of which partial sequences or total sequences are known and have different sequences, are immobilized on a surface of a solid phase carrier in arrayed dots, and the positions of the dots on the surface and the sequences of the nucleic acids are linked (also called "addressing"). A nucleic acid having an unknown sequence (the nucleic acid may consist of one or more kinds of nucleic acids) is uniformly spread on the surface and exposed to a hybridization condition. If a nucleic acid having a sequence complementary to the nucleic acid of the unknown sequence exists on the solid phase carrier surface, the both form a hybrid and remain on the solid phase surface even after washing.

At this time, if the hybrid can be detected by a certain method, it becomes possible to determine whether or not the sequence to be detected exists among the unknown nucleic acids or to perform quantification or identification of the sequence by referring to the sequence corresponding to the position of the hybrid remaining on the solid phase surface. A probability is extremely low that different genes of an organism having 20 or more nucleotides are homologous, and therefore, when suitable sequences of 20 or more nucleotides are selected as the aforementioned nucleic acid sequences immobilized on the solid phase surface, it becomes possible to establish one for one relationship between the immobilized nucleic acids having known sequences and genes to be detected. Most of the methods known so far, referred to as DNA arrays, are based on the aforementioned principle. Based on the aforementioned principle, only a single operation successfully give information as to presence or absence and quantity of a large number of (i.e., the number of kinds of nucleic acids having sequences corresponding to the genes immobilized on the solid phase surface) genes or expressed genes. As described above, the DNA array techniques are supported by the addressing technique and the hybridization technique for proving correlative homology between nucleic acids.

On the basis of the above techniques, genic information on each organism can be obtained by digesting a genomic gene into fragments of suitable lengths and analyzing the fragments by using a DNA array. Moreover, amplification can be performed for a sequence of a specific region by using a technique such as PCR to obtain genomic information on each organism such as monobasic polymorphism, and it is also possible to know a state of gene expression in each tissue in an individual, or a level of gene expression in each time scale of development stage or growth phase. This method is generally called gene expression analysis, and utilized for obtaining information of kinds or quantities of mRNAs transcribed from a genomic DNA or for comparing such information.

Targets of the analysis using a DNA array are mostly genomic DNA or mRNA. As described above, the DNA array technique needs to detect a hybrid multi-stranded nucleic acid formed with a complementary nucleic acid. However, nucleic acids can be isolated from a living body only in an extremely small amount, and nucleic acids themselves are not suitably detectable chemical species. For these reasons, methods most widely used at present for the detection are those called "nucleic acid labeling methods". As for these methods, specific methods are described in a large number of published books and literatures, and many analogous methods are available. The principle of the labeling resides in that a labeled nucleotide triphosphate is used as a raw material for replication in preparation of a replicate of a nucleic acid to be detected, so that the material is incorporated and label a nucleic acid polymer (oligomer) formed. As for types of the label, direct labeling methods utilizing RI (radioisotope) or fluorescent dye and indirect labeling methods such as those utilizing biotin or digoxigenin are known. After a suitable treatment which enables detection of, for example, radiation for RI labeling, fluorescence for fluorescent dye labeling, fluorescence or chemiluminescence for biotin or digoxigenin, the detection is performed. A detection system is designed so as to achieve each detection with high sensitivity.

However, as for the detection of a hybrid based on these labeling methods, two major problems are pointed out. One problem is that a quantitative relationship in an original nucleic acid mixture may not possibly be reflected accurately in a replicate in the methods of replication of nucleic acids used in the labeling, e.g., PCR (polymerase chain reaction) or RT (reverse transcription) reaction. Another problem is that, except for the RI labeling method, a replicated nucleic acid of a gene binds to a labeling compound to become a different chemical substance which, in a strict sense, is not a replicate. It is considered that specific examples concerning the above problems include reduction of hybridization efficiency (quantitative degradation) and erroneous recognition (qualitative degradation of information) due to a lowering of recognition ability of a probe nucleic acid and a sample nucleic acid, which may possibly become fundamental problems of the labeling methods.

As described above, the labeling methods have essential problems. For this reason, as a method that can solve the aforementioned problems, a method has been proposed in which a non-labeled hybrid double-stranded nucleic acid is detected without a labeling process. Japanese Patent Laid-open Publication (Kokai) No. (Hei)5-199898 discloses a method for detecting a formation of a hybrid, in which the hybrid is formed on an electrode and the formation of the hybrid is detected by using an intercalater modified with an electrochemically active chemical species. According to this method, a double-stranded hybrid formed by hybridization in the DNA array method can be detected without labeling. Therefore, it is believed that the method can solve the problems of the labeling methods. In fact, this method has several advantages, for example, an apparatus for the detection can be made smaller. However, this method requires immobilization of a probe nucleic acid on the electrode surface to electrochemically perform the detection. Therefore, for a simultaneous detection of a large number of hybrids, it is required that a large number of electrodes are formed on the surface and each probe nucleic acid is immobilized on each of the electrodes. Therefore, for use as a detection method, this method also has many problems to be solved. Moreover, as for a signal/background ratio, discrimination of a hybrid and non-hybrid (single-stranded probe) is not fully satisfactory, and thus a quantitative detection is difficult.

As another method, a method may also be proposed in which a fluorescent dye is used of which fluorescence intensity is increased by an interaction with a multi-stranded nucleic acid. It is considered that if a dye exists of which fluorescence intensity remains weak during an interaction with a probe nucleic acid (single-stranded), whilst the intensity markedly increases by an interaction with a multi-stranded nucleic acid formed by hybridization, a hybrid double-stranded nucleic acid can be detected without labeling. It is reported that a dye marketed by Molecular Probes, Inc. with a trade name of PicoGreen enables quantification of double-stranded nucleic acid. However, the dye emits non-negligible fluorescence even in the presence of a single-stranded nucleic acid, and accordingly, a high signal/background ratio cannot be expected.

In a detection method for a trace amount component, a probe is generally used in an excess amount relative to an object to be detected. For this reason, a method is required which can strictly discriminate a single-stranded nucleic acid, existing in an excess amount, and a double-stranded nucleic acid formed by hybridization. However, such a method has not been known so far.

Dyes as means for detecting nucleic acid have been studied since relatively old days. Ethidium bromide, as a fluorescent dye for staining a nucleic acid to determine a position where the nucleic acid exists in nucleic acid separation utilizing electrophoresis, is an example of such dyes. With the aforementioned innovation of techniques for handling nucleic acids, importance of fluorescent dyes for nucleic acid detection has become increasingly higher, and wide variety of dyes have been developed. Examples are detailed in Handbook of Fluorescent Probes and Research Chemicals, 8th Edition (CD-ROM, pressed by Molecular Probes, Inc., 2001).

As for functions required for dyes for nucleic acid detection, the dyes are required to have a moderate interaction with a nucleic acid, weak fluorescent property in the absence of a nucleic acid, whilst strong fluorescent property in the presence of a nucleic acid. In addition, strongly desired are high water-solubility and expansion of variations of excitation wavelength and emission wavelength so as to cover various excitation wavelengths and emission wavelengths.

Dyes such as asymmetric cyanine dyes having a cationic chromophore disclosed in U.S. Patent No. 5,658,751 have been developed which is used not only for simple detection of a nucleic acid but for quantification of a double-stranded nucleic acid even when a single-stranded nucleic acid coexists. U.S. Patent No. 5,656,449 discloses asymmetric cyanine dyes having an electrically neutral chromophore, and the patent document discloses a characteristic feature of the dye applicable to fluorescent detection of a nucleic acid even in viable cells. Also for these purposes, novel fluorescent dyes are desired which provide more precise quantitative results and variations of excitation wavelength and emission wavelength, and enable more sensitive detection of nucleic acids. Asymmetric cyanine dyes and their uses are also described in WO 00/66664.

### Disclosure of Invention

An object of the present invention is to provide a compound which emits a strong signal by an interaction with a multi-stranded nucleic acid under discrimination of a single-stranded nucleic acid, and further to provide a method for detecting a multi-stranded nucleic acid by utilizing such a compound.

Another object of the present invention is to provide a novel highly fluorescent dye for detection of a nucleic acid, which provides a wide variety of excitation wavelengths and emission wavelengths and thus can be used for a wide variety of purposes. A still further object of the present invention is to provide a fluorescent dye having higher selectivity in a method for detecting a double-stranded nucleic acid under discrimination from a single-stranded nucleic acid.

The inventors of the present invention conducted various researches to achieve the foregoing objects. As a result, they concluded that, for strict discrimination of a single-stranded nucleic acid and a multi-stranded nucleic acid, it was extremely difficult to design a dye, so as to bind only to a multi-stranded nucleic acid and emit a detectable signal, simply by controlling affinity for a single-stranded nucleic acid and for a multi-stranded nucleic acid. Accordingly, based on a concept completely different from the conventional methods, the inventors of the present invention conceived the following method. That is, when a compound is used, which is substantially colorless under a condition where only a single-stranded nucleic acid exists (condition where no multi-stranded nucleic acid exists), but changes into a colored state and emits fluorescence when a multi-stranded nucleic acid exists, no fluorescence is observed due to no light absorption with a single-stranded nucleic acid, whilst light absorption occurs in the presence of a multi-stranded nucleic acid to enable detection of fluorescence. The inventors of the present invention studied the aforementioned method and verified that the above method is an extremely excellent method for measurement of a multi-stranded nucleic acid. The present invention was achieved on the basis of the findings.

The present invention thus provides a method for optical measurement of a multi-stranded nucleic acid which comprises the step of bringing a compound into contact with a multi-stranded nucleic acid wherein said compound is capable of interacting with the multi-stranded nucleic acid, wherein the compound has the following properties:
(a) the compound can exist in a substantially colorless and non-fluorescent state under at least one condition in an aqueous solution in the absence of the multi-stranded nucleic acid, and
(b) when the multi-stranded nucleic acid is allowed to exist in the condition defined in the above (a), the compound changes to a substantially colored state based on an interaction with the multi-stranded nucleic acid and substantially expresses fluorescent property based on said interaction and wherein the compound is as specified in appended claim 1.

The present invention also provides a method for optical measurement of a multi-stranded nucleic acid which comprises the step of bringing a compound into contact with a multi-stranded nucleic acid wherein the compound is capable of interacting with the multi-stranded nucleic acid, wherein the compound has the following properties:
(c) the compound is in a non-protonated state and is substantially non-fluorescent in an aqueous solution in the absence of the multi-stranded nucleic acid,
(d) the compound can exist in a substantially colorless and substantially non-fluorescent state in a protonated state in an aqueous solution under at least one condition in the absence of the multi-stranded nucleic acid, and
(e) when the multi-stranded nucleic acid is allowed to exist in the condition defined in the above (d), the compound changes to a substantially colored state based on an interaction with the multi-stranded nucleic acid and substantially expresses fluorescent property based on said interaction , and wherein the compound is as specified in appended claim 2.

Thus, according to the present invention, there are provided the aforementioned methods, wherein the compound capable of interacting with the multi-stranded nucleic acid is represented by the following formula (V). Comparative compounds are represented by the following formulae (VI), (VII) or (VIII): 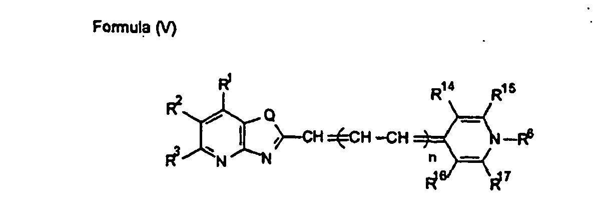 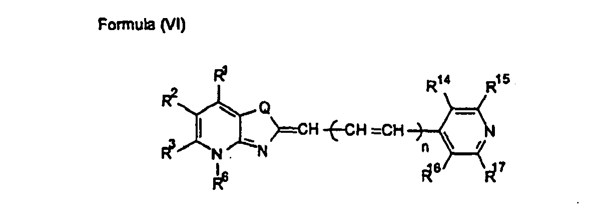 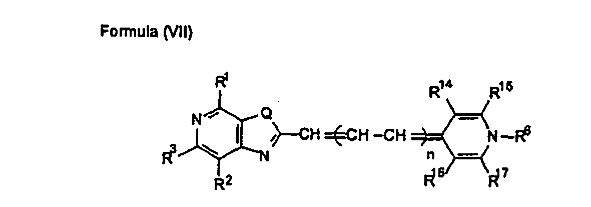 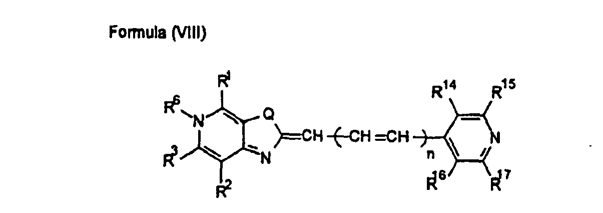 wherein the substituent definitions are as specified in appended claim 1.

The present invention further provides a method for optical measurement of a multi-stranded nucleic acid comprising:
(1) the step of bringing Compound (B) of the following general formula into contact with a multi-stranded nucleic acid, and
(2) the step of measuring fluorescence of Compound (C) produced by an interaction of Compound (B) and the multi-stranded nucleic acid: 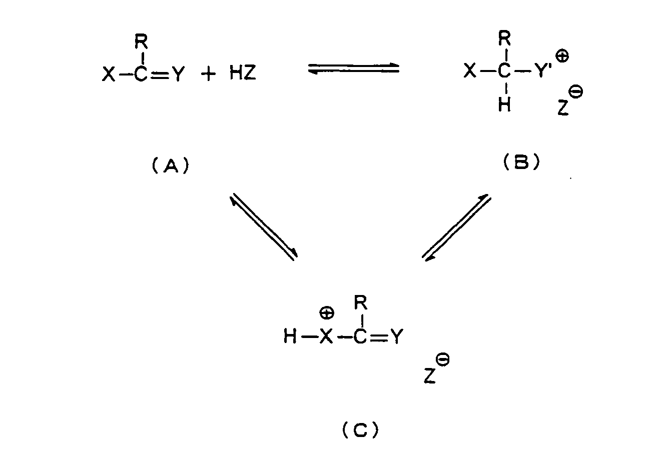
wherein the two way arrows represent chemical equilibrium;
Compound (A) represents a substantially non-fluorescent compound,
Compound (B) represents a substantially non-fluorescent compound that does not have an absorption maximum in the range of from 400 to 1000 nm under at least one condition;
Compound (C) represents a compound which is produced by an interaction of Compound (B) and the multi-stranded nucleic acid under the condition mentioned above, and has an absorption maximum in the range of from 400 to 1000 nm and substantially expresses fluorescent property based on the interaction;
X represents a group of atoms required to form a dye molecule by bonding to -C(R)=Y; Y represents a group of atoms required to form a dye molecule by bonding to X-C(R)=; R represents hydrogen atom or a substituent and may bind to X and/or Y to form a ring; Y' represents a residue remaining as a result of occurrence of an electron transfer so that the double bond of C=Y in Compound (A) is protonated; and Z represents a conjugate base of acid H, Z and may bind to X, Y (or Y') or R,
wherein Compound (A) is as specified in appended claim 1.

According to preferred embodiments of these inventions, there are provided the aforementioned methods, which comprise the step of allowing the interaction with the multi-stranded nucleic acid in a state of solution; and the aforementioned methods, which comprise the step of allowing the interaction with the multi-stranded nucleic acid immobilized on a solid phase carrier.

From a still further aspect, the present invention provides a reagent for optical measurement of a multi-stranded nucleic acid which comprises a compound capable of interacting with a multi-stranded nucleic acid, wherein the compound has the following properties:
(a) the compound can exist in a substantially colorless and non-fluorescent state under at least one condition in an aqueous solution in the absence of the multi-stranded nucleic acid, and
(b) when the multi-stranded nucleic acid is allowed to exist in the condition defined in the above (a), the compound changes to a substantially colored state based on an interaction with the multi-stranded nucleic acid and substantially expresses fluorescent property based on the interaction, and wherein the compound is as specified in appended claim 1.

The present invention also provides a reagent for optical measurement of a multi-stranded nucleic acid which comprises a compound capable of interacting with a multi-stranded nucleic acid, wherein the compound has the following properties:
(c) the compound is in a non-protonated state and is substantially non-fluorescent in an aqueous solution in the absence of the multi-stranded nucleic acid,
(d) the compound can exist in a substantially colorless and substantially non-fluorescent state in a protonated state in an aqueous solution under at least one condition in the absence of the multi-stranded nucleic acid, and
(e) when the multi-stranded nucleic acid is allowed to exist in the condition defined in the above (d), the compound changes to a substantially colored state based on an interaction with the multi-stranded nucleic acid and substantially expresses fluorescent property based on said interaction , and wherein the compound is as specified in appended claim 1.

The present invention further provides a novel asymmetric dye compound as specified in appended claims 1 and 6.

These dyes are characterized to have a neutral chromophore and a class of compounds generally classified as dye bases. The aforementioned dye compounds found by the inventors of the present invention are characterized to have a neutral chromophore, and moreover, a structure containing a heterocyclic ring in which 5-membered and 6-membered rings are condensed (i.e., an azole ring and a pyridine ring are condensed). Dyes having such a condensed ring system are highly stable, and have characteristic features for variety of purposes. As dyes having a cationic chromophore in which an azole ring and a pyridine ring are condensed, for example, the dyes disclosed in Japanese Patent Laid-open Publication No. 2001-270957, the dyes disclosed in Japanese Patent Laid-open Publication No. 2001-163895 and the like are known. However, the dye structure having the neutral chromophore according to the present has not been known.

### Preferred Embodiments of the Invention

In one embodiment, the method of the present invention is used for optical measurement of a multi- stranded nucleic acid, and comprises the step of bringing a compound into contact with a multi-stranded nucleic acid wherein the compound is capable of interacting with the multi-stranded nucleic acid, characterized in that the compound has the following properties:
(a) the compound can exist in a substantially colorless and non-fluorescent state under at least one condition in an aqueous solution in the absence of the multi-stranded nucleic acid, and
(b) when the multi-stranded nucleic acid is allowed to exist in the condition defined in the above (a), the compound changes to a substantially colored state based on an interaction with the multi-stranded nucleic acid, and substantially expresses fluorescent property based on said interaction.

In the specification, the term "colorless" means that the compound has a maximum molecular extinction coefficient of zero to less than 8000, preferably zero to less than 5000, most preferably zero to less than 3000, in the range of from 400 to 1000 nm (numerical ranges indicated by "from -- to" in the specification are those including an upper limit and a lower limit, unless otherwise specifically mentioned). Further, the term "colored" means that the compound has a maximum molecular extinction coefficient of 20000 or more, preferably 30000 or more, most preferably 40000 or more, in the range of from 400 to 1000 nm. A ratio of the change in the molecular extinction coefficient from the colorless state to the colored state is 5 times or more, preferably 7 times or more, most preferably 10 times or more.

In the specification, the wording "capable of interacting" means that the aforementioned compound and a multi-stranded nucleic acid have affinity for each other, and they successfully yield a kind of physicochemical interaction such as a transfer of energy via a reversible binding. However, the wording should not be construed in any limitative way, and be construed in its broadest sense. The wording "can exist in a substantially colorless and non-fluorescent state in an aqueous solution under at least one condition" means that the compound has the above defined property in a state wherein conditions in a solution such as a pH and a salt concentration are suitably chosen, and the compound may have any properties under a condition other than the above-defined condition. Those skilled in the art can easily determine whether or not a compound has the desired properties by appropriately choosing a pH and a salt concentration of a solution according to ordinary methods.

In the specification, the term "multi-stranded nucleic acid" refers to a state of nucleic acids under association by an interaction resulting from complementary sequences (a process of formation of the multi-stranded nucleic acid by the interaction may sometimes be called "hybridization"). It is known that a multi-stranded nucleic acid may exist in a double-stranded, triple-stranded, quadruple-stranded state or the like. The term "multi-stranded nucleic acid" in the specification encompasses these multiplex strands. As nucleic acids, DNA and RNA, as well as many chemically modified nucleic acids are known, and nucleic acid analogues called PNA, which have a polypeptide chain as a backbone, are also known. The multi-stranded nucleic acid in the specification encompasses all of these substances. Nucleic acids more preferably used in the present invention are DNA, RNA and chemically modified substances thereof. Double-stranded nucleic acids are preferred among double-stranded, triple-stranded and quadruple-stranded nucleic acids. A multi-stranded nucleic acid produced by hybridization of a probe nucleic acid and a sample nucleic acid is referred to as "hybrid multi-stranded nucleic acid" in the specification.

As a compound having the aforementioned characteristics used for the method of the present invention, for example, dye compounds can be used which become substantially colorless when they are protonated in a solution. For example, some of cyanine dyes are protonated and become colorless in an aqueous solution, and many of benzimidacarbocyanine dyes have the aforementioned property. As for the above phenomenon, it is considered that a dye conjugated system is cleaved as a result of protonation on a carbon atom in a methine chain of a cyanine dye, thereby the compound becomes colorless. In the above phenomenon, it is also considered that the cyanine dye is in equilibrium between an intrinsic colored state and a protonated colorless state depending on a proton concentration in the system. Compounds used for the method of another embodiment of the present invention are those capable of interacting with the multi-stranded nucleic acid and having the following properties:
(c) in the absence of the multi-stranded nucleic acid, the compound is in a non-protonated state and is substantially non-fluorescent in an aqueous solution,
(d) in the absence of the multi-stranded nucleic acid, the compound in a protonated state can exist in a substantially colorless and substantially non-fluorescent state in an aqueous solution under at least one condition, and
(e) when the multi-stranded nucleic acid is allowed to exist in the condition defined in the above (d), the compound changes to a substantially colored state based on an interaction with the multi-stranded nucleic acid and substantially expresses fluorescent property based on said interaction.

Although it is not intended to be bound by any specific theory, the embodiments of the present invention are based on the principle in that, when the dye compound, having become colorless due to protonation, interacts with a multi-stranded nucleic acid, an equilibrium is shifted toward an intrinsic dye state (recovery of a dye conjugated system) by an interaction energy received from the multi-stranded nucleic acid, thereby the dye is colored, and in the absence of the multi-stranded nucleic acid, the substantially colorless state is maintained. Even if a single-stranded nucleic acid exists in the reaction system, the substantially colorless state is maintained, because an energy of the interaction between the dye and a single-strand nucleic acid is small. Whilst, the energy of the interaction between the dye and a multi-stranded nucleic acid is sufficiently large, and therefore the dye having made colorless will change into a colored dye based on the interaction with the multi-stranded nucleic acid. As a result, light absorption occurs, and thus it will be possible to have the dye express fluorescent property.

As a preferred embodiment of the present invention, an example includes a method comprising:
(1) the step of bringing Compound (B) in the following formula into contact with a multi-stranded nucleic acid, and
(2) the step of measuring fluorescence of Compound (C) produced by an interaction of Compound (B) and the multi-stranded nucleic acid: 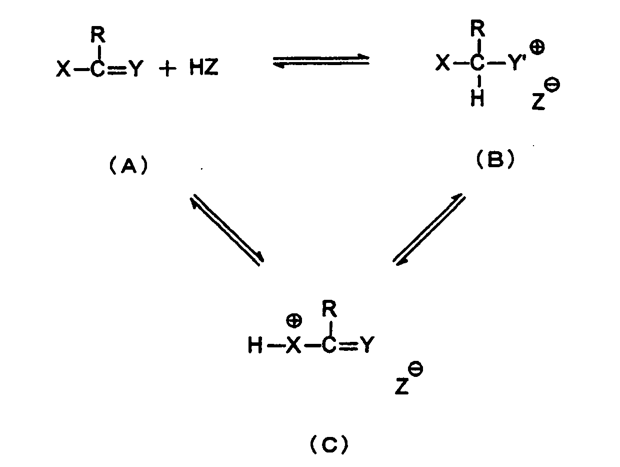
wherein the two way arrows represent chemical equilibrium;
Compound (A) represents a substantially non-fluorescent compound,
Compound (B) represents a substantially non-fluorescent compound that does not have an absorption maximum in the range of from 400 to 1000 nm under at least one condition;
Compound (C) represents a compound which is produced by an interaction of Compound (B) and the multi-stranded nucleic acid under the condition mentioned above, and has an absorption maximum in the range of from 400 to 1000 nm and substantially expresses fluorescent property based on the interaction;
X represents a group of atoms required to form a dye molecule by bonding to -C(R)=Y; Y represents a group of atoms required to form a dye molecule by bonding to X-C(R)=; R represents hydrogen atom or a substituent and may bind to X and/or Y to form a ring; Compound (A) is as specified in appended claim 1; Y' represents a residue remaining as a result of occurrence of an electron transfer so that the double bond of C=Y in Compound (A) is protonated; and Z represents a conjugate base of acid HZ and may bind to X, Y (or Y') or R.

Preferred basic structures formed by X and Y are listed below as (DX) and (DY), respectively. However, the scope of the compounds used for the method of the present invention is not limited to these examples. In the structures represented as (DX) and (DY), A represents an alkyl group, an aromatic group, or a lone pair, and when two or more of A exist, they may be the same or different (in the formulas, when A represented an alkyl group or an aromatic group, counter ions and signs indicating ions are omitted). A' and A" each represent an alkyl group or an aromatic group. Symbol k represents an integer of 0, 1 or 2. Hydrogen atoms may be replaced with a substituent, and a condensed ring structure may be formed, if possible. 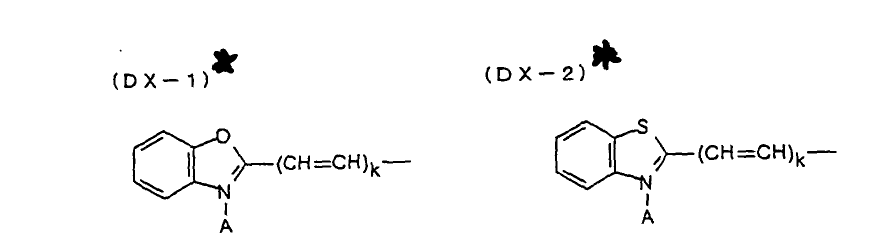 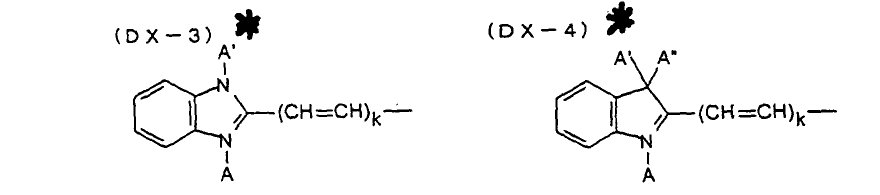 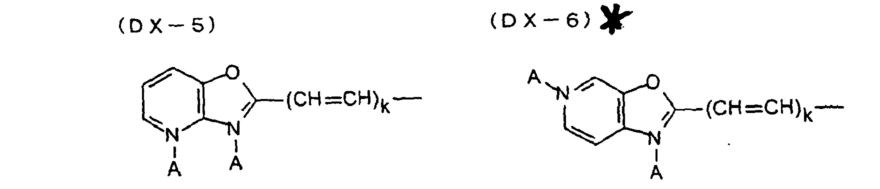 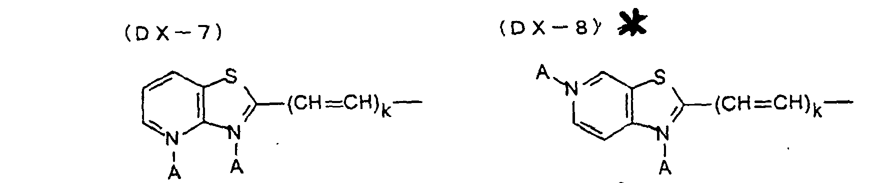 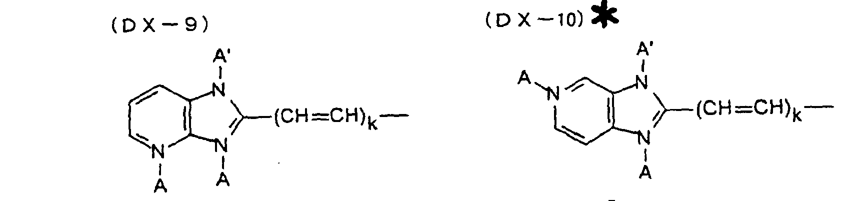 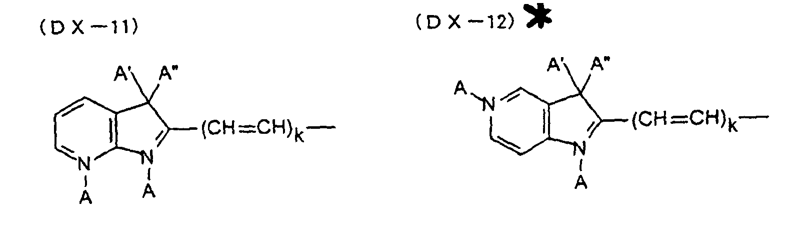 *not in accordance with the present invention 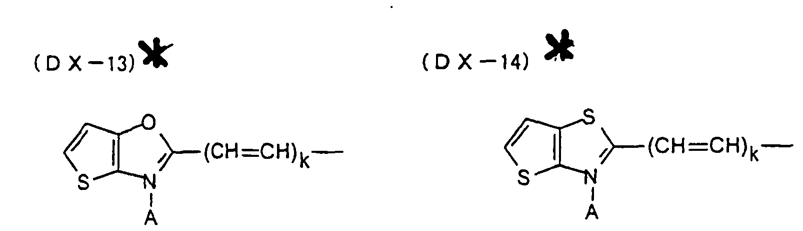 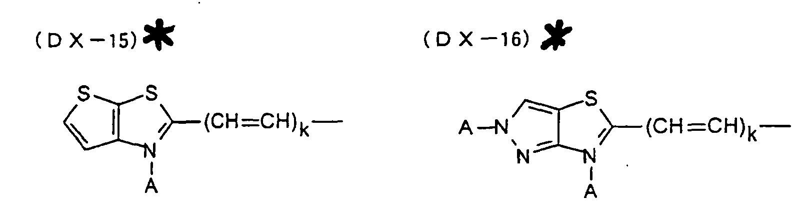 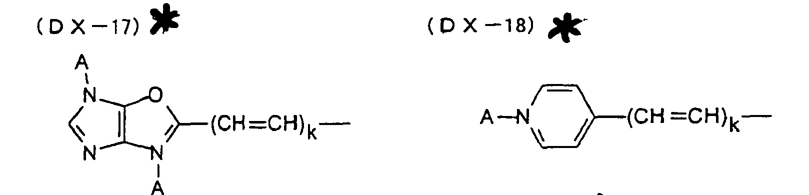 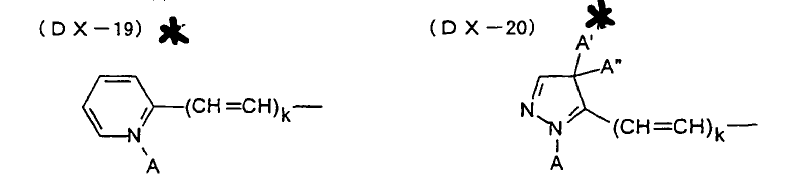 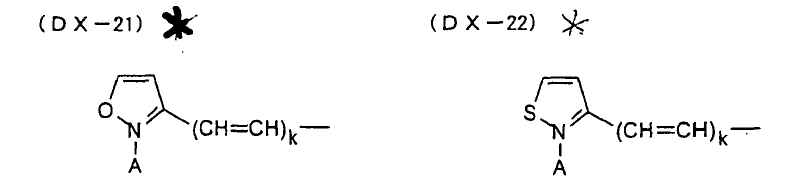 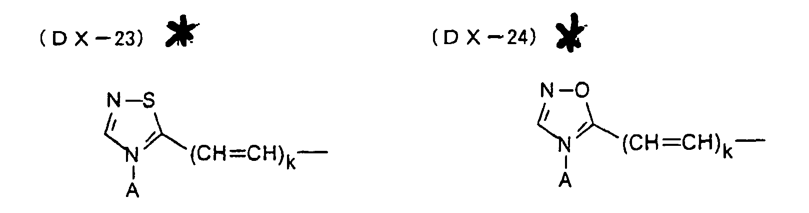 *not in accordance with the present invention 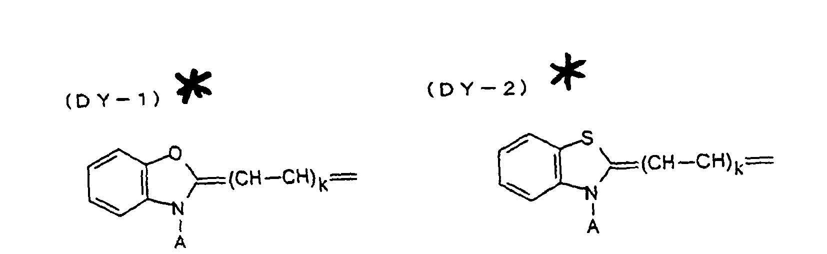 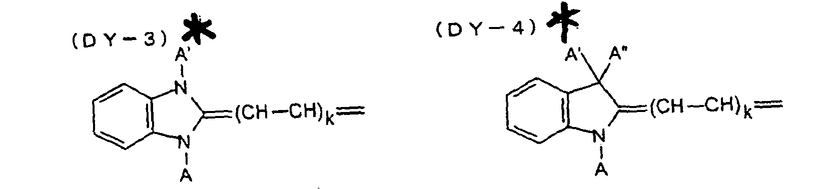 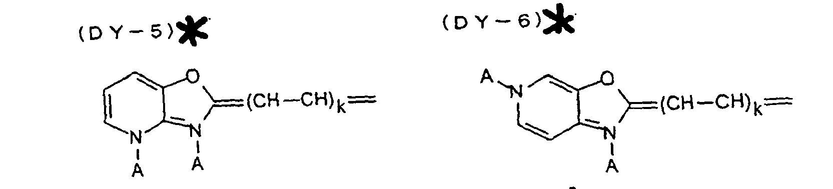 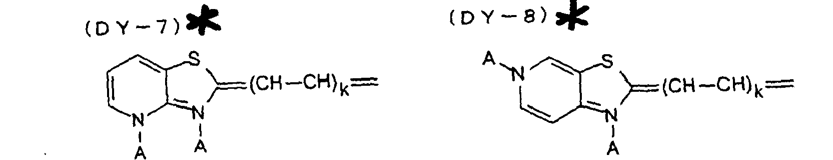 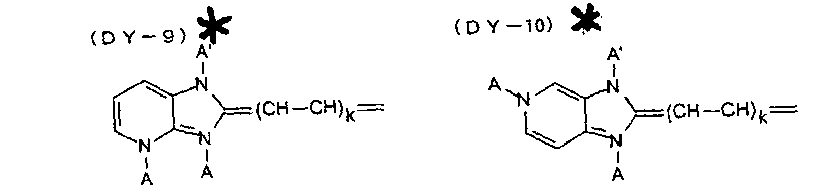 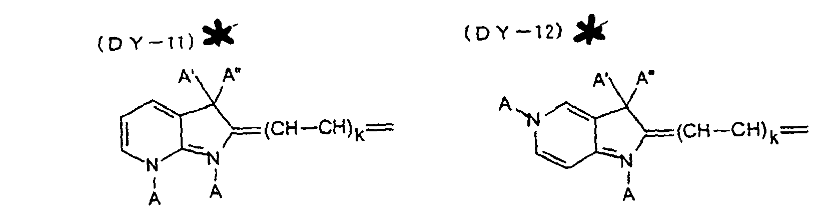 *not in accordance with the present invention 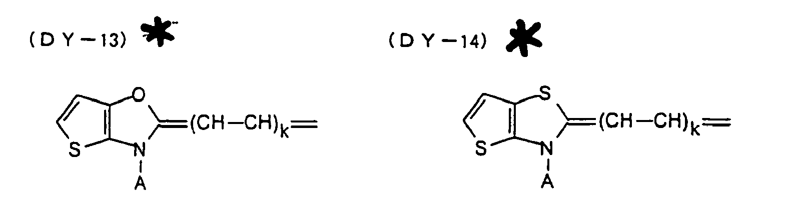 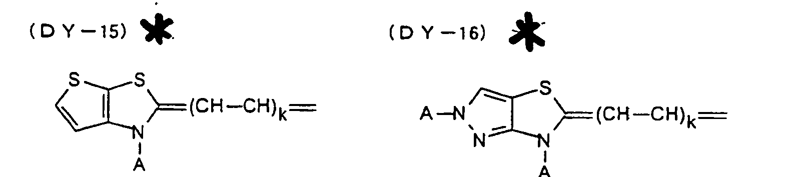 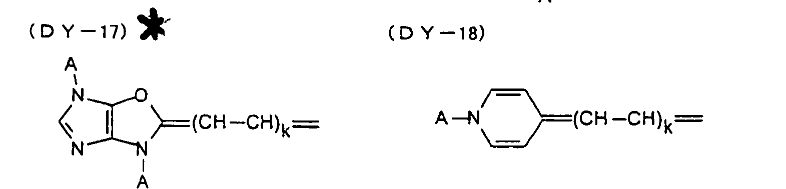 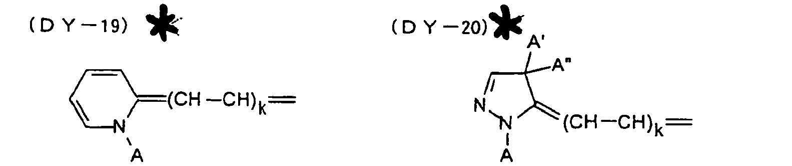 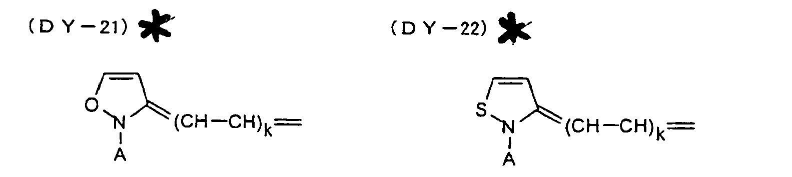 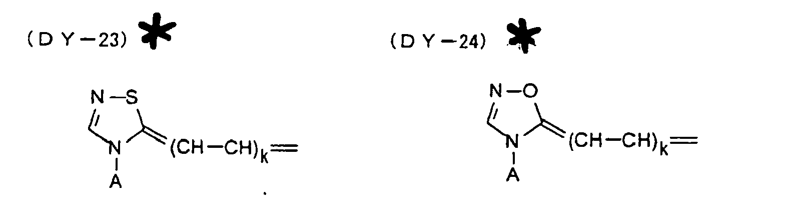 *not in accordance with the present invention

The compound as a dye compound capable of interacting with the multi-stranded nucleic acid, which has the following properties (c) the compound is in a non-protonated state and is substantially non-fluorescent in an aqueous solution in the absence of the multi-stranded nucleic acid, (d) the compound in a protonated state can exist in a substantially colorless and substantially non-fluorescent state in an aqueous solution under at least one condition in the absence of the multi-stranded nucleic acid, and (e) the compound changes to a substantially colored state based on an interaction with the multi-stranded nucleic acid and substantially expresses fluorescent property based on the interaction when the multi-stranded nucleic acid is allowed to exist in the condition defined in the above (d), and is selected from the compounds represented by formula (V) as specified in appended claim 1.

In formula (V): R¹, R² and R³ each independently represent hydrogen atom or a substituent, R¹ and R², and R² and R³ may bond to each other to form a ring. These substituents represented by R¹, R² and R³ are selected from the group consisting of a halogen atom, an alkyl group, an alkenyl group, an alkynyl group, an aryl group, an amino group, hydroxyl group, an alkoxyl group, an aryloxy group, an alkylthio group, an arylthio group, an acylamino group, a sulfonylamino group, a heterocyclic group, cyano group, a sulfonyl group, a sulfinyl group, nitro group, sulfo group, carboxyl group, an alkoxycarbonyl group, an aryloxycarbonyl group, a carbamoyl group and a sulfamoyl group. These substituents may be further substituted, and the total atomic number is from 1 to 50, more preferably from 1 to 35, further preferably from 1 to 25. When R¹ and R², and R² and R³ bond to each other to form a ring, 5- to 8-membered rings are preferred. The ring to be formed may be either non-aromatic or aromatic, and may be either a carbon ring or a heterocyclic ring.

Q represents oxygen atom, sulfur atom, -N(R²⁴)- or -C(R²⁴)(R²⁵)-. R²⁴ and R²⁵ each independently represent an alkyl group, an aryl group, or a heterocyclic group. R²⁴ and R²⁵ may bond to each other to form a 3- to 8-membered ring. The alkyl group, aryl group or heterocyclic group represented by R²⁴ or R²⁵ may be further substituted, and the total atomic number is preferably from 3 to 50, more preferably from 3 to 35, further preferably from 3 to 25. Q is preferably oxygen atom or sulfur atom, most preferably sulfur atom. R⁶ represents an alkyl group, an aryl group or a heterocyclic group, and these substituents may be further substituted. The total atomic number is preferably from 4 to 50, more preferably from 4 to 35, further preferably from 4 to 25. R⁶ may bond to R¹⁵ or R¹⁷ to form a ring, if possible.

Symbol n represents an integer of 0, 1, 2 or 3.

In order to enhance the interaction with a nucleic acid, the aforementioned compounds preferably have one or more cationic groups in any of the substituents. The term "cationic group" is used as a concept including those that can become cations by protonation, as well as cations. Examples of the cationic group include a sulfonium group, a phosphonium group, an amino group and an ammonium group, and an amino group and an ammonium group are preferred.

The compound expressing fluorescent property upon contact with a multi-stranded nucleic acid is represented by the aforementioned formula (V).

These compounds have significant differences in fluorescence intensity depending on presence or absence of a nucleic acid, especially presence or absence of a multi-stranded nucleic acid, and they are preferably used for the detection of a nucleic acid.

R¹⁴, R¹⁵, R¹⁶ and R¹⁷ each represent hydrogen atom, a halogen atom, an alkyl group, an alkenyl group, an alkynyl group, an aryl group, amino group, hydroxyl group, an alkoxyl group, an aryloxy group, an alkylthio group, an arylthio group, an acylamino group, a sulfonylamino group, a heterocyclic group, cyano group, a sulfonyl group, a sulfinyl group, nitro group, sulfo group, carboxyl group, an alkoxycarbonyl group, an aryloxycarbonyl group, a carbamoyl group, a sulfamoyl group, an acyl group or a mercapto group, more preferably hydrogen atom, a halogen atom, an alkyl group, an alkenyl group, an alkynyl group, an aryl group, amino group, an alkoxy group, an aryloxy group, an alkylthio group, an arylthio group, an acylamino group, a sulfonylamino group, a heterocyclic group, cyano group, a sulfonyl group, a sulfinyl group, sulfo group, carboxyl group, an alkoxycarbonyl group, an aryloxycarbonyl group, a carbamoyl group, a sulfamoyl group or an acyl group. Most preferred are hydrogen atom, a halogen atom, an alkyl group, an aryl group, an amino group, an alkoxy group, an aryloxy group, an alkylthio group, an arylthio group, an acylamino group, cyano group, a sulfonyl group, sulfo group, carboxyl group, an alkoxycarbonyl group and a carbamoyl group. Specific examples thereof include those mentioned as examples of R¹, R² and R³. These substituents may be further substituted. The total atomic number of each of R¹⁴, R¹⁵, R¹⁶ and R¹⁷ is preferably from 1 to 50, more preferably from 1 to 35, further preferably from 1 to 25.

R¹⁴ and R¹⁵, and R¹⁶ and R¹⁷ may bind to each other to form a 5- to 8-membered ring. The ring to be formed may be either non-aromatic or aromatic, and may be either a carbon ring or a heterocyclic ring. An aromatic 6-membered ring is most preferred. In the present invention, most preferably R¹⁴ and R¹⁵ or R¹⁶ and R¹⁷ form a benzo condensed ring to form a quinoline structure.

The compounds of the present invention preferably have one or more cationic groups in any of the substituents in order to enhance the interaction with a nucleic acid. The term "cationic group" is used as a concept including those that can become cations by protonation, as well as cations. Examples of the cationic group include a sulfonium group, a phosphonium group, an amino group and an ammonium group, and an amino group and an ammonium group are preferred. The binding position is preferably R¹, R², R³, R¹⁴, R¹⁵ R¹⁶, R¹⁷ or R⁶ in the formula (V).

Specific examples of the compounds suitably used for the method of the present invention are mentioned below. However, the method of the present invention is not limited to use of these compounds. 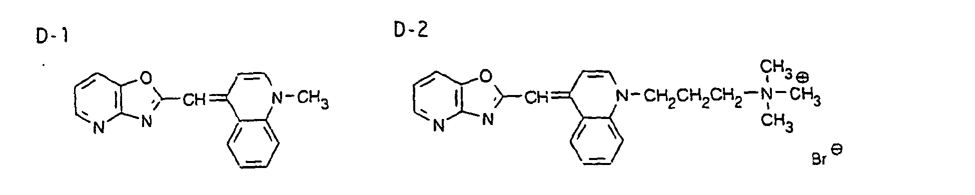 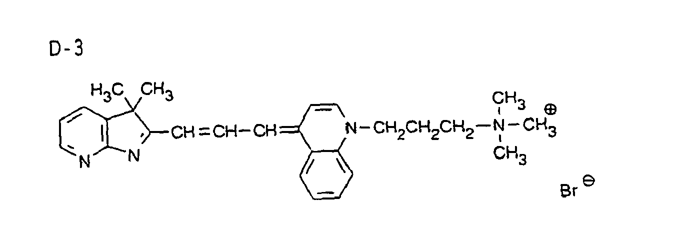 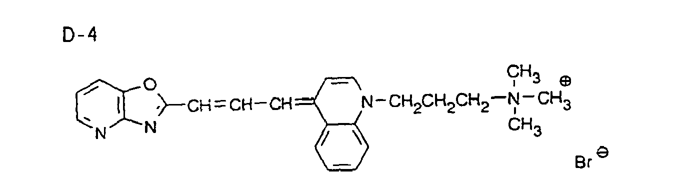 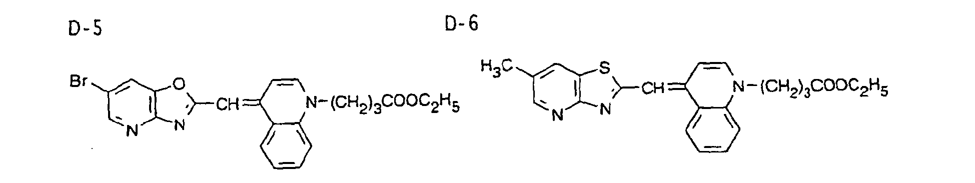 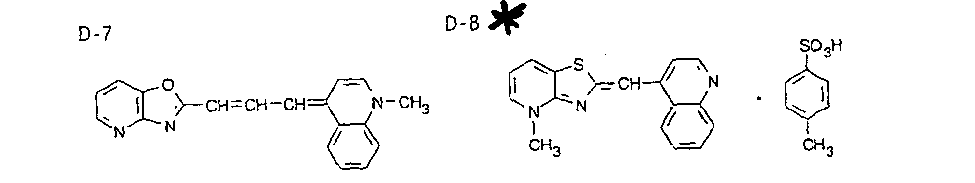 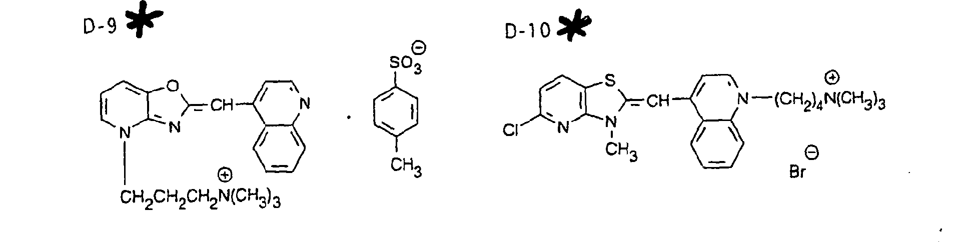 *not in accordance with the present invention 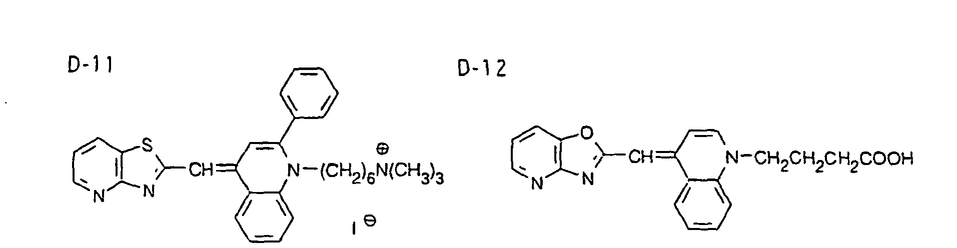 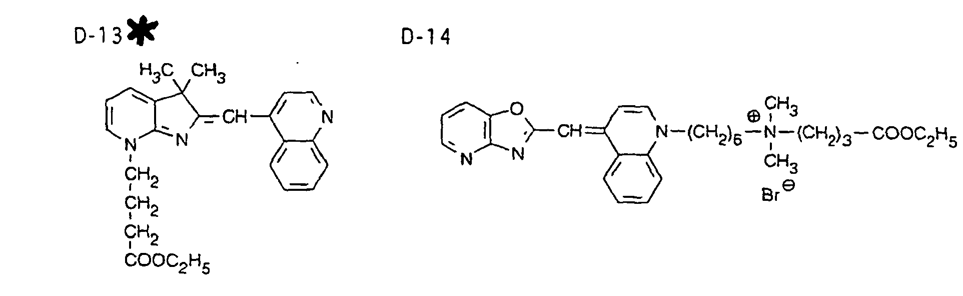 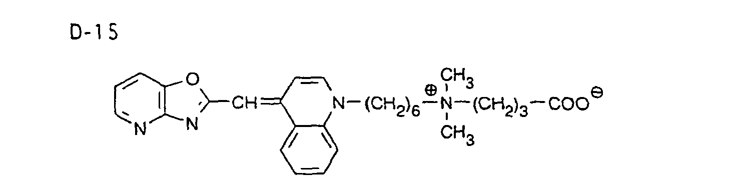 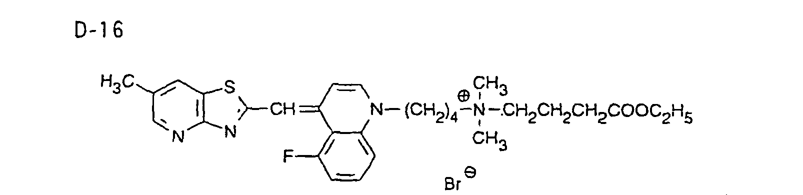 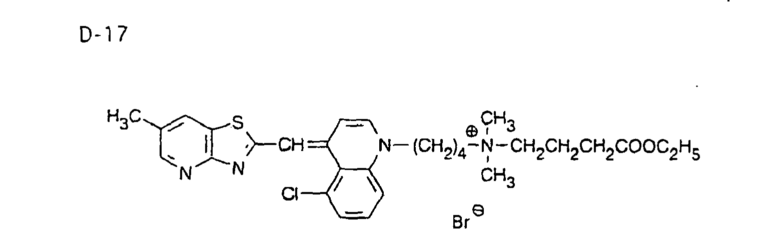 *not in accordance with the present invention 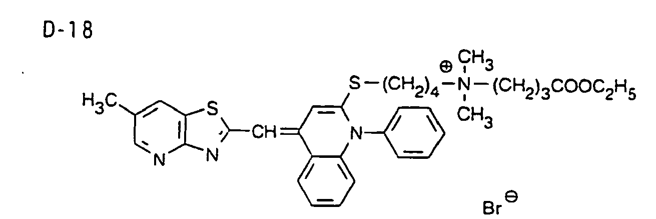 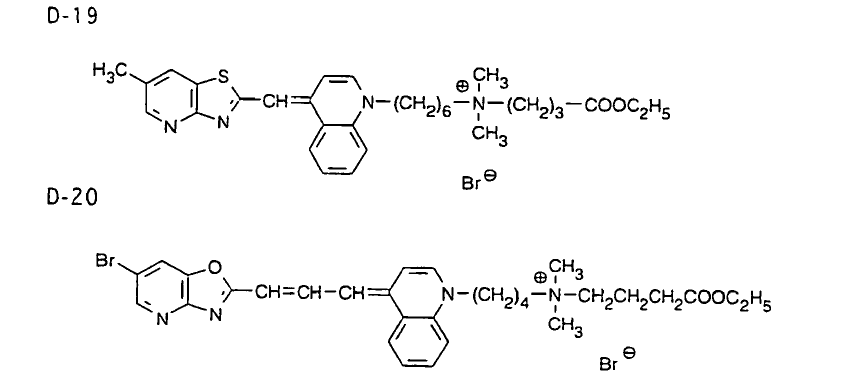 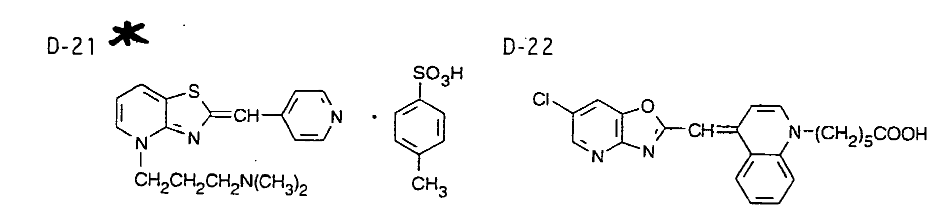 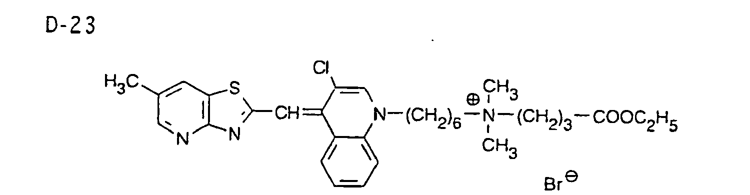 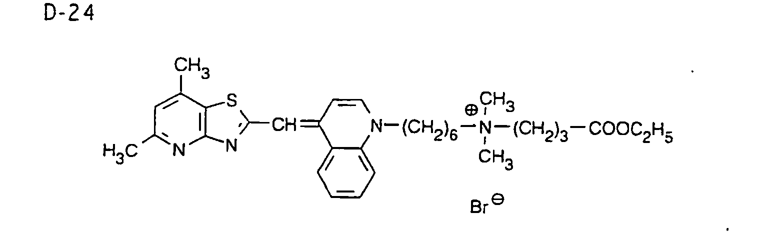 *not in accordance with the present invention 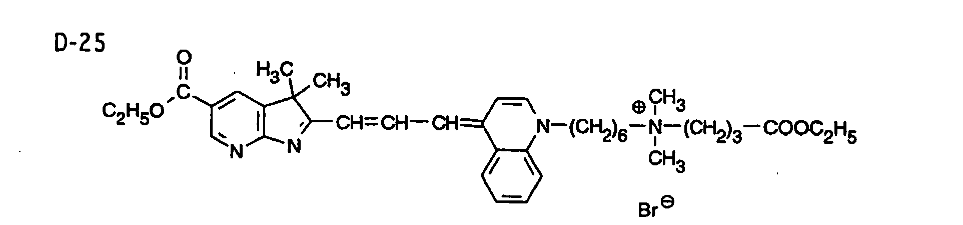 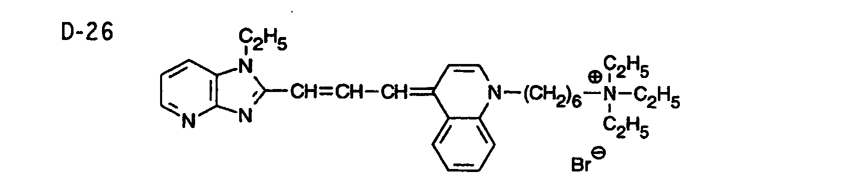 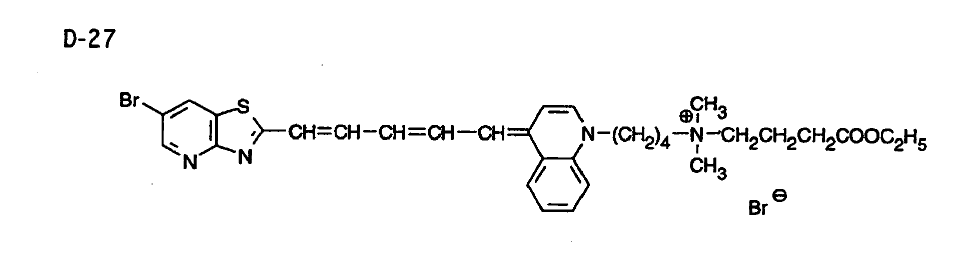 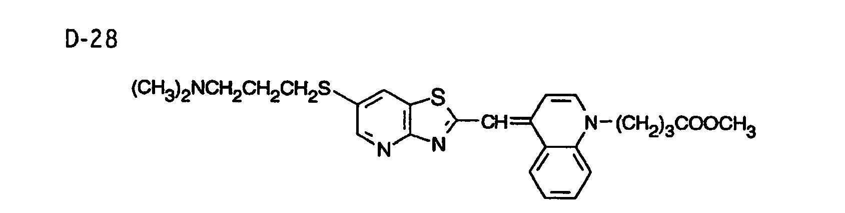 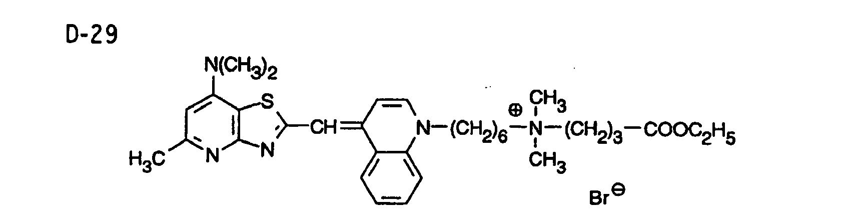 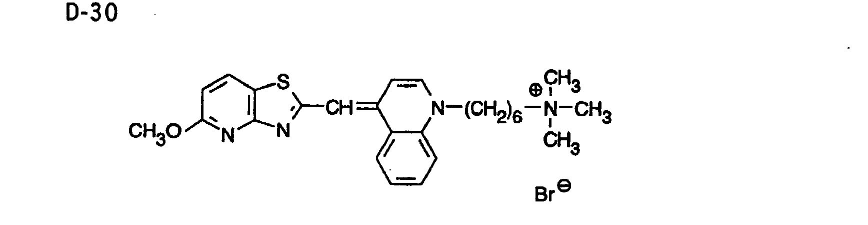 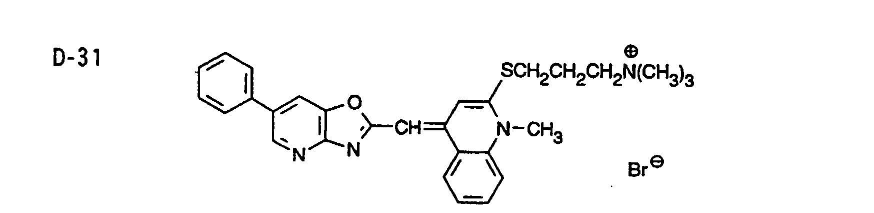 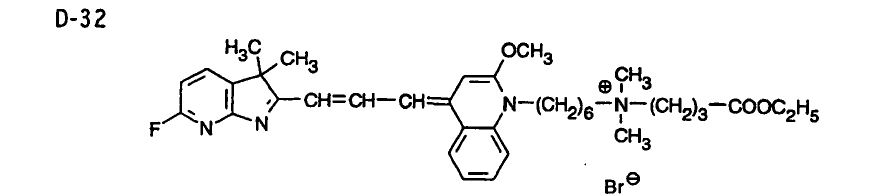 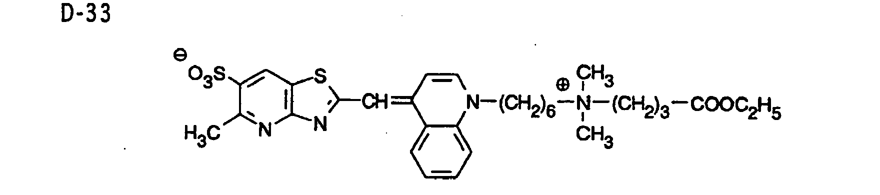 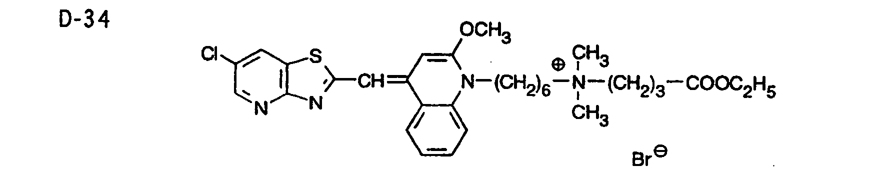 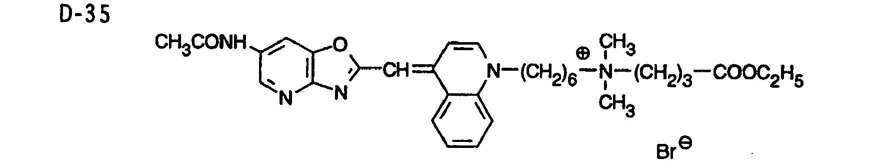 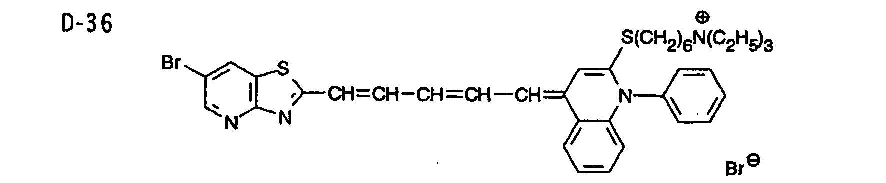 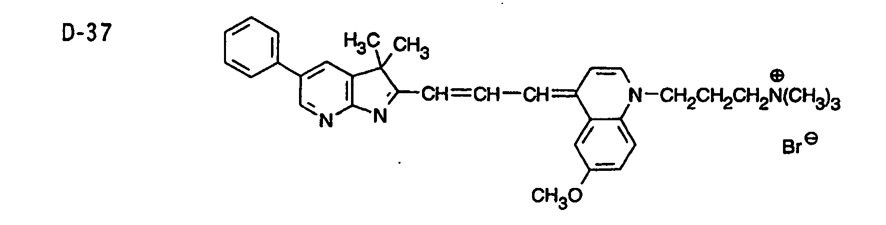 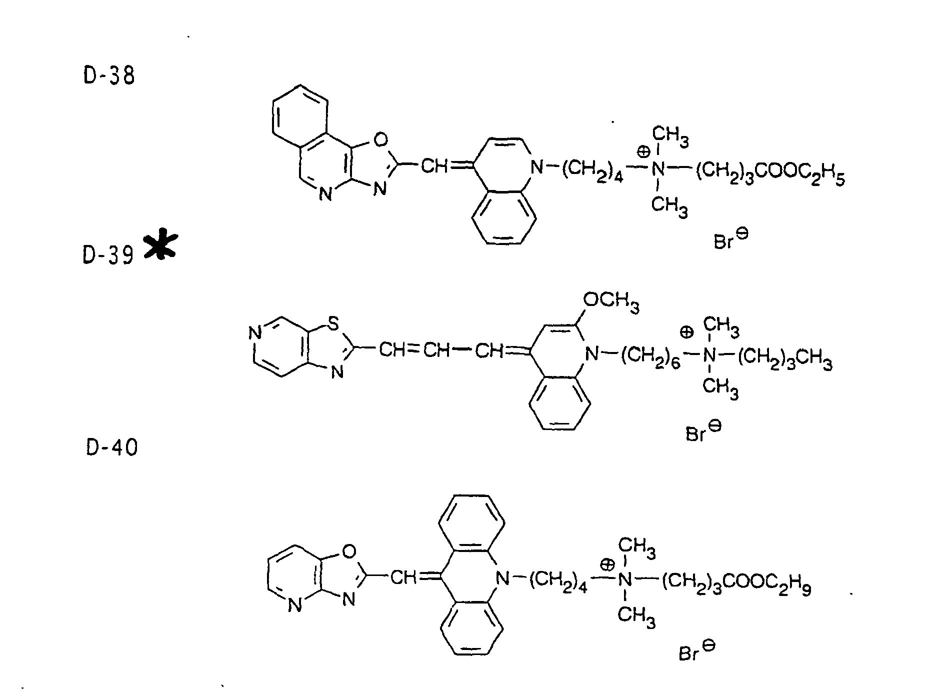 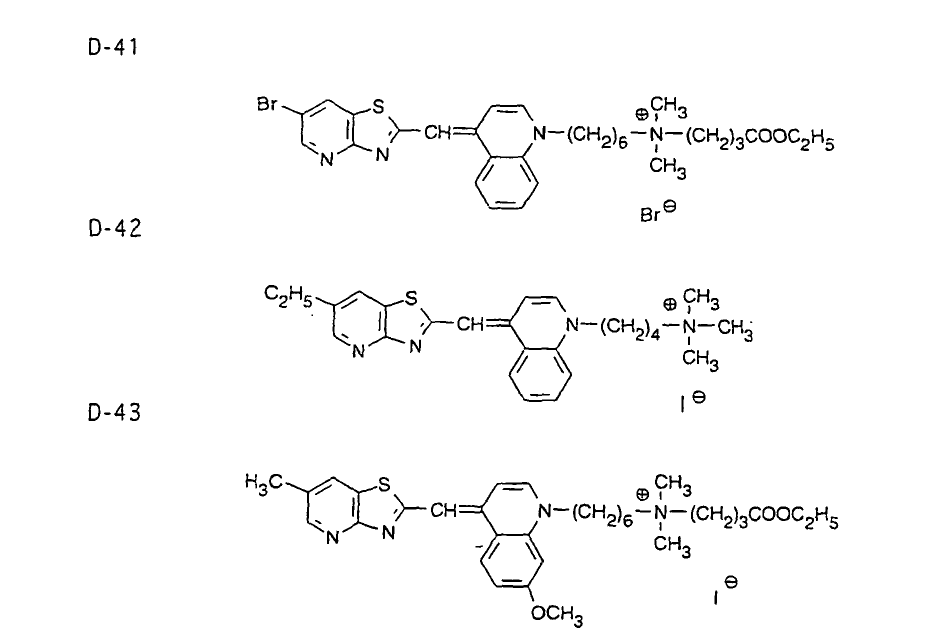 *not in accordance with the present invention 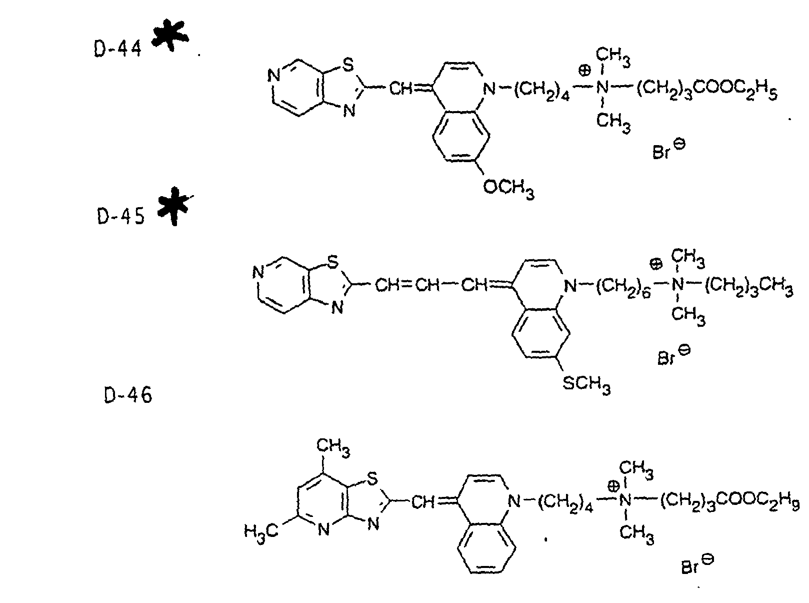 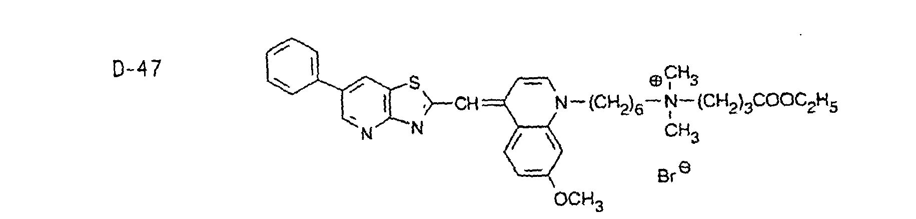 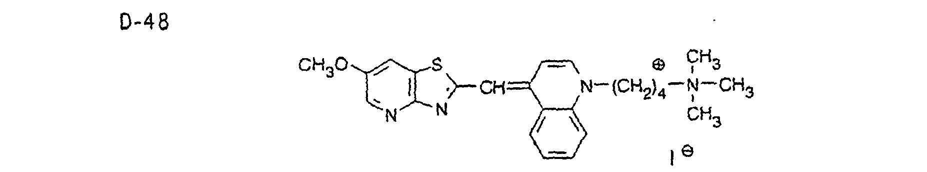 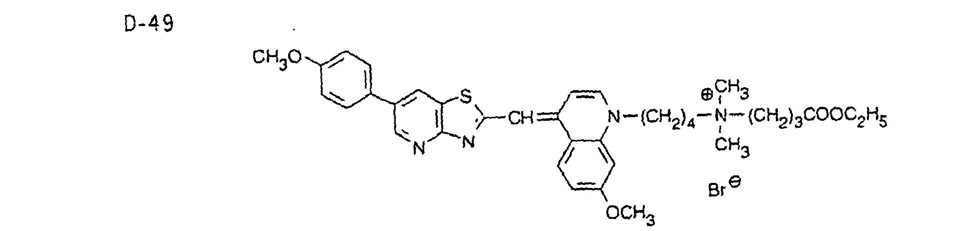 *not in accordance with the present invention

The compounds of the present invention represented by the aforementioned formula (V) may form salts. The types of the salts are not particularly limited, and they may be either acid addition salts or base addition salts depending on type of substituent and the like. Further, the aforementioned compounds and salts may form hydrates or solvates. Any of these substances fall within the scope of the present invention. Furthermore, the compounds of the present invention may have 1 or more asymmetric carbons depending on types of substituents, and isomers such as enantiomers and diastereoisomers may exist. As for compounds containing an olefinic double bond, geometrical isomers may exist. Any isomers, any mixtures of isomers, racemates and the like all fall within the scope of the present invention. Further, the compounds of the present invention may exist also as tautomers, and any tautomers and mixtures thereof also fall within the scope of the present invention.

The aforementioned compounds are generally classified into dye bases among cyanine dyes, and general synthesis methods for said compounds are known in this field. Therefore, those skilled in the art can easily synthesize the compounds. For example, the methods described in U.S. Patent Nos. 5,656,449 and 5,658,751 can be preferably used. For example, an objective substance can be easily produced by employing any of the synthetic methods explained below depending on whether n in the formula (V) is 0 or 1.

### <Compounds where n = 0>

The objective compound of the method 1 corresponds to a comparative compound of the formula (VI), and exactly the same reactions can also be applied to a comparative compound of the formula (VIII). In the formula, L represents a group that can be eliminated, and a halogen atom, an alkylthio group, an arylthio group, an alkoxyl group, an aryloxy group or the like can be preferably used. An alkylthio group is generally used. As for the dye-producing reaction, a dye can be synthesized by reacting acetic anhydride using an acid such as p-toluenesulfonic acid as a catalyst in an aprotic polar solvent such as dimethylformamide, as U.S. Patent No. 5,656,449 mentions specific examples thereof.

The objective compound of the method 2 corresponds to a compound of the formula (V), and exactly the same reactions can also be applied to a comparative compound of the formula (VII). In the formula, Prot. represents an alkyl group that can be deprotected, and methoxyethoxymethyl group described in U.S. Patent No. 5,656,449 as well as methylthiomethyl group, 4-methoxybenzyl group and the like can be used. The dye-producing reaction can be performed by using a base such as triethylamine in an aprotic polar solvent such as dichloromethane and dimethylformamide. The deprotection reaction after the dye-producing reaction may differ depending on the type of a protective group used. The deprotection can be carried out under an acidic condition when methoxyethoxymethyl group, 4-methoxybenzyl group or the like is used, or even under an oxidizing condition when 4-methoxybenzyl group is used. For details, "Protective Groups in Organic Synthesis, 3rd Edition", Theodora W. Greene, Peter G.M. Wuts (Wiley, 1999) can be referred to.

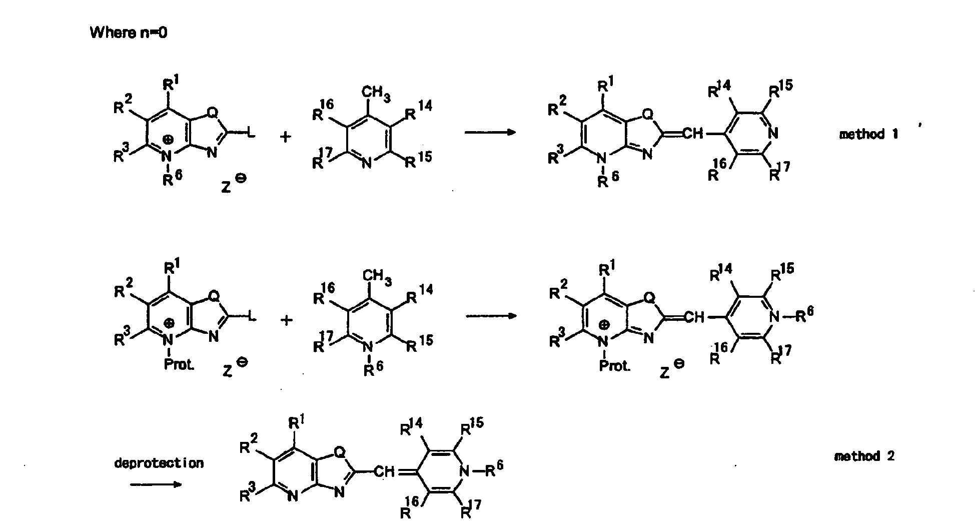

When Q is sulfur atom, for preparation of 2-alkylthiothiazolopyridines used as raw materials of the dyes, methyl iodide can be allowed to react on corresponding thiazolopyridine-2-thiones in the presence of a base such as potassium carbonate in dimethylformamide, as described in, for example, Heterocycles, p.133, 1993. In order to synthesize the thiazolopyridine-2-thione structure, a synthetic route can be appropriately selected from the two synthetic routes using 2-aminopyridines as starting materials which are mentioned below.

As the first method, thiazolopyridine-2-thiones can be obtained by reacting aminopyridines in which the ortho position relative to the amino group is brominated or chlorinated with potassium ethylxanthate in 1-methyl-2-pyrolidone under a heating condition (from 160 to 170°C). Further, it is also possible to synthesize 2-amino-3-bromopyridines by brominating pyridine-2-ones used as starting materials in a position-selective manner, and then successively reacting the product with phosphorous oxychloride and aqueous ammonia, as described in Heterocycles, p.133, 1993.

In the second method, 2-aminothiazolopyridines are first synthesized by reacting potassium thiocyanate with 2-aminopyridines in the presence of bromine, as described in Journal of Medicinal Chemistry (J. Med. Chem.), p.126, 1967. The obtained 2-aminothiazolopyridines are converted into diazonium salts by reacting sodium nitrite in a conventional manner, and the diazonium salts can be reacted with copper(I) chloride to synthesize corresponding 2-chlorothiazolopyridines. Further, 2-chlorothiazolopyridines can be reacted with thiourea and hydrolyzed to obtain the objective thiazolopyridine-2-thiones.

Aminopyridines or pyridine-2-ones can be used as starting materials for both of the methods for synthesizing the thiazolopyridine-2-thione structure mentioned above. However, the starting material is not limited to these compounds. The methods can also be used for aminoquinolines (specific examples are described in Chemische Berichte (Chem. Ber.) p.869, 1959), aminoisoquinolines (specific examples are described in Canadian Journal of Chemistry (Can. J. Chem.), p.2466, 1966) and other condensed ring type compounds containing hetero atoms (specific examples are described in Journal of Medicinal Chemistry (J. Med. Chem.), p.2546, 1995). It is possible to introduce various substituents into R¹, R² and R³ of the 2-aminopyridines, which are the starting materials of the synthetic process mentioned above, and thereby the compounds can be derived into thiazolopyridine-2-thiones, which are synthetic intermediates of the compounds of the present invention.

As for aminopyridine analogues, there are many commercially available regents of aminopyridines in which the ortho position relative to the amino group is brominated or chlorinated. However, such aminopyridines can be easily synthesized by halogenating aminopyridines. Bromination of aminopyridines is detailed in Synthesis, p.2175, 2001, and 3-bromo and 3,5-dibromo compounds from 2-amino compounds, 2-bromo and 2,6-dibromo compounds from 3-amino compounds, and 3-bromo and 3,5-dibromo compounds from 4-amino compounds, respectively, can be selectively synthesized by changing the reaction conditions. As for chlorination of 2-aminopyridines, 3-chloro compounds and 3,5-dichloro compounds can be obtained by reacting chlorine as described in literature (Zh. Russ. Fiz. -Khim. O-va, p.685, 1928). As for iodination of 2-aminopyridines, 5-iodine compounds can be obtained by reacting iodine and periodic acid as described in Tetrahedron Letters (Tetrahedron Lett.), p.7493, 1993.

When one of R¹, R² and R³ is a halogen, the compounds can be further derived by a substitution reaction. When R¹, R² or R³ is chlorine atom, the chlorine atom can be converted into methoxy group by a reaction with sodium methoxide as described in Australian Journal of Chemistry (Aust. J. Chem.), p.2025, 1982. Further, the chlorine atom can be converted into ethoxy group by a reaction with sodium ethoxide as described in Recl. Trav. Chim. Pays-Bas, p.1187, 1956. It can be converted into hydroxyl group by a reaction with aqueous 3 N sodium hydroxide as described in Org. Prep. Proced. Int., p.117, 1997. When R¹, R² or R³ is bromine atom, the atom can be converted into 2-pyridylthio group by a reaction with 2-mercaptopyridine in the presence of potassium t-butoxide and potassium iodide as described in Bull. Chim. Soc. Fr., p.290, 1995. The bromine atom can be converted into methylamino group by a reaction with methylamine in the presence of copper sulfate as described in Chem. Zentralbl., p.1219, 1936. The bromine atom can be converted into 2-amino-5-stilylpyridine by a reaction with vinylbenzene in the presence of a palladium catalyst as described in Journal of Organometallic Chemistry (J. Organomet. Chem.), p.259, 1995.

When R¹, R² or R³ is iodine atom, the iodine atom can be converted into methylthio group by a reaction with methanethiol in the presence of copper powder in aqueous sodium hydroxide as described in Australian Journal of Chemistry (Aust. J. Chem.), p.877, 1992. Further, the iodine atom can be converted into propylthio group by a reaction with propanethiol in the presence of copper powder in aqueous sodium hydroxide as described in Australian Journal of Chemistry (Aust. J. Chem.), p.877, 1992. The iodine atom can be converted into cyano group by a reaction with copper cyanide as described in Journal of American Chemical Society (J. Am. Chem. Soc), p.1479, 1944. The iodine atom can be converted into methoxy group by a reaction with methanol in the presence of sodium and copper as described in Journal of Medicinal Chemistry (J. Med. Chem.), 3109, 1997. The iodine atom can be converted into 1-propoxy group by a reaction with 1-propanol in the presence of sodium ethoxide and copper as described in Journal of Medicinal Chemistry (J. Med. Chem.), p.1518, 1981.

When R¹, R² or R³ is a halogen, the halogen can be converted into any of wide variety of substituents by using a coupling reaction utilizing a transition metal catalyst. For example, arylation can be conducted by a coupling reaction with 2-amino-5-bromopyridine and an arylboric acid using a palladium catalyst as described in Heterocycles, p.2711, 1987. Further, arylation can be conducted by a coupling reaction with 2-amino-6-bromopyridine and an arylboric acid using a palladium catalyst as described in, for example, Journal of Medicinal Chemistry (J. Med. Chem.), p.675, 2000, and corresponding acetylene derivatives can be obtained by reacting 2-amino-6-bromopyridine and acetylenes in the presence of a palladium catalyst and a copper reagent as described in Journal of American Chemical Society (J. Am. Chem. Soc), p.12416, 1995.

As for methods of introducing other functional groups into 2-aminopyridines, when 2-aminopyridines are nitrated by using a mixed acid, 2-amino-5-nitropyridines introduced with nitro group can be obtained as described in, for example, Journal of American Chemical Society (J. Am. Chem. Soc), p.3154, 1955. When 2-aminopyridines are treated with fuming sulfuric acid or sulfuric acid and sulfur trioxide, 6-aminopyridine-3-sulfonic acids introduced with sulfo group can be obtained as described in Bull. Soc. Chim. Fr., p.736, 1939. When benzyl chloride is allowed to react on 2-aminopyridines, 2-amino-5-benzylpyridines can be obtained as described in Polish Journal of Chemistry (Pol. J. Chem.), p.959, 1984.

When R¹, R² or R³ is carboxyl group, said group can also be converted into a heterocyclic group. For example, 2-(2-aminopyridin-5-yl)benzothiazoles are obtained by a reaction with 6-aminonicotinic acid and 2-aminobenzenethiol as described in Journal of Medicinal Chemistry (J. Med. Chem.), p.3375, 1996. As for 2-aminopyridine derivatives to which another heterocyclic ring is bonded, the synthetic method of 3,4'-bipyridine-6-amine (R¹ = R³ = H and R² = 4-pyridyl) is described in U.S. Patent No. 4,276,293. As for the 2-ethyl[3,4-bipyridine]-6-amine (R¹ = H, R² = 4-pyridyl, and R³ ethyl), the synthetic method is described in U.S. Patent No. 4,313,951.

Various methods of introducing a substituent into pyridin-2-ones are also known. Pyridin-2-ones are important for the synthesis of the compounds of the present invention, because pyridin-2-ones can be easily converted into 2-aminopyridines after introduction of a substituent. When pyridin-2-ones and benzophenones are reacted, the addition reaction for carbonyl group will advance, and adducts of which R¹ is a tertiary alcohol can be obtained as described in Tetrahedron, p.2343, 1987. When aryl aldehydes are reacted with pyridin-2-ones, adducts of which R¹ is a secondary alcohol can be obtained as described in Tetrahedron, p.2343, 1987. When carbon dioxide is reacted with pyridin-2-ones, R¹ can be converted into carboxyl group as described in Tetrahedron, p.2343, 1987. When phenyl isocyanate is reacted with pyridin-2-ones, R¹ can be converted into anilide group as described in Tetrahedron, p.2343, 1987. When phenylacetyl chloride is reacted with pyridin-2-ones, R¹ can be converted into phenylacetyl group as described in Tetrahedron, p.2343, 1987. When methyl iodide is reacted with pyridin-2-ones, R¹ can be converted into methyl group as described in Tetrahedron, p.2343, 1987. When potassium peroxodisulfate is reacted with pyridin-2-ones, R² can be converted into hydroxyl group as described in Tetrahedron, p.2891, 1990. When benzyl bromide is reacted with pyridin-2-ones, R² can be converted into benzyl group as described in Chemistry Letters (Chem. Lett.), p.333, 1996.

Methods of cyclizing the thiazole ring on the pyridine ring are explained above. A synthetic route in which the thiazole ring is previously constructed, and then the pyridine ring is formed may also be applied, and a further variety of derivatives can be synthesized as described below. Dipotassium cyanoimidodithiocarboxylate is a common intermediate that gives many kinds of 2-alkylthio-4-aminothiazoles, and the compound can be derived into thiazolopyridines by increasing carbon atoms. For example, 7-hydroxy-2-methylthiothiazolo[4,5-b]pyridine-6-carboxylic acid esters can be obtained according to the following synthesis route described in Journal of Heterocyclic Chemistry (J. Heterocyclic Chem.), p.1361, 1984. Further, by using a similar reaction, 7-hydroxythiazolo[4,5-b]pyridones can be synthesized as described in a literature (J. Prakt. Chem., p.260, 1979).

Similarly, as a method of starting the synthesis from construction of a thiazole ring, aminoacetonitrile can be used as a starting material to obtain 2-methylthiothiazolo[5,4-b]pyridines. Specific examples include the synthetic route described in Journal of Heterocyclic Chemistry (J. Heterocyclic Chem.). In addition, a thiazolonaphthylidine structure as condensed ring type compounds can be synthesized as follows. The synthesis of 1,6-naphthylidinones as intermediates is described in Journal of Heterocyclic Chemistry (J. Heterocyclic Chem.), p.2085, 1990, and their derivatization into thiazolonaphthylidines is detailed in Heterocycles, p.133, 1993.

When Q is oxygen atom, a generally used method includes, basically, the step of allowing carbon disulfide, thiocarbonyl chloride, or potassium xanthate to react on 2-aminopyridine having hydroxyl group at the 3-position for conversion into oxazolopyridine-2-thione and alkylating the sulfur atom bonding at the 2-position with an alkylating reagent, as in the production of thiazolopyridines. 2-Alkylthio-oxazolopyridines can be easily obtained by allowing methyl iodide to react on corresponding oxazolopyridine-2-thiones in dimethylformamide in the presence of a base such as potassium carbonate as described in, for example, Heterocycles, p.133, 1993. In order to synthesize oxazolopyridine-2-thiones, 3-hydroxypyridines is first nitrated with a mixed acid, and the resulting nitro compounds are reduced to synthesize 2-amino-3-hydroxypyridines. As these steps, those described in, for example, Pharmaceutical Bulletin (Pharm. Bull.) are known. Further, oxazolopyridine-2-thiones can be synthesized by allowing potassium ethylxanthate to react on 2-amino-3-hydroxypyridines in ethanol as described in Chemistry of Heterocyclic Compound (Chem. Heterocycl. Compd.), p.441, 1981. Alternatively, oxazolopyridine-2-thiones can also be synthesized by allowing carbon disulfide to react on 2-amino-3-hydroxypyridines in a solution of potassium hydroxide in water-containing methanol as described in Pharmaceutical Bulletin (Pharm. Bull.), p.4625, 1957.

As for nitration of 3-hydroxypyridines, the following synthesis examples are known. As for the nitration of 3-hydroxy-4-methylpyridines, for example, the method described in Hetereoctcles, p.529, 1994 is known. As for the nitration of 3-hydroxy-5-methylpyridines, for example, the method described in Journal of Medicinal Chemistry (J. Med. Chem.), p.2031, 1987 is known. As for the nitration of 3-hydroxy-6-methylpyridines, for example, the method described in Journal of Medicinal Chemistry (J. Med. Chem.), p.2031, 1987 is known. Further, 4-hydroxyisoquinolines can also be similarly nitrated, and for example, the method described in Chemical Pharmaceutical Bulletin (Chem. Pharm. Bull.), p.501, 1959 is known.

As methods for introducing amino group into 3-hydroxypyridines, those methods describe below are known in addition to the aforementioned reduction of nitro group. As a method of allowing ammonia to react on 3-hydroxy-2-iodopyridines in the presence of copper sulfate, the method described in Rocz. Chem., p.502, 1936 is known. As a method of allowing sodium amide to react on 3-hydroxypyridines, the method described in the literature (Zh. Prikl. Khim., p.1103, 1949) is known. As a method of allowing sodium hypobromate to react on 3-hydroxypyridine-2-carboxylic acid amides, the method described in the literature (Biochem. J., p.506, 1950) is known. As a method of allowing sodium hydrosulfite to react on 3-hydroxy-2-phenylazopyridines, the method described in the literature (Prace. Minist. Prizem. Chem., p.14, 1952) is known. As a method of allowing hydrazine hydrate to react on 3-hydroxy-2-chloropyridines, the method described in Journal of Medicinal Chemistry (J. Med. Chem.), p.248, 1994 is known.

When Q is -N(R²⁴)-, a generally used method comprises, basically, the step of converting 2-aminopyridine derivatives having a substituted amino group at the 3-position into imidazolopyridine-2-thiones using carbon disulfide or thiocarbonyl chloride and then alkylating the sulfur atom at the 2-position as in the production of thiazolopyridines. In order to obtain 2-aminopyridine derivatives having a substituted amino group at the 3-position, the aforementioned method of introducing a substituent used for the compound where Q is sulfur atom can be referred to. When Q is - C(R²⁴)(R²⁵)-, the compounds can be synthesized by first synthesizing the pyrrolino[2,3-b]pyridin-2-one structure, introducing groups corresponding to R²⁴ and R²⁵ at the 3-position to obtain a disubstituted compound, and converting the lactam oxygen atom at the 2-position into sulfur atom using a Lawesson's reagent or the like.

As for the synthetic method for the compounds where n≧ 1, the objective compounds of the method 3 and the method 4 correspond to compounds of the formula (V), and exactly the same reactions can also be applied for comparative compounds of the formula (VII). Both of the methods 3 and 4 can be preferably used, and either of them can be suitably selected depending on yields of intermediates and dyes to be produced or ease of purification thereof. What is different from the synthesis of the compounds where n = 0 is that the azolopyridines used as starting materials have methyl group at the 2-position. The synthesis method of 2-methyl compounds will be described later.

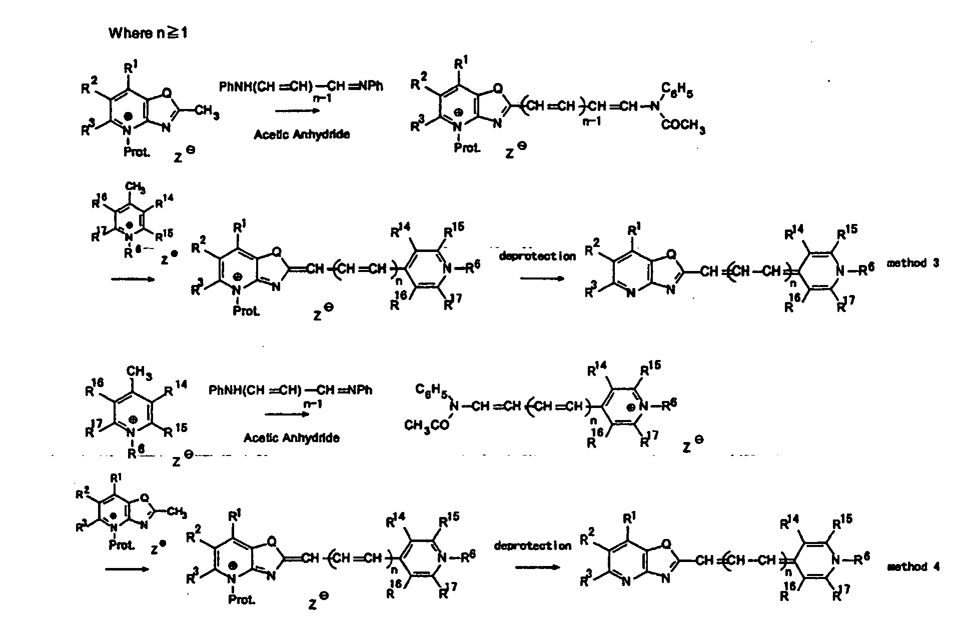

As for the compounds where n ≧ 1, the extension of the methine carbon chain can be performed by a reaction of an acid anhydride such as acetic anhydride by using, when n = 1, N,N'-diphenylformamidine, or when n ≧ 2, a vinylog compound added with vinyl groups in a number corresponding to the methine carbon chains. The reaction temperature can be selected from the range of around room temperature to 180°C, and acid anhydrides such an acetic anhydride can be used as the solvent. However, various aprotic solvents can also be preferably used. These reactions are most usual methods in the field.

For the dye-producing reaction, either a protic or aprotic solvent can be used, and the reaction may proceed even by simple heating. Preferably, the reaction can be performed by using a base such as triethylamine and pyridines in an aprotic solvent, and the reaction temperature is preferably from around room temperature to 120°C. This reaction is also a most usual reaction in the field, and various reaction conditions can be used in addition to those mentioned above.

In the formula, Prot. represents an alkyl group that can be deprotected, and methoxyethoxymethyl group described in U.S. Patent No. 5,656,449 as well as methylthiomethyl group, 4-methoxybenzyl group and the like can be used. The deprotection reaction after the dye-producing reaction may differ depending on type of a protecting group used. The deprotection can be carried out under an acidic condition when methoxyethoxymethyl group, 4-methoxybenzyl group or the like is used, or even under an oxidizing condition when 4-methoxybenzyl group is used. For details, "Protective Groups in Organic Synthesis, 3rd Edition", Theodora W. Greene, Peter G.M. Wuts (Wiley, 1999) can be referred to.

The objective compounds of the method 5 and the method 6 correspond to compounds of the formula (V), and exactly the same reactions can also be used for the comparative compounds of the formula (VIII). The reactions used in these methods are the same as those used for the method 3 and 4.

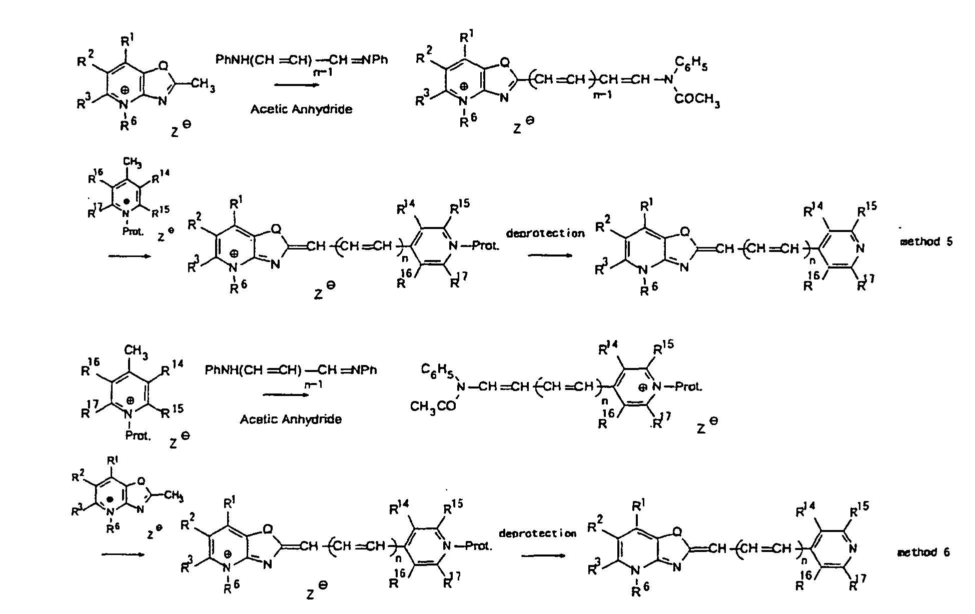

As for the synthesis of 2-methylazolopyridines, the compounds where Q is oxygen atom or -N(R²⁴)- can be synthesized by a method comprising the use of acetic anhydride with heating instead of carbon disulfide, thiocarbonyl chloride, or potassium xanthate in the reaction for the conversion into azolopyridine-2-thiones using carbon disulfide, thiocarbonyl chloride, or potassium xanthate, a method comprising the heating in the presence of an orthoacetic acid ester such as triethyl orthoacetate and an acid catalyst such as p-toluenesulfonic acid or the like. For the above preparation, the reaction temperature is preferably from 100 to 170°C, and usually the reaction is preferably performed by using a reaction regent as a solvent.

The compounds where Q is sulfur atom can be produced by converting the intermediates mentioned in the synthetic method of the compounds where n = 0, 2-amino-3-halopyridines, into 2-acetylamino-3-halopyridines with heating in the presence of an orthoacetic acid ester and an acid catalyst such as p-toluenesulfonic acid, further converting the 2-acetylamino-3-halopyridines into 2-thioacetylamino-3-halopyridines using phosphorus pentasulfide, a Lawesson's reagent or the like, and then converting the 2-thioacetylamino-3-halopyridines into 2-methylthiazolopyridines by using a strong base such as sodium hydride and potassium t-butoxide. When a strong base is used, ether type solvents such as dioxane and tetrahydrofuran can be used as the solvent. The compounds where Q is -C(R²⁴)(R²⁵)- can be synthesized by utilizing the reaction known as the Fischer's indole synthesis reaction described in of Japanese Patent Laid-open Publication No. 2001-270864 or Chemical Review (Chem. Rev.), vol. 63, p.373, 1963.

Since a huge number of synthetic methods are known for the compounds of which nitrogen-containing 6-membered ring is pyridine ring, those skilled in the art can synthesize such compounds by referring to various kinds of literature. Moreover, a huge number of the compounds are commercially available, and they can be easily obtained. As for the compounds where the nitrogen-containing 6-membered ring is quinoline ring, the synthetic methods of 4-methylquinolines (lepidines) and 2-methylquinolines (quinaldines) used as starting materials are described below. As for lepidines, a substituent can be introduced at the 2-position of lepidine by a reaction of commercially available 2-chlorolepidine and a suitable nucleophilic agent. Examples of reactions of 2-chlorolepidine and a primary amine, secondary amine, cyclic amine, alcohol, thiol, thiourea, fluoride and the like are known.

Examples of the reaction with a primary amine include, for example, the reaction described in Journal of American Chemical Society (J. Am. Chem. Soc.), p.2322, 1949. Examples of the reaction with a secondary amine include, for example, the reaction described in Yakugaku Zasshi, p.137, 1949. As the reaction with a cyclic amine, for example, the reactions described in Synthetic Communication (Synth. Commun.), p.4479, 2000 and Journal of Organic Chemistry (J. Org. Chem.), p.771, 1949 are known. As the reaction with an alcohol, for example, the reactions described in Justus Liebigs Annalen der Chemie (Justus Liebigs Ann.Chem.), p.102, 1886 and Journal of Organic Chemistry (J. Org. Chem.), p.1529, 1951 are known. Examples of the reaction with a thiol include, for example, the reaction described in Journal of American Chemical Society (J. Am. Chem. Soc.), p.491, 1948. As the reaction with a thiourea, for example, the reaction described in Chemische Berichte (Chem. Ber.), p.2732, 1929 is known. As for the reaction with a fluoride, for example, a reaction with potassium fluoride is described in J. Chem. Res. Synop., p.586, 1998 and a reaction with tetrabutylammonium fluoride is described in Mutat. Res., p.173, 2000. Further, it is also possible to arylate by a coupling reaction of 2-chlorolepidine and an arylboric acid using a palladium catalyst, and for example, the reaction described in Tetrahedron, p.8117, 1992 is known.

It is also possible to introduce a substituent at the 2-position of lepidines by a cyclization reaction of anilines and α,β-enones. As the synthesis of 2,4-dimethylquinolines, for example, the synthesis described in Justus Liebigs Annalen der Chemie (Justus Liebigs Ann. Chem.), p.7, 1887 is known. As the synthesis of 4-methyl-2-phenylquinoline, for example, the synthesis described in J. Prakt. Chem., p.73, 1925 is known.

As for the synthesis of lepidines having a suitable substituent at the 3-position, the compounds having chlorine or bromine at the 3-position can be synthesized by using 3-methylindole as a starting material. As for the compounds having chlorine, the synthesis described in, for example, Chemische Berichte (Chem. Ber.), p.252, 1887 is known. As for the compound having bromine, the synthesis described in, for example, Chemische Berichte (Chem. Ber.), p. 2613, 1887 is known. The halogen of the halogenated compounds obtained above can further be converted into another substituent. For example, arylated compounds can be synthesized by coupling the halogenated compounds to phenylboric acid in the presence of a palladium catalyst and a base. As a specific example, the reaction described in Tetrahedron, p.8117, 1992 is known. Further, it is also possible to introduce a substituent at the 3-position of lepidines by a cyclization reaction of anilines and α,β-enones. Specific examples are described below. As the synthesis of 2,4-dimethylquinolines, the synthesis described in Journal of Organic Chemistry (J. Org. Chem.), p.2782, 1957 is known.

Lepidines having a suitable substituent at the 6-position is easily synthesized by a cyclization reaction of p-substituted anilines and 3-buten-2-ones or their synthetically equivalent substances. As the reaction with 4-fluoroaniline, for example, the method described in the literature (Mutat. Res., 173, 2000) is known. As the reaction with 4-chloroaniline, for example, the reaction described in Journal of Organic Chemistry (J. Org. Chem.), p.682, 1957 is known. As the reaction with 4-bromoaniline, for example, the reaction described in Tetrahedron Letters (Tetrahedron Lett.), p.531, 2000 is known. As the reaction with 4-methoxyaniline, for example, the reaction described in Journal of American Chemical Society (J. Am. Chem. Soc.), p.86, 1945 is known. As the reaction with 4-methylaniline, for example, the reaction described in Journal of American Chemical Society (J. Am. Chem. Soc.), p.2397, 1920 is known. As the reaction with 4-dimethylaminoaniline, for example, the reaction described in the literature (Bull. Chim. Soc. Fr., p.434, 1920) is known. As the reaction with 4-nitroaniline, for example, the reaction described in Justus Liebigs Annalen der Chemie (Justus Liebigs Ann. Chem.), p.174, 1948 is known. As the reaction with 4-benzothiazo-2-yl-aniline, for example, the reaction described in the literature (Sog. Prog. Chem., p.62, 1977) is known. As the reaction with 4-benzodiphenylphosphonoylaniline, for example, the reaction described in Chemistry of Heterocyclic Compound (Chem. Heterocycl. Compd.), p.315, 1982 is known. As the reaction with 4-acetylaniline, for example, the reaction described in Chemistry of Heterocyclic Compound (Chem. Heterocycl. Compd.), p.767, 1984 is known. As the reaction with 4-diethylsulfamidoaniline, for example, the reaction described in Chemistry of Heterocyclic Compound (Chem. Heterocycl. Compd.), p.767, 1984 is known.

It is also possible to further convert the quinolines obtained by the reactions mentioned above. 6-Bromo-4-methylquinolines can be subjected to a coupling reaction with a phosphonic acid diester in the presence of a palladium catalyst and a base to obtain a phosphonic acid derivative. Specifically, the method described in the literature (Zh. Obshch. Khim., p.2503, 1986) is known. To synthesize 6-amino-4-methylquinolines, 4-methyl-6-nitroquinolines obtained by using 4-nitroanilines as a starting material can be reduced. For example, the method described in Justus Liebigs Annalen der Chemie (Justus Liebigs Ann. Chem.), p.174, 1948 is known.

In order to synthesize 4-methyl-quinoline-6-carboxylic acids, 4,6-dimethylanilines obtained by using 4-methylanilines as a starting material can be oxidized. For example, the method described in Chemische Berichte (Chem. Ber.), p.2265, 1890 is known. In order to synthesize 6-(2-benzooxazolyl)-4-methyl-quinolines, 2-aminophenol can be reacted with 4-methyl-quinoline-6-carboxylic acids. For example, the method described in Journal of Heterocyclic Chemistry (J. Heterocycl. Chem.), p.937, 1977 is known. Similarly, in order to synthesize 6-(2-benzothiazolyl)-4-methylquinolines, 2-aminobenzenethiol can be reacted with 4-methyl-quinoline-6-carboxylic acids, in order to synthesize 4-methyl-6-(2-oxazolo[4,5-b]pyridyl)quinolines 2-amino-3-hydroxypyridine can be reacted with 4-methyl-quinoline-6-carboxylic acids, in order to synthesize 6-[2-(1H-imidazo[4,5-b]pyridyl)]-4-methylquinolines, 2,3-diaminopyridine can be reacted with 4-methyl-quinoline-6-carboxylic acids, and in order to synthesize 6-[2-(1H-imidazo[4,5-c]pyridyl)]-4-methylquinolines, 3,4-diaminopyridine can be reacted with 4-methyl-quinoline-6-carboxylic acids. For all of these syntheses, the reactions described in Journal of Heterocyclic Chemistry (J. Heterocycl. Chem.), p.937, 1977 are known. In order to synthesize 4-methyl-quinoline-6-sulfonic acids, 4-methylquinolines can be reacted with sulfuric acid. For example, the reaction described in Chemische Berichte (Chem. Ber.), p.2680, 1890 is known.

Lepidines having a suitable substituent at the 7-position can easily be synthesized by a cyclization reaction of m-substituted anilines and 3-butene-2-one or their synthetically equivalent substances. As the reaction with 3-fluoroaniline, for example, the reaction described in Mutat. Res., p.173, 2000 is known. As the reaction with 3-chloroaniline, for example, the reaction described in Journal of American Chemical Society (J. Am. Chem. Soc.), p.1851, 1946 is known. As the reaction with 3-methylaniline, for example, the reaction described in Journal of Organic Chemistry (J. Org. Chem.), p.682, 1957 is known. As the reaction with 3-aminophenol, for example, the reaction described in Tetrahedron Letters (Tetrahedron Lett.), p.531, 2000 is known.

Lepidines having a suitable substituent at the 8-position can be easily synthesized by a cyclization reaction of o-substituted anilines and 3-buten-2-one or their synthetically equivalent substances. As the reaction with 2-methoxyaniline, for example, the reaction described in DE518291 is known. As the reaction with o-toluidine, for example, the reaction described in Journal of Organic Chemistry (J. Org. Chem.), p.682, 1957 is known. As the reaction with 2-aminophenol, for example, the reaction described in Journal of American Chemical Society (J. Am. Chem. Soc.), p.3986, 1949 is known. As the reaction with 2-nitroaniline, for example, the reaction described in Nihon Kagaku Zassi (Journal of Japan Chemical Society), p.408, 1958 is known. Further, 4-methyl-8-nitroquinolines can also be obtained by treating lepidines with a mixed acid. For example, the reaction described in Journal of American Chemical Society (J. Am. Chem. Soc.), p.380, 1947 is known. As the reaction with 2-ethylaniline, for example, the reaction described in Tetrahedron Asymmetry, p.419, 1995 is known. As the reaction with ethyl 2-aminobenzoate, for example, the reaction described in DE518291 is known, and 4-methylquinoline-8-carboxylic acids are obtained by the reaction. As for 4-methylquinoline-8-carboxylic acids, the carboxyl group at the 8-position can be further converted. In order to synthesize 8-(2-benzoxazolyl)-4-methylquinolines, 2-aminophenol can be reacted, and in order to synthesize 8-(2-benzothiazolyl)-4-methylquinolines, 2-aminobenzenethiol can be reacted. For example, the reaction described in Journal of Heterocyclic Chemistry (J. Heterocycl. Chem.), p.1579, 1979 is known. In order to synthesize 4-methyl-8-aminoquinolines, 4-methyl-8-nitroquinolines can be reduced. For example, the reaction described in Journal of American Chemical Society (J. Am. Chem. Soc.), p.1524, 1946 is known. The above explanations were made for the syntheses of mono-substituted compounds of lepidines. However, if a further substituted compound is desired, the desired substance can be synthesized by combining these reactions.

As typical embodiments of the method of the present invention, the following two of embodiments, (1) and (2), can be mentioned.
(1) Measurement is performed for a multi-stranded nucleic acid in a solution.
(2) Measurement is performed by using a DNA array for a spot (gene) in which hybridization is caused on a solid phase carrier.

The term "measurement" used in the specification should be interpreted in its broadest sense including detection, quantification and the like, and the term should not be interpreted in any limitative way. Among the aforementioned embodiments, the embodiment in which detection and quantification are performed for a multi-stranded nucleic acid in a solution is extremely effective for use in simple measurement of a multi-stranded nucleic acid in a sample solution, as well as use as a method for measuring a target nucleic acid sequence in a sample nucleic acid using a nucleic acid amplification method of which typical example is PCR (polymerase chain reaction), and the optical measurement method of the present invention can be preferably used for such purposes.

When a multi-stranded nucleic acid in a solution is measured, preferred concentration of the aforementioned compound varies also depending on the concentration of multi-stranded nucleic acid to be detected. The concentration may usually be 1 x 10⁻¹⁰ mol/L to 1 x 10⁻⁸ mol/L, more preferably 1 x 10⁻⁹ mol/L to 1 x 10⁻⁴ mol/L, most preferably 5 x 10⁻⁸ mol/L to 1 x 10⁻⁵ mol/L. In order to attain contact of a multi-stranded nucleic acid and the aforementioned compound, a solution of sample nucleic acid and a solution of the compound of the present invention can be mixed, in general. However, it is also possible to add the aforementioned compound as solid to a solution of sample nucleic acid, or the compound of the present invention may be adsorbed on another medium and mixed with a sample nucleic acid solution. When the optical measurement is performed by bringing the aforementioned compound into contact with a multi-stranded nucleic acid, the compound may be brought into contact with the multi-stranded nucleic acid while the optical measurement is performed, or the optical detection may be performed after the compound is brought into contact with the multi-stranded nucleic acid. Preferably, the detection is performed within from 0.01 to 1000 seconds, more preferably from 0.1 to 600 seconds, after the multi-stranded nucleic acid and the aforementioned compound are brought into contact with each other.

pH of a solution for the measurement is preferably 2.0 to 10.0, more preferably 3.0 to 9.0, most preferably 4.0 to 8.0. It is also preferable to use a buffer for pH adjustment. Buffers are described in detail in "Tanpakusitu Koso no Kiso Jikken Ho (Fundamental Experimental methods for Proteins and Enzymes)", compiled by Sikiichi Horio, Nankodo, 1994 and the like. Preferably used buffers are potassium hydrogenphthalate/hydrochloric acid buffer, citrate/disodium phosphate buffer, citric acid/sodium citrate buffer, acetic acid/sodium acetate buffer, succinate/sodium hydroxide buffer, sodium maleate/sodium hydroxide buffer, phosphate buffer, imidazole/hydrochloric acid buffer, triethanolamine and hydrochloric acid/sodium hydroxide buffer, N-ethylmorpholine/hydrochloric acid buffer, Tris buffer, glycylglycine/sodium hydroxide buffer, diethanolamine/hydrochloric acid buffer, borate buffer, Britton-Robinson wide range buffer, GTA wide range buffer and the like. Concentration of the buffer is preferably 0.1 mM to 500 mM, more preferably 0.5 mM to 200mM, further preferably 1 mM to 50 mM. When a compound that can become colorless by protonation is used for the measurement, pH is preferably adjusted to pH at which the protonation occurs or pH lower than that level. A solution for performing the measurement is preferably an aqueous solution. Water-miscible solvents including alcohols such as methanol, ethanol, ethylene glycol, glycerol and diethylene glycol, dimethyl sulfoxide, dimethylformamide, dimethylacetamide, sulforane and the like may also be used in combination.

The optical measurement is preferably performed by the fluorescence detection method from a viewpoint of sensitivity. The measurement can be usually performed by using a fluorometer, and use of a method of passing an excitation light through a solution and measuring fluorescence from a direction perpendicular to the light is preferably used. A method of measuring a reflected light may also be used. Further, a method of spreading or spotting a sample solution on a solid phase carrier and then measuring fluorescence with a fluorescence laser scanner, CCD (charge coupled device) or the like after drying the solution or without drying the solution can also be used. Further, the measurement may be performed for each sample, or the measurement may be performed for many samples at one time by using a 96-well or 384-well microplate or the like.

In the aforementioned embodiment (2) using a DNA array, the method of the present invention can preferably be used for the measurement for a spot (gene) on a solid phase carrier in which hybridization occurs. In this embodiment, the probe nucleic acid may preferably be immobilized on a solid phase carrier, although the probe nucleic acid may exist in a solution. As the solid phase carrier, a carrier with low planarity having a rough surface may also be used. The material of the solid phase carrier is not particularly limited, and examples include glass, cement, ceramics such as pottery or new ceramics, polymers such as polyethylene terephthalate, cellulose acetates, polycarbonate of bisphenol A, polystyrene and polymethyl methacrylate, silicon, activated carbon, porous substances such as porous glass, porous ceramics, porous silicon, porous activated carbon, woven fabric, non-woven fabric, base paper, short fiber, and membrane filter. The shape of the solid phase carrier is not particularly limited, and the carrier may be in plate-like, sphere-like, fiber-like, rod-like shape or the like. The size of the solid phase carrier is not also particularly limited, and the carrier may have a size of from nanometer to centimeter order. The pore size of the porous substances is preferably, for example, in the range of from 2 to 1000 nm, most preferably in the range of from 2 to 500 nm. Where a plate-like solid phase carrier is used, the solid phase carrier preferably has a thickness in the range of from 100 to 2000 µm.

As the method for immobilizing a probe nucleic acid on a solid phase carrier, an appropriate method can be chosen depending on the type of the nucleic acid fragment and the type of the solid phase carrier (Protein, Nucleic acid and Enzyme, Vol. 43, No. 13, pp.2004-2011 (1998)). For example, where the nucleic acid fragment is cDNA or a PCR product, applicable methods include a method of electrostatically coupling the fragment by utilizing charge of DNA to a substrate subjected to a surface treatment with a cation such as polylysine, polyethylenimine, or polyalkylamine. To obtain stronger coupling, a method of irradiating a UV ray may also be used. A layer of hydrophilic polymer substance or the like having an electric charge or a layer comprising a crosslinking agent may further be provided on the surface-treated substrate. Further, depending on a type of the substrate, a hydrophilic polymer or the like can be contained in the substrate, and a substrate subjected to such a treatment can also be preferably used. By applying the surface treatment, electrostatic interaction between a hydrophobic substrate or weakly hydrophilic substrate and a nucleic acid fragment can be enhanced. Slide glass is preferably used as the substrate from viewpoints of easiness of the surface treatment and readiness of analysis.

When synthetic nucleotides are immobilized, applicable methods include a method of directly synthesizing nucleotides on a substrate, or a method of synthesizing an oligomer whose end is introduced beforehand with a functional group for forming a covalent bond, and then covalently bonding the oligomer to a surface-treated substrate. Examples of the functional group include an amino group, an aldehyde group, a mercapto group, biotin and the like. The method describe in Japanese Patent Laid-open Publication No. 2001-228152 can also be preferably used. As the substrate, glass or silicon is preferably used, and a known silane-coupling agent is preferably used for the surface treatment of glass or silicon. The nucleic acid fragment to be immobilized on the substrate may be a sample nucleic acid fragment to be detected, which is explained later. The following explanations will be made with a premise that the nucleic acid fragments immobilized on a substrate are nucleic acid fragments of which nucleotide sequences are known, and the nucleic acid fragments to be brought into contact with the nucleic acid analysis device are sample nucleic acid fragments.

DNA fragments can be classified into two groups depending on purposes. In order to investigate gene expression, polynucleotides such as cDNA, a part of cDNA and EST are preferably used. Functions of these polynucleotides may be unknown, and they are generally prepared by amplifying a DNA fragment by PCR using a cDNA library, genome library, or the whole genome as a template based on a sequence registered in a database (referred to as a "PCR product" hereinafter). DNA fragments that are not amplified by PCR can also be preferably used. Further, in order to investigate gene mutation or polymorphism, it is preferable to synthesize various oligonucleotides corresponding to mutation or polymorphism based on known sequences as a standard and use them. Further, for nucleotide sequence analysis, 4ⁿ (n is number of nucleotides) kinds of synthesized oligonucleotides are preferably used. The nucleotide sequence of the DNA fragment is preferably determined beforehand by an ordinary nucleotide sequencing method. The DNA fragment is preferably from 2- to 100-mer, most preferably from 20- to 80-mer.

Spotting of the DNA fragments on a solid phase carrier is preferably performed by, for example, preparing an aqueous liquid containing the DNA fragments by dissolving or dispersing the fragments in an aqueous medium, introducing this aqueous liquid into each well of a 96-well or 384-well plastic plate, and dropping the introduced aqueous liquid on a solid phase carrier surface by using a spotting device or the like. In order to prevent the DNA fragments from drying after the spotting, a substance having a high-boiling point may be added to the aqueous liquid in which the DNA fragments are dissolved or dispersed. As the substance having a high-boiling point, a substance is preferred that is dissolvable in the aqueous liquid in which the DNA fragments are dissolved or dispersed, that is free from inhibition of hybridization of the DNA fragments and the sample nucleic acid, and that is not viscous. Examples of such substance include glycerin, ethylene glycol, dimethyl sulfoxide, and low molecular hydrophilic polymers. Examples of the hydrophilic polymers include, polyacrylamide, polyethylene glycol, sodium polyacrylate and the like. The molecular weight of the polymer is preferably in the range of from 10³ to 10⁵. As the substance having a high-boiling point, glycerin or ethylene glycol is more preferably used, and glycerin is most preferably used. The concentration of the substance having a high-boiling point in the aqueous liquid of the DNA fragments is preferably in the range of from 0.1 to 2% by volume, most preferably in the range of from 0.5 to 1% by volume. Further, for the same purpose, it is also preferable to place the solid phase carrier after the spotting of the DNA fragments under conditions of a humidity of 90% or more and a temperature of from 25 to 50°C

After the DNA fragments are spotted on the solid phase carrier, a post-treatment by using ultraviolet rays, sodium borohydride, Schiff reagent or the like may be applied. For the post-treatment, plural kinds of treatments may be performed in combination, and a heat treatment and ultraviolet irradiation or the like are most preferably performed in combination. Incubation is also preferably carried out after the spotting. After the incubation, it is preferable to remove unimmobilized DNA fragments by washing.

The density of the DNA fragments is preferably in the range of from 10² to 10⁵ kinds/cm² on the solid phase carrier surface. The amount of the DNA fragments is preferably in the range of from 1 to 10⁻¹⁵ moles, and the mass is preferably a few ng or less. By the spotting, the aqueous liquid containing the DNA fragments is immobilized on the solid phase carrier surface in a shape of a dot. The shape of the dots is usually a substantially circular form. For a quantitative analysis of gene expression or analysis of a single base mutation, it is important that the dot shape remains unchanged. The distance between the dots is preferably in the range of from 0 to 1.5 mm, most preferably in the range of from 100 to 300 µm. The size of one dot is preferably in the range of from 50 to 300 µm in diameter. The volume of the aqueous liquid to be spotted is preferably in the range of from 100 pL to 1 µL, most preferably in the range of from 1 to 100 nL.

The lifetime of a solid phase carrier immobilized with DNA fragments, which is prepared through the aforementioned steps, is several weeks for a cDNA chip immobilized with cDNAs, and is still longer for an oligo DNA chip immobilized with oligo DNAs. These DNA chips can be used in monitoring of gene expression, nucleotide sequencing, mutation analysis, polymorphism analysis and the like. The detection principle consists of the optical measurement of a non-labeled nucleic acid based on the principle of the present invention.

When the method of the present invention is performed, another nucleic acid labeling method may be used in combination. As other labeling methods, RI methods and non-RI methods (fluorescence method, biotin method, chemiluminescence method etc.) are known, and the method of the present invention can be performed in combination with either kind of methods. As fluorescent substances, any of fluorescent substances that can bind to a base moiety of a nucleic acid can be used. For example, cyanine dyes (for example, Cy3, Cy5 etc. in the CyDye^{™} series), Rhodamine 6G reagent, N-acetoxy-N2-acetylaminofluorene (AAF) or AAIF (iodine derivative of AAF) can be used.

As the sample nucleic acid fragments, a sample containing a DNA fragment or RNA fragment of which sequence or function is unknown is preferably used. For the purpose of investigation of gene expression, the sample nucleic acid is preferably isolated from a cell or tissue sample of a eukaryote. When the sample is a genome, the sample nucleic acid can be isolated from any tissue samples except for erythrocytes. Any tissues except for erythrocytes may preferably be peripheral blood lymphocytes, skin, hair, sperm or the like. When the sample is mRNA, the sample is preferably extracted from a tissue sample in which mRNA is expressed. Although mRNA can be directly detected by the method of the present invention, cDNA prepared from mRNA by reverse transcription to take up labeled dNTP ("dNTP" refers to a deoxyribonucleotide having a base of adenine (A), cytosine (C), guanine (G) or thymine (T)) may also be used. As dNTP, dCTP is preferably used from a viewpoint of chemical stability. An amount of mRNA required for one hybridization varies depending on a liquid volume or a labeling method. The amount may preferably be a few µg or less. When DNA fragments on a DNA chip are oligo DNAs, the sample nucleic acid is preferably made into lower molecules beforehand. Since it is difficult to selectively extract mRNA from prokaryotic cells, it is preferable to label total RNA. For the purpose of investigation of mutation or polymorphism of genes, the sample nucleic acid fragments can also be obtained by performing PCR for the target region in the presence of suitable primers.

Hybridization can be performed by spreading the aqueous liquid dissolving or dispersing sample nucleic acid fragments on the DNA chip prepared as described above. Although the volume of the aqueous liquid to be spread is not particularly limited, it is preferably, for example, in the range of from 1 to 100 µL. Hybridization can be usually performed, for example, at a temperature in the range of room temperature to 70t for a reaction time in the range of from 1 to 20 hours. After completion of the hybridization, the chip is preferably washed with a mixed solution of a surfactant and a buffer to remove unreacted sample nucleic acid. As the surfactant, sodium dodecylsulfate (SDS) is preferably used. As the buffer, citrate buffer, phosphate buffer, borate buffer, Tris buffer, Good's buffer and the like can be used. Citrate buffer is most preferably used. When the aforementioned compound is allowed to coexist during the hybridization and interact with a hybrid multi-stranded nucleic acid, although the chip may be washed in the same manner as described above, optical detection can also be performed for the DNA chip as it is without particular washing, and it constitute a preferred embodiment of the present invention.

Hybridization using a DNA array is characterized in that a very small amount of a sample nucleic acid is used. For this reason, optimal conditions for hybridization should be applied depending on lengths of DNA fragments immobilized on a solid phase carrier and the type of the sample nucleic acid. For a gene expression analysis, it is preferable to perform hybridization for a long period of time so that low expression genes can also be satisfactorily detected. For detection of single base mutation, it is preferable to perform hybridization for a short period of time.

Although the solvent used when contacting the aforementioned compound to a hybrid multi-stranded nucleic acid is not particularly limited, usually, water or any of various buffers as well as a water-miscible organic solvent can be suitably used. Preferred examples of the water-miscible organic solvent include, for example, dimethyl sulfoxide, dimethylformamide, methanol, ethanol, ethylene glycol, glycerin and the like. Further, it is also preferable to mix water or buffer with these organic solvents for use. In the measurement of a multi-stranded nucleic acid of the present invention, preferred concentration of the aforementioned compound varies also depending on the concentration of multi-stranded nucleic acid to be detected. However, the concentration may usually be from 1 x 10⁻¹⁰ mol/L to 1 x 10⁻⁸ mol/L, more preferably from 1 x 10⁻⁹ mol/L to 1 x 10⁻⁴ mol/L, most preferably from 5 x 10⁻⁸ mol/L to 1 x 10⁻⁵ mol/L.

When the aforementioned compound is contacted with a multi-stranded nucleic acid by using a DNA array, a method of spreading a solution of the aforementioned compound on the array is most preferably used. However, a method of immersing a DNA array after hybridization into a solution of the aforementioned compound can also be preferably used. Further, a method of making the aforementioned compound existing at the time of the hybridization can also be employed. When the optical measurement is performed by contacting the aforementioned compound with a multi-stranded nucleic acid, the detection is preferably started within from 0.01 to 1000 seconds, more preferably from 0.1 to 600 seconds, after the multi-stranded nucleic acid and the aforementioned compound are brought into contact with each other.

pH of a solution for the optical measurement is preferably from 2.0 to 10.0, more preferably from 3.0 to 9.0, most preferably from 4.0 to 8.0. It is also preferable to use a buffer for pH adjustment. Although buffers are described in detail in "Tanpakusitu Koso no Kiso Jikken Ho (Fundamental Experimental methods for Proteins and Enzymes)", compiled by Sikiichi Horio, Nankodo, 1994 and the like, preferably used are potassium hydrogenphthalate/hydrochloric acid buffer, citrate/disodium phosphate buffer, citric acid/sodium citrate buffer, acetic acid/sodium acetate buffer, succinate/sodium hydroxide buffer, sodium maleate/sodium hydroxide buffer, phosphate buffer, imidazole/hydrochloric acid buffer, triethanolamine and hydrochloric acid/sodium hydroxide buffer, N-ethylmorpholine/hydrochloric acid buffer, Tris buffer, glycylglycine/sodium hydroxide buffer, diethanolamine/hydrochloric acid buffer, borate buffer, Britton-Robinson wide range buffer, GTA wide range buffer and the like. Concentration of the buffer is preferably from 0.1 mM to 500 mM, more preferably from 0.5 mM to 200mM, further preferably from 1 mM to 50 mM.

After the compound is brought into contact with the hybrid multi-stranded nucleic acid, washing may be performed to remove an excess amount of the compound. The washing can be performed by a procedure similar to the washing after the hybridization. Optical detection is achievable without performing the washing in the methods of the present invention as described above, and omission of the washing is also preferred.

Where the hybrid multi-stranded nucleic acid is detected in a solution system, a usual fluorometer can also be used for the optical detection, or a fluorescence scanner is also preferably used from viewpoints of ability to simultaneously detect a large number of nucleic acids and sensitivity. Further, quantification of fluorescence may be carried out by a conventional method by using a fluorescence laser scanner for the substrate dried after hybridization or in the presence of an aqueous solvent, or the measurement may be performed by the cooled CCD (charge coupled device) method or the fluorescence laser scanner method for a substrate covered with cover glass to prevent the substrate from drying.

The compounds of the present invention represented by the formula (V) are useful as fluorescent dyes for nucleic acid measurement. Further, they can also be used as fluorescent dyes having higher selectivity in a method of distinguishing a double-stranded nucleic acid from a single-stranded nucleic acid in nucleic acid measurement techniques. However, the use of the compounds of the present invention is not limited to these examples, and they can be used for various applications.

### Examples

The present invention will be explained more specifically by means of examples. However, the scope of the present invention is not limited to the following examples.

### [Synthesis Example 1] Synthesis of Dye (D-1)

### (1-1) Synthesis of 2-mercaptooxazolo[4,5-b]pyridine

The synthesis was carried out by referring to the method described in Journal of Organic Chemistry (J. Org. Chem.), vol. 60, p.5721 (1995). In an amount of 10 g of 2-amino-3-hydroxypyridine, 20 g of potassium xanthate and 200 mL of ethanol were mixed and refluxed for 15 hours. Then, the solvent was evaporated under reduced pressure. The residue was dissolved in 80 mL of water and added with acetic acid, and crystals were deposited. The crystals were collected by filtration, washed with water and dried. Yield: 11 g.

### (1-2) Synthesis of 2-methylthiooxazolo[4,5-b]pyridine

In an amount of 3.9 g of a starting material (2-mercaptooxazolo[4,5-b]pyridine) was dissolved in dimethylformamide and added with 3.0 g of potassium t-butoxide. Then, the reaction mixture was added with 2 mL of methyl iodide and stirred at room temperature for 30 minutes. The starting material substantially disappeared, and then the reaction mixture was added with water and extracted twice with ethyl acetate. The organic phase was dried over anhydrous magnesium sulfate, and the solvent was evaporated under reduced pressure to obtain the desired substance as oil. The resulting oil was left stand to allow crystallization.

### (1-3) Synthesis of 4-(2-carbamoylethyl)-2-methylthiooxazolo[4,5-b]pyridinium bromide

In an amount of 1 g of 2-methylthiooxazolo[4,5-b]pyridine was added with 3 g of 3-bromopropionic acid amide, heated to 110°C for 1 hour and then cooled. The reaction mixture was added with ethyl acetate, and the components dissolved in the ethyl acetate were removed by decantation. This procedure was repeated 3 times, and then the residue was used for the following reaction without treatment.

### (1-4) Synthesis of 1,4-dimethylquinolinium p-toluenesulfonate

In an amount of 10 g of 4-methylquinoline (lepidine) and 19.5 g of methyl p-toluenesulfonate were mixed and allowed to react at 150°C for 1 hour. The reaction mixture was added with ethyl acetate and stirred, and crystals deposited. The crystals were collected by filtration and washed ethyl acetate. The resulting substance had hygroscopic property.

### (1-5) Synthesis of Dye (D-1)

In an amount of 100 mg of the 4-(2-carbamoylethyl)-2-methylthiooxazolo[4,5-b]pyridinium bromide synthesized in Synthesis Example (1-3) and 40 mg of the 1,4-dimethylquinolinium p-toluenesulfonate synthesized in Synthesis Example (1-4) were dissolved in 4 mL of dimethylformamide and added with 0.5 mL of triethylamine at room temperature. After a reaction was allowed at a room temperature for 1 hour, the reaction mixture was added with 0.5 mL of triethylamine and allowed to react at 80°C for 1 hour. The reaction mixture was subjected without treatment to silica gel column chromatography, and yellow fractions were collected to obtain the target substance. The purification by chromatography was repeated to obtain 2 mg of the desired substance.

### [Synthesis Example 2] Synthesis of Dye (D-2)

### (2-1) Synthesis of 4-methyl-1-(3-trimethylammoniopropyl)quinolinium dibromide

In an amount of 5 g of (3-bromopropyl)trimethylammonium bromide (Aldrich Co.) and 10 g of 4-methylquinoline were mixed and allowed to react at 120°C for 3 hours. After the reaction mixture was cooled, the reaction mixture was added with ethyl acetate, and the substances dissolved in the ethyl acetate were removed by decantation. This procedure was repeated 3 times, and then the residue was used for the following reaction without further purification.

### (2-2) Synthesis of Dye (D-2)

In an amount of 100 mg of the 4-(2-carbamoylethyl)-2-methylthiooxazolo[4,5-b]pyridinium bromide synthesized in Synthesis Example (1-3) and 50 mg of the 4-methyl-1-(3-trimethylammoniopropyl)quinolinium dibromide synthesized in Synthesis Example (2-1) were dissolved in 5 mL of dimethylformamide and added with 0.5 mL of triethylamine at room temperature. After a reaction was allowed at room temperature for 1 hour, the reaction mixture was added with 0.5 mL of triethylamine and allowed to react at 80°C for 1 hour. The reaction mixture was added with ethyl acetate, and the substances dissolved in the ethyl acetate were removed by decantation. Then, the residue was dissolved in methanol and purified 3 times by column chromatography using Sephadex LH-20 (elution solvent: methanol) and once by short silica gel column chromatography (elution solvent: chloroform/methanol = 2/1) to obtain 4 mg of the desired substance.

### [Synthesis Example 3] Synthesis of Dye (D-3)

### (3-1) Synthesis of 4-(2-(N-acetyl-N-phenylamino)vinyl)-1-(3-trimethylammoniopropyl)-quinolinium dibromide

In an amount of 500 mg of the 4-methyl-1-(3-trimethylammoniopropyl)-quinolinium dibromide synthesized in Synthesis Example (2-1) was added with 2 g of 1,3-diphenylformamidine, added with 10 mL of acetic anhydride and allowed to react at 120°C for 3 hours. After the reaction mixture was cooled, the mixture was added with ethyl acetate, and the substances dissolved in the ethyl acetate were removed. This procedure was repeated 3 times, and the residue was used for the following reaction without further purification.

### (3-2) Synthesis of 7-(2-methoxyethoxymethyl)-2,3,3-trimethyl-3H-pyrrolo[2,3-b]pyridinium chloride

### (3-2-1) Synthesis of 2,3,3-trimethyl-3H-pyrrolo[2,3-b]pyridine (7-aza-2,3,3-trimethylindolenine)

In an amount of 200 g of 2-hydrazinopyridine and 300 mL of toluene are mixed, added with 250 g of 3-methyl-2-butanone and refluxed by heating for 30 minutes. Then, the solvent was completely concentrated to obtain oil. This oil was added with 10 g of zinc chloride, heated to 215°C and allowed to react for 5 hours. The reaction mixture was cooled, then added with 400 mL of water and extracted 4 times with 300 mL of chloroform, and the organic phase was dried over anhydrous sodium sulfate. Then, the solvent was evaporated under reduced pressure, and the residue was distilled under reduced pressure (112-135°C/0.2 mmHg). The distilled component was recrystallized from 1.5 L of hexane to obtain the desired substance. Yield: 70.0 g, 23.8%.

### (3-2-2) Synthesis of 7-(2-methoxyethoxymethyl)-2,3,3-trimethyl-3H-pyrrolo[2,3-b]pyridinium chloride

In an amount of 1 g of the 2,3,3-trimethyl-3H-pyrrolo[2,3-b]pyridine synthesized in Synthesis Example (3-2-1) was mixed with 3 g of 2-methoxyethoxymethyl chloride and allowed to react at 90°C for 1 hour. The reaction mixture was cooled and then added with ethyl acetate, and the components dissolved in the ethyl acetate were removed by decantation. The residue was used for the following reaction without further purification.

### (3-3) Synthesis of Dye (D-3)

In an amount of 50 mg of the 4-(2-(N-acetyl-N-phenylamino)vinyl)-1-(3-trimethylammoniopropyl)quinolinium dibromide synthesized in Synthesis Example (3-1) and 100 mg of the 7-(2-methoxyethoxymethyl)-2,3,3-trimethyl-3H-pyrrolo[2,3-b]pyridinium chloride synthesized in Synthesis Example (3-2) were dissolved in 5 mL of dimethylformamide, added with 0.5 mL of acetic anhydride, then added with 1 mL of triethylamine and allowed to react at room temperature for 2 hours. The reaction mixture was added with ethyl acetate, and the substances dissolved in the ethyl acetate were removed by decantation. Then, the residue was purified by column chromatography using Sephadex LH-20 (elution solvent: methanol) to obtain a crude product of precursor of Dye (D-3) in blue color. The crude product of precursor was added with 3% hydrobromic acid and allowed to react at 80°C for 2 hours, and then the reaction mixture was evaporated under reduced pressure. The residue was purified 3 times by column chromatography using Sephadex LH-20 (elution solvent: methanol) and once by silica gel column chromatography (elution solvent: chloroform/methanol = 3/1) to obtain 3 mg of the desired substance.

### [Synthesis Example 4] Synthesis of Dye (D-4)

### (4-1) Synthesis of 2-methyloxazolo[4,5-b]pyridine

In an amount of 15 g of 2-amino-3-hydroxypyridine (Aldrich Co.) was added with 80 mL of ethyl orthoacetate and a catalytic amount of p-toluenesulfonic acid monohydrate and allowed to react at 120°C for 4 hours. The reaction mixture was cooled and then added with 2 mL of triethylamine, and then the solvent was evaporated under reduced pressure. The residue was purified by silica gel column chromatography (elution solvent: hexane/ethyl acetate = 2/1) to obtain the desired substance as crystals.

### (4-2) Synthesis of Dye (D-4)

Dye (D-4) was obtained by using the 2-methyloxazolo[4,5-b]pyridine synthesized in (4-1) according to the method described in Synthesis Example 3.

### [Synthesis Example 5] Synthesis of Dye (D-5)

### (5-1) Synthesis of 6-bromo-2-mercaptooxazolo[4,5-b]pyridine

In an amount of 4 g of 2-amino-5-bromo-3-hydroxypyridine, 8 g of potassium xanthate and 50 mL of ethanol were mixed and refluxed by heating for 10 hours. The solvent was evaporated under reduced pressure, and the residue was added with 40 mL of water and dissolved. The solution was added with acetic acid, and crystals deposited. The crystals were collected by filtration under reduced pressure, washed with water and dried. Yield: 4.0 g, 81.8%.

### (5-2) Synthesis of 6-bromo-2-methylthiooxazolo[4,5-b]pyridine

In an amount of 2 g of the 5-bromo-2-mercaptooxazolo[4,5-b]pyridine synthesized in Synthesis Example (5-1) was added with 15 mL of dimethylformamide and added with 2 mL of triethylamine for dissolution. Then, the solution was added with 1 mL of methyl iodide and allowed to react at room temperature for 1 hour. After completion of the reaction, the reaction mixture was added with water, and the deposited crystals were collected by filtration under reduced pressure, washed with water and dried to obtain the desired substance. Yield: 2 g, 94.3%.

### (5-3) Synthesis of 1-(3-ethoxycarbonylpropyl)-4-methylquinolinium bromide

In an amount of 19.5 g of 4-bromobutylic acid ethyl ester and 14.3 g of lepidine were mixed and allowed to react at 130°C for 2 hours. After the reaction mixture was cooled, the mixture was added with ethyl acetate, and the substances dissolved in the ethyl acetate were removed. This procedure was repeated to obtain the desired substance as oil. The resulting oil was used for the following reaction without further purification.

### (5-4) Synthesis of Dye (D-5)

Dye (D-5) was synthesized by using the starting materials synthesized in Synthesis Examples (5-2) and (5-3) according to the method described in Synthesis Example 1.

### [Synthesis Example 6] Synthesis of Dye (D-6)

### (6-1) Synthesis of 6-methyl-2-mercaptothiazolo[4,5-b]pyridine

The synthesis was performed according to the method described in Journal of Medicinal Chemistry (J. Med. Chem.), vol. 37, p.248, 1994. In an amount of 10 g of 2-amino-3-bromo-5-methylpyridine, 120 mL of N-methylpyrolidone and 16.7 g of potassium xanthate were mixed and heated to 170°C for 3.5 hours. The reaction mixture first became black and then gradually became dark reddish brown. After completion of the reaction, the reaction mixture was cooled to room temperature. The reaction mixture was made acidic with acetic acid, and crystals of the desired substance (grayish yellow) were obtained. The crystals were collected by filtration, washed with water and dried. Yield: 8.7 g, 89.2%.

### (6-2) Synthesis of 6-methyl-2-methylthiothiazolo[4,5-b]pyridine

In an amount of 2 g of the starting material thiol was dissolved in 10 mL of dimethylformamide and added with 2 mL of triethylamine. Then, the reaction mixture was added with 1.5 mL of methyl iodide, and a reaction was allowed at room temperature for 30 minutes. The reaction mixture was added with 100 mL of water, and the deposited crystals were collected by filtration, washed with water and dried to obtain the desired substance. Yield: 1.7 g, 78.9%

### (6-3) Synthesis of Dye (D-6)

Dye (D-6) was synthesized by using the starting materials synthesized in Synthesis Example (6-2) and Synthesis Example (5-2) according to the method described in Synthesis Example 1.

### [Synthesis Example 7] Synthesis of Dye (D-15)

### (7-1) Synthesis of 4-dimethylaminobutylic acid ethyl ester

In a volume of 250 mL of 50% aqueous dimethylamine solution and 200 mL of ethanol were mixed and added dropwise with 120 g of 4-bromobutylic acid ethyl ester while the mixture was kept at 10°C or lower. After completion of the addition, a reaction was allowed at room temperature for 2 hours. Then, the temperature was increased to 60°C, and a reaction was allowed for 2 hours. The solvent was concentrated to an about half volume, and the residue was made basic by addition of sodium hydroxide while cooling with ice water and added with 400 mL of ethyl acetate for extraction. The organic phase was extracted 4 times with 150 mL of 4 M hydrochloric acid, and the organic phase was discarded. The hydrochloric acid extract was cooled with ice water, made basic by addition of sodium hydroxide and extracted 3 times with 200 mL of ethyl acetate. The organic phase was dried over potassium carbonate and concentrated to obtain the desired substance. Yield: 45 g.

### (7-2) Synthesis of (6-bromohexyl)(3-ethoxycarbonylpropyl)dimethylammonium bromide

In an amount of 15 g of the 4-dimethylaminobutylic acid ethyl ester synthesized in Synthesis Example (7-1) was mixed with 50 g of 1,6-dibromohexane and left overnight at room temperature. The crystals deposited, and then the reaction mixture was added with ethyl acetate to triturate the crystals and filtered under reduced pressure to obtain the desired substance as hygroscopic crystals. Yield: 21 g.

### (7-3) Synthesis of 1-(6-(dimethyl(3-ethoxycarbonylpropyl)ammonio)hexyl)-4- , methylquinolinium dibromide

In an amount of 8 g of 6-bromohexyldimethyl(3-ethoxycarbonylpropyl)-ammonium bromide and 12 g of lepidine were mixed and allowed to react at 125°C for 1 hour. The reaction mixture was cooled, then added with ethyl acetate and stirred, and the substances dissolved in the ethyl acetate were removed by decantation. Then, the residue was dissolved in methanol and purified by using Sephadex LH-20 (eluent: methanol) to obtain the desired substance as oil. Yield: 6 g.

### (7-4) Synthesis of Dye (D-15)

Dye (D-15) was synthesized by using the starting materials synthesized in Synthesis Example (7-3) and Synthesis Example (1-4) according to the synthetic method described in Synthesis Example 1.

### [Synthesis Example 8] Synthesis of Dye (D-16)

### (8-1) Synthesis of 5-fluoro-4-methylquinoline p-toluenesulfonic acid salt

Australian Journal of Chemistry (Aust. J. Chem.), vol. 45, p.1119, 1992 and Journal of American Chemical Society (J. Am. Chem. Soc.), vol. 67, p.86, 1945 were referred to. In an amount of 33.2 g (0.225 mol) of 3-fluoroaniline hydrochloride, 97.2 g (0.36 mol) of ferric chloride hexahydrate, 3.6 g of anhydrous zinc chloride and 160 mL of 95% ethanol were mixed and slowly added dropwise with 0.18 mol of methyl vinyl ketone on a water bath at 60-65°C. After completion of the addition, the reaction mixture was refluxed by heating for 2 hours and left overnight. Almost all of the solvent was evaporated, and the residue was made alkaline with 25% sodium hydroxide. The residue was filtered with Cerite and washed with ethyl acetate. The filtrate was separated, and the organic phase was subjected to purification by silica gel column chromatography. The obtained mixture of isomers (5-fluoro-4-methylquinoline and 7-fluoro-4-methylquinoline) was distilled. The initially distilled fraction having a high content of 5-fluoro-4-methylquinoline was dissolved in ethyl acetate, added with a solution of p-toluenesulfonic acid monohydrate in acetone and cooled with ice water to obtain crystals of 5-fluoro-4-methylquinoline p-toluenesulfonic acid salt. Yield: 1.7 g.

Further, 7-fluoro-4-methylquinoline was also isolated as crystals of p-toluenesulfonic acid salt.

### (8-2) Synthesis of (4-bromobutyl)(3-ethoxycarbonylpropyl)dimethylammonium bromide

In an amount of 15 g of the 4-dimethylaminobutylic acid ethyl ester synthesized in Synthesis Example (7-1) was mixed with 50 g of 1,4-dibromobutane and left overnight at room temperature. The crystals deposited, and then the reaction mixture was added with ethyl acetate to crumble the crystals and filtered under reduced pressure to obtain the desired substance. Yield: 30 g.

### (8-3) Synthesis of 1-(4-(dimethyl(3-ethoxycarbonylpropyl)ammonio)butyl)-5-fluoro-4-methylquinolinium dibromide

In an amount of 1 g of 4-bromobutyldimethyl(3-ethoxycarbonylpropyl)-ammonium bromide and 0.1 g of 5-fluoro-4-methylquinoline p-toluenesulfonic acid salt were mixed, added with 0.2 mL of triethylamine and allowed to react at 130°C for 1 hour. The reaction mixture was cooled, then added with ethyl acetate and stirred, and the substances dissolved in the ethyl acetate were removed by decantation. Then, the residue was dissolved in methanol and purified by using Sephadex LH-20 (eluent: methanol) to obtain the desired substance as oil.

### (8-4) Synthesis of Dye (D-16)

Dye (D-16) was obtained by using the compounds synthesized in Synthesis Example (8-3) and Synthesis Example (6-2) as the starting materials according to the same synthesis method as described in Synthesis Example 1.

### [Synthesis Example 9] Synthesis of Dye (D-17)

### (9-1) Synthesis of 5-chloro-4-methylquinoline

Australian Journal of Chemistry (Aust. J. Chem.), vol. 45, p.1119, 1992 and Journal of American Chemical Society (J. Am. Chem. Soc.), vol. 67, p.86, 1945 were referred to. In an amount of 40.0 g (0.225 mol) of 3-chloroaniline hydrochloride, 97.2 g (0.36 mol) of ferric chloride hexahydrate, 3.6 g of anhydrous zinc chloride and 160 mL of 95% ethanol were mixed and slowly added dropwise with 12.6 g (0.18 mol) of methyl vinyl ketone on a water bath at 60-65°C. After completion of the addition, the reaction mixture was refluxed by heating for 2 hours and left overnight. Almost all of the solvent was evaporated, and the residue was made alkaline with 25% sodium hydroxide. The residue was filtered with Cerite and washed with ethyl acetate, and the filtrate was separated. The organic phase contained 5-chloro-4-methylquinoline and 7-chloro-4-methylquinoline, and the mixture was subjected to purification by silica gel column chromatography (elution solvent: hexane/ethyl acetate = 9/1) to separate the compounds.

### (9-2) Synthesis of 1-(4-(dimethyl(3-ethoxycarbonylpropyl)ammonio)butyl)-5-chloro-4-methylquinolinium dibromide

In an amount of 1 g of 4-bromobutyldimethyl(3-ethoxycarbonylpropyl)-ammonium bromide (Synthesis Example 8-2) and 0.1 g of 5-chloro-4-methylquinoline were mixed and allowed to react at 130°C for 1 hour. The reaction mixture was cooled, then added with ethyl acetate and stirred, and the components dissolved in the ethyl acetate were removed by decantation. The residue was dissolved in methanol and purified by using Sephadex LH-20 (eluent: methanol) to obtain the desired substance as oil.

### (9-3) Synthesis of Dye (D-17)

Dye (D-17) was obtained by using the compounds synthesized in Synthesis Example (9-2) and Synthesis Example (6-2) as the starting materials according to the same synthesis method as the method described in Synthesis Example 1.

### [Synthesis Example 10] Synthesis of Dye (D-18)

### (10-1) Synthesis of 4-methyl-1-phenyl-1,2-dihydroquinolin-2-one

In a volume of 150 mL of concentrated sulfuric acid was added portionwise with 60 g of N,N-diphenylacetoacetamide at 5-10°C. After substantially complete dissolution was attained, the solution was left overnight at room temperature. After completion of the reaction, the reaction mixture was poured into ice water, added with 200 mL of ethyl acetate and stirred, and crystals deposited. The crystals were collected by filtration and dried to obtain the desired substance.

### (10-2) Synthesis of 4-methyl-1-phenyl-1,2-dihydroquinoline-2-thione

In an amount of 10 g of 4-methyl-1-phenyl-1,2-dihydroquinolin-2-one (0.0425 mol) and 9 g of Lawesson's reagent were mixed in toluene and refluxed by heating. After the starting materials disappeared, the reaction mixture was subjected without treatment to silica gel column chromatography. Elution was performed with chloroform, and the product was recrystallized from hexane/ethyl acetate.

### (10-3) Synthesis of 2-(4-(dimethyl(3-ethoxycarbonylpropyl)ammonio)butylthio)-4-methyl-1-phenylquinolinium dibromide

In an amount of 0.5 g of the 4-methyl-1-phenyl-1,2-dihydroquinoline-2-thione synthesized in Synthesis Example (10-2) and 1 g of the 4-bromobutyldimethyl(3-ethoxycarbonylpropyl)ammonium bromide synthesized in Synthesis Example (8-2) were mixed and allowed to react at 130°C for 1 hour. The reaction mixture was cooled and then added with ethyl acetate, and the substances dissolved in the ethyl acetate were removed. The residue was dissolved in methanol and purified by using Sephadex LH-20 (eluent: methanol).

### (10-4) Synthesis of Dye (D-18)

Dye (D-18) was synthesized by using the starting materials synthesized in Synthesis Example (6-2) and Synthesis Example (10-3) according to the method described in Synthesis Example 1.

### [Synthesis Example 11] Synthesis of Dye (D-19)

### (11-1) Synthesis of Dye (D-19)

Dye (D-19) was synthesized by using the compounds synthesized in Synthesis Example (7-3) and Synthesis Example (6-2) as the starting materials according to the method described in Synthesis Example 1.

### [Synthesis Example 12] Synthesis of Dye (D-23)

### (12-1) Synthesis of 3-chloro-4-methylquinoline

The synthesis was performed by referring to Synthesis, p.798, 1976. That is, 1.31 g (10 mmol) of 3-methylindole, 50 mg of benzyltrimethylammonium chloride and 3.2 mL of a 1:1 mixture of chloroform (ethanol free)/benzene were vigorously stirred at 409°C and added dropwise with a solution of 3 g of sodium hydroxide in 6 mL of water over 15 minutes. After a reaction was allowed for 8 hours, the reaction mixture was diluted with ethyl acetate and water and extracted with 2 N hydrochloric acid. This acidic solution was made into weakly basic with concentrated sodium hydroxide and extracted with ethyl acetate. The organic phase was dried over anhydrous sodium sulfate, and the solvent was evaporated to obtain the desired substance as a crude product. The product was further purified by short silica gel column chromatography to obtain the desired substance.

### (12-2) Synthesis of 1-(6-(dimethyl(3-ethoxycarbonylpropyl)ammonio)hexyl)-3-chloro-4-methylquinolinium dibromide

In an amount of 1 g of 6-bromohexyldimethyl(3-ethoxycarbonylpropyl)-ammonium bromide (Synthesis Example 7-2) and 0.1 g of 3-chloro-4-methylquinoline (Synthesis Example 12-1) were mixed and allowed to react at 130°C for 1 hour. The reaction mixture was cooled, then added with ethyl acetate and stirred, and the substances dissolved in the ethyl acetate were removed by decantation. The residue was dissolved in methanol and purified by using Sephadex LH-20 (eluent: methanol) to obtain the desired substance as oil.

### (12-3) Synthesis of Dye (D-23)

Dye (D-23) was synthesized by using the compounds synthesized in Synthesis Example (12-2) and Synthesis Example (6-2) as the starting materials according to the method described in Synthesis Example 1.

### [Synthesis Example 13] Synthesis of Dye (D-25)

### (13-1) Synthesis of 2,3,3-trimethyl-3H-pyrrolo[2,3-b]pyridine-5-carboxylic acid ethyl ester

### (13-1-1) Synthesis of 2-hydrazinopyridine-5-carboxylic acid

In an amount of 50 g of 6-chloronicotinic acid was mixed with 300 mL of n-butanol, added with 80 g of hydrazine monohydrate and refluxed by heating for 10 hours. The reaction mixture was cooled and poured into 500 mL of diluted hydrochloric acid. The deposited crystal was collected by filtration, washed with water and dried to obtain the desired substance. Yield: 44.2 g, 90.9%.

### (13-1-2) Synthesis of 2,3,3-trimethyl-3H-pyrrolo[2,3-b]pyridine-5-carboxylic acid

In an amount of 30 g of 3-hydrazinopyridine-5-carboxylic acid and 30 g of 3-methyl-2-butanone were mixed, added with 120 mL of ethylene glycol and allowed to react at 100°C for 1 hour. Under reduced pressure, excess 3-methyl-2-butanone was evaporated, and then the reaction mixture was heated and refluxed for 5 hours. The reaction mixture was cooled and then subjected without treatment to purification by silica gel column chromatography, and a fraction colored with a coloring solution (p-anisaldehyde/ethanol/sulfuric acid = 5/90/5 (volume percent)) in TLC was collected. The fraction contained a mixture of the desired substance and ethylene glycol ester of the desired substance, and the mixture was subjected to hydrolysis with 1 N sodium hydroxide as the mixture and neutralized to obtain the desired substance as crystals. Further, about 40% of the desired substance was collected as a hydrazone produced from the starting material hydrazine and 3-methyl-2-butanone. Yield: 1.8 g, 4.5%.

### (13-1-3) Synthesis of 2,3,3-trimethyl-3H-pyrrolo[2,3-b]pyridine-5-carboxylic acid ethyl ester

In an amount of 1 g of 2,3,3-trimethyl-3H-pyrrolo[2,3-b]pyridine-5-carboxylic acid, 1.5 g of p-toluenesulfonic acid monohydrate and 100 mL of ethanol were mixed, heated and allowed to react for 8 hours, while the solvent was evaporated little by little and ethanol was added in a suitable amount. The reaction mixture was cooled, and then the solvent was evaporated to an about 70% volume. The residue was added with water and ethyl acetate for extraction. The organic phase was washed with aqueous sodium carbonate and then concentrated. The residue was purified by silica gel column chromatography in a short column to obtain the desired substance. Yield: 0.8 g, 70.3%.

### (13-2) Synthesis of 4-(2-(N-acetyl-N-phenylamino)vinyl)-1-(6-(dimethyl(3-ethoxycarbonylpropyl)ammonio)hexyl)quinolinium dibromide

The desired substance was obtained by using the 1-(6-(dimethyl(3-ethoxycarbonylpropyl)ammonio)hexyl)-4-methylquinolinium dibromide synthesized in Synthesis Example (7-3) according to the same synthesis method as described in the Synthesis Example (3-1).

### (13-3) Synthesis of 5-ethoxycarbonyl-7-(2-methoxyethoxymethyl)-2,3,3-trimethyl-3H-pyrrolo[2,3-b]pyridinium chloride

In an amount of 0.1 g of the 2,3,3-trimethyl-3H-pyrrolo[2,3-b]pyridine-5-carboxylic acid ethyl ester synthesized in Synthesis Example (13-1) and 0.8 g of 2-methoxyethoxymethyl chloride were mixed and allowed to react at 110°C for 1 hour. The reaction mixture was cooled and then added with ethyl acetate, and the substances dissolved in the ethyl acetate were removed by decantation. The residue was used for the following reaction without further purification.

### (13-4) Synthesis of Dye (D-25)

Dye (D-25) was obtained by using the compounds synthesized in Synthesis Example (13-2) and Synthesis Example (13-3) as the starting materials according to the method described in the Synthesis Example (3-3).

### [Synthesis Example 14] Synthesis of Dye (D-27)

### (14-1) Synthesis of 6-bromo-2-methylthiazolo[4,5-b]pyridine

In an amount of 25.2 g of 2-amino-3,5-dibromopyridine was suspended in 500 mL of acetonitrile, added with 50 mL of acetic anhydride and refluxed by heating for 3 hours. The reaction mixture was cooled and then added with water, and the deposited crystals were collected by filtration. The crystals were dried, then suspended in 300 mL of dioxane and added with 6 g of sodium hydride. The suspension was refluxed by heating for 4 hours, then cooled and added with 20 mL of ethanol. The solvent was evaporated, and the residue was purified by silica gel column chromatography to obtain the desired substance.

### (14-2) Synthesis of 1-(4-(dimethyl(3-ethoxycarbonylpropyl)ammonio)butyl)-4-methylquinolinium dibromide

In an amount of 1 g of 4-bromobutyldimethyl(3-ethoxycarbonylpropyl)-ammonium bromide and 2 g of 4-methylquinoline (lepidine) were mixed and allowed to react at 130°C for 1 hour. The reaction mixture was cooled, then added with ethyl acetate and stirred, and the substances dissolved in the ethyl acetate were removed by decantation. The residue was purified by using Sephadex LH-20 (eluent: methanol) to obtain the desired substance as crystals.

### (14-3) Synthesis of 4-(4-(N-acetyl-N-phenylamino)-1,3-butadiene-1-yl)-1-(4-(dimethyl(3-ethoxycarbonylpropyl)ammonio)butyl)-4-methylquinolinium dibromide

In an amount of 0.5 g of the 1-(4-(dimethyl(3-ethoxycarbonylpropyl)-ammonio)butyl)-4-methylquinolinium dibromide synthesized in Synthesis Example (14-2) was added with 2 g of malonaldehyde dianilide hydrochloride, mixed with 5 mL of acetic anhydride and allowed to react at 110°C for 2 hours. The reaction mixture was cooled and then added with ethyl acetate, and the components dissolved in the ethyl acetate were removed by decantation. The residue was used for the following reaction without further purification.

### (14-3) Synthesis of Dye (D-27)

Dye (D-27) was synthesized by using the compounds shown in Synthesis Examples (14-1) and (14-3) as the starting materials according to the same method as described in Synthesis Example 3.

### [Synthesis Example 15] Synthesis of Dye (D-35)

### (15-1) Synthesis of 5-acetylamino-2-mercaptooxazolo[4,5-b]pyridine

### (15-1-1) Synthesis of 5-amino-3-benzyloxy-2-nitropyridine

The synthesis was performed by referring to the method described in Journal of Organic Chemistry (J. Org. Chem), vol. 57, p.4784, 1992. That is, the synthesis was performed by allowing a nitrogen anion of N,N-tetramethylenethiocarbamoyl-sulfenamide to react on 3-benzyloxy-2-nitropyridine in the presence of a strong base.

### (15-1-2) Synthesis of 5-acetylamino-3-benzyloxy-2-nitropyridine

In an amount of 3 g of 5-amino-3-benzyloxy-2-nitropyridine (Synthesis Example 15-1-1) was suspended in 30 mL of acetic anhydride, added with a catalytic amount of concentrated sulfuric acid and allowed to react at 70°C for 1 hour. After completion of the reaction, the reaction mixture was poured into ice water, and the deposited crystals were collected by filtration and dried. Then, the crystals were purified by short silica gel column chromatography.

### (15-1-3) Synthesis of 2-amino-5-acetylamino-3-hydroxypyridine

In an amount of 1 g of 5-acetylamino-3-benzyloxy-2-nitropyridine was added with 50 mL of methanol and subjected to a hydrogenation reaction at 50°C using 5% palladium activated carbon as a catalyst in an autoclave. After the reaction mixture was left overnight, the catalyst was separated by filtration, and the solvent was evaporated under reduced pressure. The product was used for the following reaction without further purification.

### (15-1-4) Synthesis of 5-acetylamino-2-mercaptooxazolo[4,5-b]pyridine

The product of Synthesis Example (15-1-3) was added with 2 g of potassium xanthate and 50 mL of ethanol and refluxed by heating for 15 hours. The reaction mixture was cooled, then the solvent was evaporated, and the residue was added with water to dissolve the solid. Then, acetic acid was added, and crystals deposited. The crystals were collected by filtration under reduced pressure, washed with water and dried to obtain the desired substance.

### (15-2) Synthesis of 5-acetylamino-2-methylthiooxazolo[4,5-b]pyridine

The desired substance was synthesized according to the method of Synthesis Example (5-2).

### (15-3) Synthesis of Dye (D-35)

Dye (D-35) was obtained by using the compounds synthesized in Synthesis Examples (15-1-4) and (15-2) as the starting materials according to the method of Synthesis Example 1.

### [Synthesis Example 16] Synthesis of Dye (D-37)

### (16-1) Synthesis of 5-bromo-2,3,3-trimethyl-3H-pyrrolo[2,3-b]pyridine

### (16-1-1) Synthesis of 5-bromo-2-hydrazinopyridine

In an amount of 125 g of 2,5-dibromopyridine, 350 mL of ethanol and 250 mL of hydrazine hydrate were mixed and refluxed by heating for 30 hours. The ethanol was evaporated under reduced pressure, and the residue was added with water. The deposited crystals were collected by filtration, washed with water and dried to obtain 77 g of 5-bromo-2-hydrazinopyridine. Yield: 77.6%.

### (16-1-2) Synthesis of 5-bromo-2,3,3-trimethyl-3H-pyrrolo[2,3-b]pyridine

In an amount of 76 g of 5-bromo-2-hydrazinopyridine and 100 mL of 3-methyl-2-butanone were mixed and allowed to react at 100°C for 30 minutes. After excess 3-methyl-2-butanone was evaporated, the residue was added with 100 mL of 1,4-butanediol and refluxed for 5 hours by heating to 240°C.

The reaction mixture was cooled and subjected to silica gel column chromatography without treatment, and each fraction was analyzed by NMR. The fractions containing the desired substance were collected, and the solvent was evaporated. The residue was crystallized from hexane/ethyl acetate to obtain the desired substance as light brown crystals. Yield: 8.5 g, 8.8%.

### (16-2) Synthesis of 5-phenyl-2,3,3-trimethyl-3H-pyrrolo[2,3-b]pyridine

In an amount of 0.5 g of the 5-bromo-2,3,3-trimethyl-3H-pyrrolo[2,3-b]pyridine synthesized in Synthesis Example (16-1-2), 0.38 g of phenylboronic acid and 7 mL of dimethylformamide were mixed and added with 0.072 g of tetrakis(triphenylphosphine)-palladium and 0.9 g of potassium carbonate under a nitrogen flow. The reaction mixture was stirred and heated to 100°, and a reaction was allowed for 4 hours. After completion of the reaction, the reaction mixture was extracted with chloroform, and the concentrate was purified by silica gel column chromatography. Chloroform and methanol were used as eluents, and the desired substance was eluted with chloroform/methanol = 4/1. The fractions containing the desired substance were collected and concentrated to obtain the desired substance as light yellow solid. Yield: 0.45 g, 91%.

### (16-3) Synthesis of 7-(2-methoxyethoxymethyl)-5-phenyl-2,3,3-trimethyl-3H-pyrrolo[2,3-b]pyridinium chloride

In an amount of 0.2 g of the 5-phenyl-2,3,3-trimethyl-3H-pyrrolo[2,3-b]pyridine synthesized in Synthesis Example (16-2) and 1 g of 2-methoxyethoxymethyl chloride were mixed and allowed to react at 100°C for 1 hour. The reaction mixture was cooled and then added with ethyl acetate, and the components dissolved in the ethyl acetate were removed by decantation. After this procedure was repeated 3 times, the residue was used for the following reaction without further purification.

### (16-4) Synthesis of 6-methoxy-4-methyl-1-(3-trimethylammoniopropyl)quinolinium dibromide

In an amount of 3 g of (3-bromopropyl)trimethylammonium bromide (Aldrich Co.) and 5 g of 6-methoxy-4-methylquinoline (Tokyo Kasei Kogyo Co., Ltd.) were mixed and allowed to react at 120°C for 3 hours. The reaction mixture was cooled and then added with ethyl acetate, and the substances dissolved in the ethyl acetate were removed by decantation. After this procedure was repeated 3 times, the residue was used for the following reaction without further purification.

### (16-5) Synthesis of 4-(2-(N-acetyl-N-phenylamino)vinyl)-6-methoxy-1-(3-trimethylammoniopropyl)quinolinium dibromide

The quinolinium synthesized in (16-4) was added with 1.5 g of 1,3-diphenylformamidine, mixed with 5 mL of acetic anhydride and allowed to react at 120°C for 1 hour. The reaction mixture was cooled and then added with ethyl acetate, and the substances dissolved in the ethyl acetate were removed by decantation. After this procedure was repeated twice, the residue was used for the following reaction without further purification.

### (16-6) Synthesis of Dye (D-37)

The compound synthesized in Synthesis Example (16-5) was added with 5 mL of dimethylformamide and added with 1 mL of acetic anhydride. Further, the mixture was added with the compound synthesized in Synthesis Example (16-3) and made into a uniform solution at room temperature. The solution was added with 0.3 mL of triethylamine at room temperature, and a dye was produced. The dye was purified by column chromatography using Sephadex LH-20 (eluent: methanol). Then, a solution of the dye in methanol was added with a catalytic amount of pyridinium p-toluenesulfonic acid salt and left for two days at room temperature. The solution was purified again by column chromatography using Sephadex LH-20 to obtain Dye (D-37).

### [Synthesis Example 17] Synthesis of Dye (D-38)

### (17-1) Synthesis of 2-mercaptooxazolo[4,5-c]isoquinoline

### (17-1-1) Synthesis of 4-hydroxy-3-nitroisoquinoline

In an amount of 10 g of 4-hydroxyisoquinoline was dissolved in 80 g of concentrated sulfuric acid and stirred at 2°C. The solution was slowly added dropwise with 2.7 mL of fuming nitric acid, while the solution was kept at 10°C or lower. The solution was left stand to allow a reaction at room temperature for 2 hours and then poured into 1 kg of ice water, and the deposited crystals were collected by filtration.

### (17-1-2) Synthesis of 2-mercaptooxazolo[4,5-c]isoquinoline

In an amount of 4 g of 4-hydroxy-3-nitroisoquinoline was added with ethyl acetate and water, further added with an excess amount of sodium hydrosulfite and refluxed by heating for 2 hours. The organic phase was separated and washed with water, and then the solvent was evaporated. The residue was added with ethanol and 8 g of potassium xanthate and refluxed by heating for 5 hours. After the reaction mixture was cooled, the ethanol was evaporated. The residue was dissolved in 20 mL of water and added portionwise with acetic acid, and crystals deposited. The crystals were collected by filtration, washed with water and dried to obtain the desired substance.

### (17-2) Synthesis of 2-methylthiooxazolo[4,5-c]isoquinoline

In an amount of 2 g of the starting material thiol was added to 15 mL of dimethylformamide and added with 2 mL of triethylamine to dissolve the starting material. Then, the solution was added with 1.5 mL of methyl iodide, and a reaction was allowed at room temperature for 1 hour. The reaction mixture was added with 50 mL of water, and crystals deposited. The crystals were collected by filtration, washed with water and dried to obtain the desired substance.

### (17-3) Synthesis of Dye (D-38)

Dye (D-38) was synthesized by using the compounds synthesized in Synthesis Example (17-2) and Synthesis Example (14-2) as the starting materials according to the method described in Synthesis Example 3.

### [Test Example 1] Detection of multi-stranded nucleic acid in solution system (Preparation of sample nucleic acid solution)

Fibrous deoxyribonucleic acid sodium salt derived from salmon testis (Wako Pure Chemical Industries, Ltd.) as a double-stranded nucleic acid was dissolved in a phosphate buffer at pH 6.5 to prepare a 100 µg/mL aqueous solution of the nucleic acid. The solution was further heated to 100°C for 30 minutes and rapidly cooled with ice water to prepare an aqueous solution of single-stranded deoxyribonucleic acid.

### (Preparation of solutions of compounds for detection)

Each of the compounds of the present invention shown in Table 1 was dissolved in a phosphate buffer at pH 6.5 so that the solution had absorbance of 1.0 at 320 nm to obtain a solution. For comparison, PicoGreen (Molecular Probes, Inc.) was dissolved in the phosphate buffer at pH 6.5 to prepare a solution having an optical density of 0.5 in the visible region.

### (Measurement of fluorescence intensity)

A solution was prepared by mixing the double-stranded nucleic acid or single-stranded nucleic acid solution, the solution of compound for detection and a dilution buffer in a volume ratio of 1:1:8, and fluorescence intensity of the solution was measured by using a fluorometer. The results are shown in Table 1. As for the fluorescence intensity, fluorescence excitation spectrum was measured for each sample, and fluorescence intensity obtained by excitation at wavelength that gave maximum intensity was indicated in the table. From these results, it can be understood that the ranges of the excitation and fluorescence emission wavelengths can be broadened in the compounds of the present invention, and suitability for wide range of light sources was verified. Further, it was also found that the compounds of the present invention had strong fluorescence intensity and were also excellent in selectivity for a double-stranded nucleic acid.

**Table 1**

| Compound for detection | Excitation/ Emission (nm) | Fluorescence intensity for double-stranded nucleic acid | Fluorescence intensity for single-stranded nucleic acid |
|---|---|---|---|
| D-2 (Invention) | 529/543 | 12 | 4 |
| D-6 (Invention) | 556/570 | 2 | 0.5 |
| D-14 (Invention) | 528/543 | 14 | 5 |
| D-15 (Invention) | 528/543 | 12 | 5 |
| D-16 (Invention) | 558/572 | 6 | 2 |
| D-19 (Invention) | 557/570 | 24 | 10 |
| D-41 (Invention) | 560/575 | 27 | 11 |
| PicoGreen (Comparative) | 501/523 | 23 | 9 |

| | | | |
|---|---|---|---|
| Fluorometer: RF-5300PC (Shimadzu), Band width: 3, Sensitivity: Low | | | |

### [Test Example 2] Detection of multi-stranded nucleic acid in solution system (Preparation of sample nucleic acid solution)

Fibrous deoxyribonucleic acid sodium salt derived from salmon testis (Wako Pure Chemical Industries, Ltd.) as a double-stranded nucleic acid was dissolved in a 20 mM citrate/disodium phosphate buffer at pH 6.0 to prepare a 100 µg/mL aqueous solution of the nucleic acid. The solution was further heated to 100°C for 30 minutes and rapidly cooled with ice water to prepare an aqueous solution of single-stranded deoxyribonucleic acid.

### (Preparation of solutions of compounds for detection)

Each of the compounds of the present invention shown in Table 2 was dissolved in a 20 mM citrate/disodium phosphate buffer at pH 4.0 to obtain a 5 x 10⁻⁵ mol/L solution. The solution was substantially colorless and non-fluorescent for all the compounds. For comparison, PicoGreen (Molecular Probes, Inc.) was dissolved in the 20 mM citrate/disodium phosphate buffer at pH 4.0 to prepare a solution having an optical density of 0.5 in the visible region.

### (Measurement of fluorescence intensity)

A solution was prepared by mixing a sample nucleic acid solution, a solution of compound for detection and a dilution buffer in a volume ratio of 1:1:8, and fluorescence intensity of the solution was measured by using a fluorometer. The results are shown in Table 2. As for the fluorescence intensity, fluorescence excitation spectrum was measured for each sample, and fluorescence intensity obtained by excitation at wavelength that gave maximum intensity was indicated in the table.

**Table 2**

| Compound for detection | pH of buffer | Fluorescence intensity for double-stranded nucleic acid (Absorbance) | Fluorescence intensity for single-stranded nucleic acid (Absorbance) |
|---|---|---|---|
| D-2 (Invention) | 4.0 | 280 (0.01) | 60 (0.00) |
| D-19 (Invention) | 4.0 | 466 (0.01) | 31(0.00) |
| D-43 (Invention) | 4.0 | 385 (0.01) | 24 (0.00) |
| PicoGreen (Comparative) | 4.0 | 434 (0.04) | 193 (0.05) |

| | | | |
|---|---|---|---|
| Fluorometer: RF-5300PC (Shimadzu), Band width: 3, Sensitivity: High | | | |

From the results shown in Table 2, it can be understood that, when a double-stranded nucleic acid existed, high fluorescence efficiency (fluorescence intensity/absorbance) was attained in the method of the present invention. Further, the fluorescence intensity was markedly increased in the presence of the double-stranded nucleic acid, compared with the single-stranded nucleic acid, which revealed that the method of the present invention is an extremely superior method for detecting a double-stranded nucleic acid.

### [Example 3] Detection of multi-stranded nucleic acid on solid phase carrier

### (1) Preparation of DNA fragment-immobilized slide

Slide glass (25 mm x 75 mm) was immersed in a 2 weight % solution of aminopropylethoxysilane (Shin-Etsu Chemical Co., Ltd.) in ethanol for 10 minutes, taken out from the solution, washed with ethanol and dried at 110°C for 10 minutes to prepare Silane compound-coated slide (A). Then, the Silane compound-coated slide (A) was immersed in a 3 mass % solution of Compound VS-1 for 10 minutes, taken out from the solution, washed with ethanol and dried at 120°C for 15 minutes to prepare VS-1-coated slide (B).

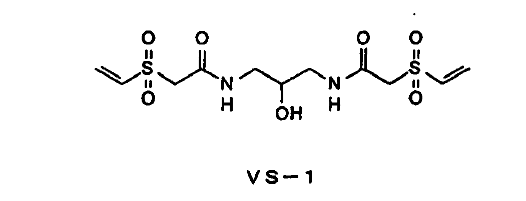

### (2) Spotting of DNA fragments and measurement of fluorescence intensity

An aqueous liquid containing a DNA fragment of which 3' end was modified with an amino group (U-1: SEQ ID NO: 1) and a complementary strand of the DNA fragment of which 3' end was similarly modified with an amino group (U-2), dispersed in 0.1 M carbonate buffer (pH 9.8, 1 x 10⁻⁵ M), was prepared and spotted on Slide (B) obtained in the above (1). Immediately after the spotting, the slide was left overnight at 60°C and humidity of 90%. This slide was successively washed twice with a mixed solution of 0.1 mass % SDS (sodium dodecylsulfate) and 2 x SSC (2 x SSC: a solution obtained by diluting a stock solution of SSC 2-fold, SSC: standard saline citrate buffer) and once with a 0.2 x SSC aqueous solution. Then, the slide after the aforementioned washing was immersed in a 0.1 M glycine aqueous solution (pH 10) for 1 hour and 30 minutes, washed with distilled water and dried at room temperature to obtain Slide (C) on which the DNA fragments were immobilized.

60-mer DNA (U-3) having a sequence complementary to the aforementioned DNA sequence (U-1) was dispersed in a hybridization solution (mixed solution of 4 x SSC and 10 mass % SDS, 20 µL), and applied to Slide (C) obtained above. After the surface of Slide (C) was protected with cover glass for microscope, the slide was incubated in a moisture chamber at 60°C for 10 hours. Then, this slide was washed with a mixed solution of 0.1 mass % SDS and 2 x SSC for 10 seconds, centrifuged at 600 rpm for 20 seconds and dried at room temperature to obtain Slide (D). Further, for comparison, a sample was prepared in the same manner by using a 60-mer DNA having the same sequence as (U-3), of which 5' end was labeled with Cy3, to obtain Slide (E). Fluorescent intensity of each slide glass was measured by a fluorescence scanning apparatus. Excitation was attained at a wavelength of 532 nm, and the detection was performed by using a band pass filter showing the maximum pass at 570 nm.

**Table 3**

| Sample | Fluorescence intensity of U-1 spot | Fluorescence intensity of U-2 spot |
|---|---|---|
| Slide (D) | 244 | 222 |
| Slide(E) (Cy3-labeled) | 23080 | 210 |

The same sample as Slide (D) was prepared, and 40 µL of a 2 x 10⁻⁵ mol/L solution of each compound in 20 mM citrate/disodium phosphate buffer at pH 5 was spread on Slide (D) by the method of the present invention. The solution was substantially colorless and non-fluorescent for all the compounds. After the surface of the slide was protected with cover glass for microscope, fluorescent intensity was measured by a fluorescence scanning apparatus in the same manner as described above. The results are shown in Table 4. From these results, it is clearly understood that, according to the method of the present invention, a multi-stranded nucleic acid can be detected without labeling.

**Table 4**

| Sample | Fluorescence intensity of U-1 spot | Fluorescence intensity of U-2 spot |
|---|---|---|
| D-2 | 12550 | 2655 |
| D-6 | 3090 | 339 |
| D-14 | 14113 | 1888 |
| D-16 | 9076 | 370 |
| D-19 | 27310 | 395 |
| D-30 | 22503 | 535 |
| D-34 | 26822 | 312 |

The same experiment was performed by using each of the nucleic acid of SEQ ID NO: 2 (80-mer), the nucleic acid of SEQ ID NO: 3 (80-mer) and the nucleic acid of SEQ ID NO: 4 (100-mer). As a result, substantially the same results as those mentioned above were successfully obtained, and thus it was verified that a multi-stranded nucleic acid was detectable without labeling.

### [Example 4] Detection of DNA/RNA hybrid

The same experiment as in Example 3 was performed except that 40-mer oligodeoxy-A was immobilized on the slide glass surface instead of the 60-mer DNA and oligo-U was used as the complementary sequence. As a result, spots in which hybridization occurred were detected, and thus it was verified that RNA was also detectable without labeling.

## Claims

1. A method for optical measurement of a multi-stranded nucleic acid which comprises the step of bringing a compound into contact with a multi-stranded nucleic acid wherein said compound is capable of interacting with the multi-stranded nucleic acid, wherein the compound has the following properties:
(a) the compound can exist in a substantially colorless and non-fluorescent state under at least one condition in an aqueous solution in the absence of the multi-stranded nucleic acid, and
(b) when the multi-stranded nucleic acid is allowed to exist in the condition defined in the above (a), the compound changes to a substantially colored state based on an interaction with the multi-stranded nucleic acid and substantially expresses fluorescent property based on said interaction wherein the compound capable of interacting with the multi-stranded nucleic acid is selected from the group consisting of compounds represented by the following formula (V): 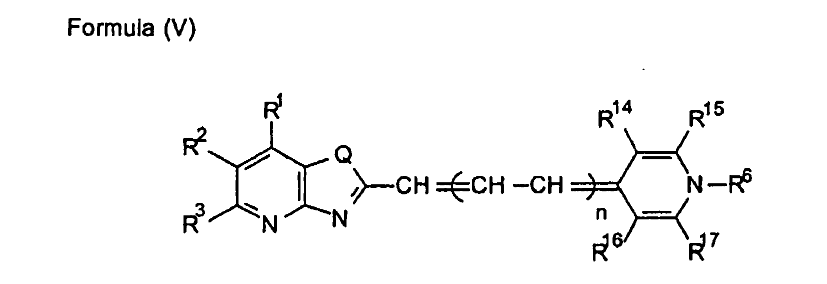 wherein R¹, R² and R³ each independently represent hydrogen atom or a substituent, selected from a halogen atom, an alkyl group, an alkenyl group, an alkynyl group, an aryl group, an amino group, hydroxyl group, an alkoxyl group, an aryloxy group, an alkylthio group, an arylthio group, an acylamino group, a sulfonylamino group, a heterocyclic group, cyano group, a sulfonyl group, a sulfinyl group, nitro group, sulfo group, carboxyl group, an alkoxycarbonyl group, an aryloxycarbonyl group, a carbamoyl group and a sulfamoyl group, wherein these substituents may be further substituted such that the total atomic number is from 1 to 50, and R¹ and R², and R² and R³ may bind to each other to form a ring; Q represents oxygen atom, sulfur atom, -N(R²⁴)- or -C(R²⁴)(R²⁵)- (R²⁴ and R²⁵ each independently represent an alkyl group, an aryl group or a heterocyclic group, and R²⁴ and R²⁵ may bind to each other to form a 3- to 8-membered ring), n represents an integer of 0, 1, 2, or 3; R⁶ represents an alkyl group, an aryl group, or a heterocyclic group, and these substituents may be further substituted such that the total atomic number is from 4 to 50 and it may bind to R¹⁵ or R¹⁷ to form a ring; R¹⁴, R¹⁵, R¹⁶, and R¹⁷ each represent hydrogen atom or a substituent selected from the group consisting of a halogen atom, an alkyl group, an alkenyl group, an alkynyl group, an aryl group, an amino group, hydroxyl group, an alkoxyl group, an aryloxy group, an alkylthio group, an arylthio group, an acylamino group, a sulfonylamino group, a heterocyclic group, cyano group, a sulfonyl group, a sulfinyl group, nitro group, sulfo group, carboxyl group, an alkoxycarbonyl group, an aryloxycarbonyl group, a carbamoyl group, a sulfamoyl group, an acyl group and a mercapto group, and R¹⁴ and R¹⁵ and R¹⁶ and R¹⁷ may bind to each other to form a 5- to 8-membered ring, and wherein the substituents R¹⁴, R¹⁵, R¹⁶ and R¹⁷ may be further substituted, and wherein the atomic number of each of R¹⁴, R¹⁵, R¹⁶ and Tr¹⁷ is from 1 to 50.

2. A method for optical measurement of multi-stranded nucleic acid which comprises the step of bringing a compound into contact with a multi-stranded nucleic acid wherein the compound is capable of interacting with the multi-stranded nucleic acid, wherein the compound has the following properties:
(c) the compound is in a non-protonated state and is substantially non-fluorescent in an aqueous solution in the absence of the multi-stranded nucleic acid,
(d) the compound can exist in a substantially colorless and substantially non-fluorescent state in a protonated state in an aqueous solution under at least one condition in the absence of the multi-stranded nucleic acid, and
(e) when the multi-stranded nucleic acid is allowed to exist in the condition defined in the above (d), the compound changes to a substantially colored state based on an interaction with the multi-stranded nucleic acid and substantially expresses fluorescent property based on said interaction wherein the compound capable of interacting with the multi-stranded nucleic acid is selected from the group consisting of compounds represented by the following formula (V): 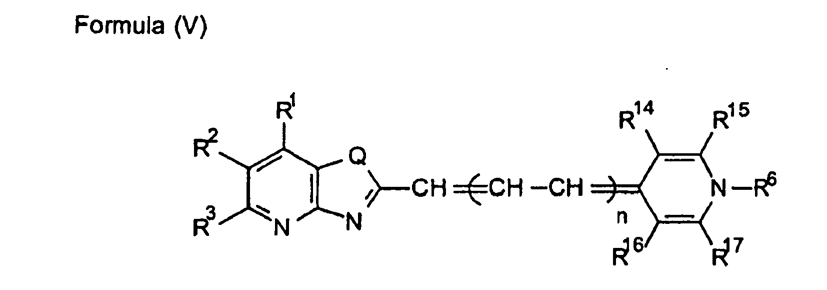
wherein R¹, R² and R³ each independently represent hydrogen atom or a substituent selected from a halogen atom, an alkyl group, an alkenyl group, an alkynyl group, an aryl group, an amino group, hydroxyl group, an alkoxyl group, an aryloxy group, an alkylthio group, an arylthio group, an acylamino group, a sulfonylamino group, a heterocyclic group, cyano group, a sulfonyl group, a sulfinyl group, nitro group, sulfo group, carboxyl group, an alkoxycarbonyl group, an aryloxycarbonyl group, a carbamoyl group and a sulfamoyl group, wherein these substituents may be further substituted such that the total atomic number is from 1 to 50, and R¹ and R², and R² and R³ may bind to each other to form a ring; Q represents oxygen atom, sulfur atom, -N(R²⁴)- or -C(R²⁴)(R²⁵)_ (R²⁴ and R²⁵ each independently represent an alkyl group, an aryl group or a heterocyclic group, and R²⁴ and R²⁵ may bind to each other to form a 3- to 8-membered ring), n represents an integer of 0, 1, 2, or 3; R⁶ represents an alkyl group, an aryl group, or a heterocyclic group, and these substituents may be further substituted such that the total atomic number is from 4 to 50 and it may bind to R¹⁵ or R¹⁷ to form a ring; R¹⁴, R¹⁵, R¹⁶, and R¹⁷ each represent hydrogen atom or a substituent selected from the group consisting of a halogen atom, an alkyl group, an alkenyl group, an alkynyl group, an aryl group, an amino group, hydroxyl group, an alkoxyl group, an aryloxy group, an alkylthio group, an arylthio group, an acylamino group, a sulfonylamino group, a heterocyclic group, cyano group, a sulfonyl group, a sulfinyl group, nitro group, sulfo group, carboxyl group, an alkoxycarbonyl group, an aryloxycarbonyl group, a carbamoyl group, a sulfamoyl group, an acyl group and a mercapto group, and R¹⁴ and R¹⁵ and R¹⁶ and R¹⁷ may bind to each other to form a 5- to 8-membered ring, and wherein the substituents R¹⁴, R¹⁵, R¹⁶ and R¹⁷ may be further substituted, and wherein the atomic number of each of R¹⁴, R¹⁵, R¹⁶ and R¹⁷ is from 1 to 50.

3. A method for optical measurement of a multi-stranded nucleic acid comprising:
(1) the step of bringing Compound (B) of the following general formula into contact with a multi-stranded nucleic acid, and
(2) the step of measuring fluorescence of Compound (C) produced by an interaction of Compound (B) and the multi-stranded nucleic acid: 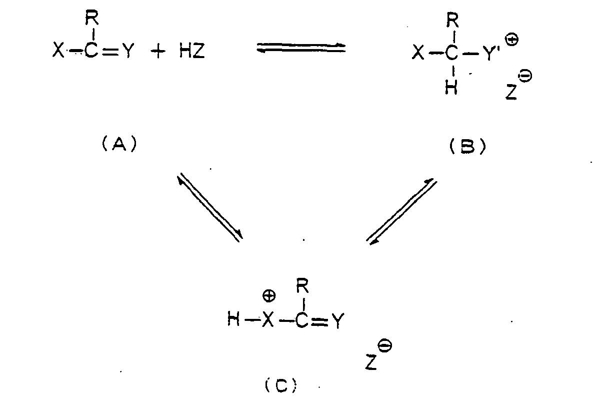
wherein the two way arrows represent chemical equilibrium;
Compound (A) represents a substantially non-fluorescent compound, Compound (B) represents a substantially non-fluorescent compound that does not have an absorption maximum in the range of from 400 to 1000 nm under at least one condition:
Compound (C) represents a compound which is produced by an interaction of Compound (B) and the multi-stranded nucleic acid under the condition mentioned above, and has an absorption maximum in the range of from 400 to 1000 nm and substantially expresses fluorescent property based on the interaction:
X represents a group of atoms required to form a dye molecule by bonding to -C(R)=Y: Y represents a group of atoms required to form a dye molecule by bonding to X-C(R)=; R represents hydrogen atom or a substituent and may bind to X and/or Y to form a ring; Y' represents a residue remaining as a result of occurrence of an electron transfer so that the double bond of C=Y in Compound (A) is protonated; and Z represents a conjugate base of acid HZ and may bind to X, Y (or Y') or R, wherein Compound (A) is selected from the group consisting of compounds represented by the formula (V) according to claim 1.

4. The method according to claim 1, which comprise the step of allowing the interaction with the multi-stranded nucleic acid in a state of solution.

5. The method according to claim 1, which comprise the step of allowing the interaction with the multi-stranded nucleic acid immobilized on a solid phase carrier.

6. A compound represented by the formula (V) according to claim 1 or an acid addition salt or a base addition salt thereof.

7. The compound according to claim 6, wherein Q is oxygen atom or sulfur atom.

## Patentansprüche

1. Verfahren zur optischen Messung einer vielsträngigen Nukleinsäure, umfassend das Kontaktieren einer Verbindung mit einer vielsträngigen Nukleinsäure, worin die Verbindung in der Lage ist, mit der vielsträngigen Nukleinsäure zu interagieren, worin die Verbindung die folgenden Eigenschaften aufweist:
(a) die Verbindung kann in einem im wesentlichen farblosen und nicht-fluoreszierenden Zustand unter zumindest einer Bedingung in einer wäßrigen Lösung in Abwesenheit der vielsträngigen Nukleinsäure existieren und
(b) wenn die vielsträngige Nukleinsäure in dem Zustand existieren kann, wie oben unter (a) definiert, ändert sich die Verbindung in einen im wesentlichen gefärbten Zustand, basierend auf der Wechselwirkung mit der vielsträngigen Nukleinsäure, und entwickelt im wesentlichen eine Fluoreszenzeigenschaft auf der Basis der Interaktion, worin die Verbindung, die mit der vielsträngigen Nukleinsäure interagieren kann, ausgewählt ist aus der Gruppe bestehend aus Verbindungen mit der folgenden Formel (V): 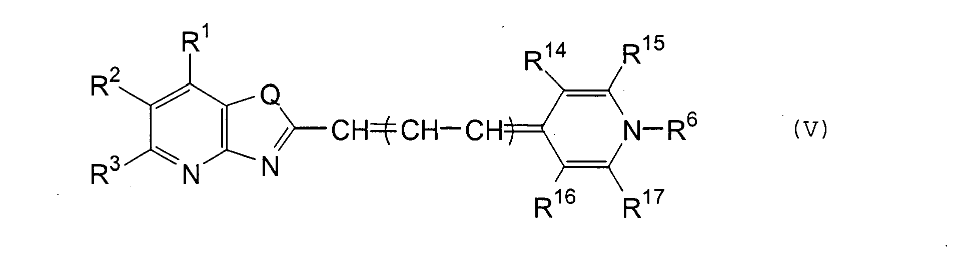 worin R¹, R² und R³ jeweils unabhängig ein Wasserstoffatom oder einen Substituenten bedeuten, ausgewählt aus einem Halogenatom, einer Alkyl-, Alkenyl-, Alkinyl-, Aryl-, Amino-, Hydroxyl-, Alkoxyl-, Aryloxy-, Alkylthio-, Arylthio-, Acylamino-, Sulfonylamino-, heterocyclischen, Cyano-, Sulfonyl-, Sulfinyl-, Nitro-, Sulfo-, Carboxyl-, Alkoxycarbonyl-, Aryloxycarbonyl-, Carbamoyl- und Sulfamoylgruppe, worin diese Substituenten weiterhin so substituiert sein können, daß die gesamte Atomzahl von 1 bis 50 ist, und R¹ und R² und R² und R³ aneinander binden können unter Bildung eines Rings; Q ein Sauerstoffatom, Schwefelatom, -N(R²⁴)- oder -C(R²⁴)(R²⁵)- ist (R²⁴ und R²⁵ bedeuten jeweils unabhängig eine Alkylgruppe, Arylgruppe oder heterocyclische Gruppe und R²⁴ und R²⁵ können aneinander zur Bildung eines 3- bis 8-gliedrigen Rings binden), n eine ganze Zahl von 0, 1, 2 oder 3 ist, R⁶ eine Alkylgruppe, Arylgruppe oder heterocyclische Gruppe ist, und diese Substituenten weiterhin so substituiert sein können, daß die gesamte Atomzahl von 4 bis 50 ist, und an R¹⁵ oder R¹⁷ zur Bildung eines Rings binden können; R¹⁴, R¹⁵, R¹⁶ und R¹⁷ jeweils Wasserstoffatom oder ein Substituent sind, ausgewählt aus der Gruppe bestehend aus einem Halogenatom, einer Alkyl-, Alkenyl-, Alkinyl-, Aryl-, Amino-, Hydroxyl-, Alkoxyl-, Aryloxy-, Alkylthio-, Arylthio-, Acylamino-, Sulfonylamino-, heterocyclischen, Cyano-, Sulfonyl-, Sulfinyl-, Nitro-, Sulfo-, Carboxyl-, Alkoxycarbonyl-, Aryloxycarbonyl-, Carbamoyl-, Sulfamoyl-, Acyl- und Mercaptogruppe, und R¹⁴ und R¹⁵ und R¹⁶ und R¹⁷ aneinander zur Bildung eines 5- bis 8-gliedrigen Rings binden können, und worin die Substituenten R¹⁴, R¹⁵, R¹⁶ und R¹⁷ weiterhin substituiert sein können und die Atomzahl von R¹⁴, R¹⁵, R¹⁶ und R¹⁷jeweils von 1 bis 50 ist.

2. Verfahren zur optischen Messung der vielsträngigen Nukleinsäure, umfassend das Kontaktieren einer Verbindung mit einer vielsträngigen Nukleinsäure, worin die Verbindung mit der vielsträngigen Nukleinsäure interagieren kann, worin die Verbindung die folgenden Eigenschaften aufweist:
(c) die Verbindung ist in einem nicht-protonierten Zustand und im wesentlichen nicht-fluoreszent in einer wäßrigen Lösung in der Abwesenheit der vielsträngigen Nukleinsäure,
(d) die Verbindung kann in einem im wesentlichen farblosen und im wesentlichen nicht-fluoreszenten Zustand in einem protonierten Zustand in einer wäßrigen Lösung unter zumindest einer Bedingung in der Abwesenheit der vielsträngigen Nukleinsäure existieren, und
(e) wenn die vielsträngige Nukleinsäure in dem Zustand existieren kann, der oben unter (d) definiert ist, ändert sich die Verbindung in einen im wesentlichen gefärbten Zustand auf der Basis einer Interaktion mit der vielsträngigen Nukleinsäure und entfaltet im wesentlichen eine Fluoreszenzeigenschaft auf der Basis der Interaktion, worin die Verbindung, die mit der vielsträngigen Nukleinsäure interagieren kann, ausgewählt ist aus der Gruppe bestehend aus Verbindungen mit der folgenden Formel (V): 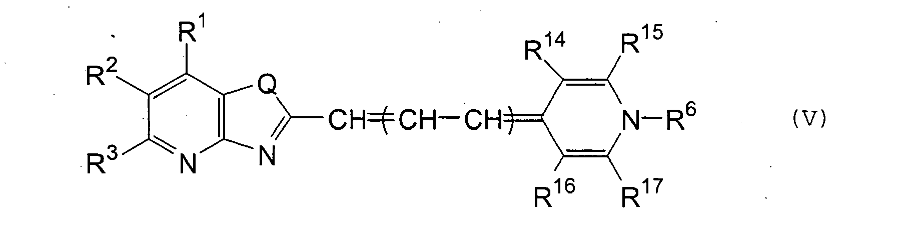
worin R¹, R² und R³ jeweils unabhängig ein Wasserstoffatom oder einen Substituenten bedeuten, ausgewählt aus einem Halogenatom, einer Alkyl-, Alkenyl-, Alkinyl-, Aryl-, Amino-, Hydroxyl-Alkoxyl-, Aryloxy-, Alkylthio-, Arylthio-, Acylamino-, Sulfonylamino-, heterocyclischen, Cyano-, Sulfonyl-, Sulfinyl-, Nitro-, Sulfo-, Carboxyl-, Alkoxycarbonyl-, Aryloxycarbonyl-, Carbamoyl- und Sulfamoylgruppe, worin diese Substituenten weiterhin so substituiert sein können, daß die gesamte Atomzahl von 1 bis 50 ist, und R¹ und R² und R² und R³ aneinander binden können unter Bildung eines Rings; Q ein Sauerstoffatom, Schwefelatom, -N(R²⁴)- oder -C(R²⁴)(R²⁵)- ist (R²⁴ und R²⁵ bedeuten jeweils unabhängig eine Alkylgruppe, Arylgruppe oder heterocyclische Gruppe und R²⁴ und R²⁵ können aneinander zur Bildung eines 3- bis 8-gliedrigen Rings binden), n eine ganze Zahl von 0, 1, 2 oder 3 ist, R⁶ eine Alkylgruppe, Arylgruppe oder heterocyclische Gruppe ist, und diese Substituenten weiterhin so substituiert sein können, daß die gesamte Atomzahl von 4 bis 50 ist, und an R¹⁵ oder R¹⁷ zur Bildung eines Rings binden können; R¹⁴, R¹⁵, R¹⁶ und R¹⁷ jeweils Wasserstoffatom oder ein Substituent sind, ausgewählt aus der Gruppe bestehend aus einem Halogenatom, einer Alkyl-, Alkenyl-, Alkinyl-, Aryl-, Amino-, Hydroxyl-, Alkoxyl-, Aryloxy-, Alkylthio-, Arylthio-, Acylamino-, Sulfonylamino-, heterocyclischen, Cyano-, Sulfonyl-, Sulfinyl-, Nitro-, Sulfo-, Carboxyl-, Alkoxycarbonyl-, Aryloxycarbonyl-Carbamoyl-, Sulfamoyl-, Acyl- und Mercaptogruppe, und R¹⁴ und R¹⁵ und R¹⁶ und R¹⁷ aneinander zur Bildung eines 5- bis 8-gliedrigen Rings binden können, und worin die Substituenten R^{14,} R¹⁵, R¹⁶ und R¹⁷ weiterhin substituiert sein können und die Atomzahl von R¹⁴, R¹⁵, R¹⁶ und R¹⁷jeweils von 1 bis 50 ist.

3. Verfahren zur optischen Messung einer vielsträngigen Nukleinsäure, umfassend:
(1) den Schritt des Kontaktierens der Verbindung (B) mit der folgenden allgemeinen Formel mit einer vielsträngigen Nukleinsäure und
(2) den Schritt der Messung der Fluoreszenz der Verbindung (C), erzeugt durch Interaktion der Verbindung (B) und der vielsträngigen Nukleinsäure: 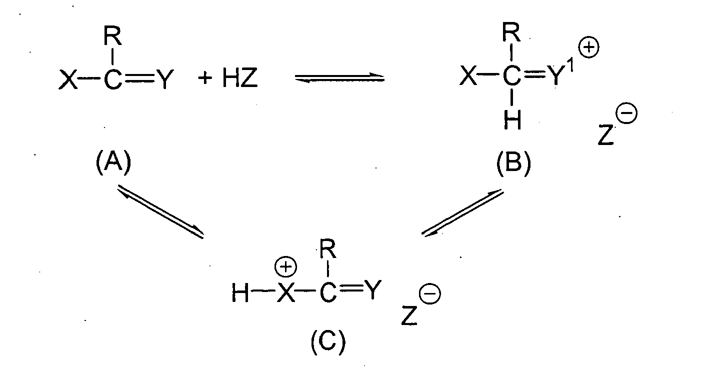
worin die jeweils zwei Pfeile das chemische Gleichgewicht darstellen;
die Verbindung (A) eine im wesentlichen nichtfluoreszente Verbindung bedeutet,
die Verbindung (B) eine im wesentlichen nichtfluoreszente Verbindung bedeutet, die kein Absorptionsmaximum im Bereich von 400 bis 1.000 nm unter zumindest einer Bedingung aufweist,
die Verbindung (C) eine Verbindung ist, die durch eine Interaktion der Verbindung (B) und der vielsträngigen Nukleinsäure unter der oben erwähnten Bedingung erzeugt ist und ein Absorptionsmaximum im Bereich von 400 bis 1.000 nm aufweist und im wesentlichen eine fluoreszente Eigenschaft auf der Basis der Wechselwirkung entfaltet;
X eine Gruppe von Atomen ist, die erforderlich ist zur Bildung eines Farbstoffmoleküls durch Bindung an -C(R)=Y, wobei Y eine Gruppe von Atomen bedeutet, die erforderlich ist zur Bildung eines Farbstoffmoleküls durch Bindung an X-C(R)=; R ein Wasserstoffatom oder ein Substituent ist und an X und/oder Y zur Bildung eines Rings binden kann; Y' ein Rest ist, der als Ergebnis des Auftretens eines Elektronentransfers verbleibt, so daß die Doppelbindung von C=Y in der Verbindung (A) protoniert ist; und Z eine Konjugatbase der Säure HZ ist und an X, Y (oder Y') oder R binden kann, worin die Verbindung (A) ausgewählt ist aus der Gruppe bestehend aus Verbindungen mit der Formel (V) nach Anspruch 1.

4. Verfahren nach Anspruch 1, umfassend das Ermöglichen der Interaktion mit der vielsträngigen Nukleinsäure in einem Lösungszustand.

5. Verfahren nach Anspruch 1, umfassend den Schritt der Ermöglichung der Interaktion mit der vielsträngigen Nukleinsäure, die auf einem Festphasenträger immobilisiert ist.

6. Verbindung mit der Formel (V) nach Anspruch 1 oder ein Säureadditionssalz oder ein Basenadditionssalz davon.

7. Verbindung nach Anspruch 6, worin Q Sauerstoff- oder Schwefelatom ist.

## Revendications

1. Procédé de mesure optique d'un acide nucléique multibrin qui comprend l'étape consistant à mettre un composé en contact avec un acide nucléique multibrin, dans lequel ledit composé est capable d'interaction avec l'acide nucléique multibrin, le composé ayant les propriétés suivantes:
(a) le composé peut exister à l'état essentiellement incolore et non fluorescent à au moins une condition, qui est en solution aqueuse en l'absence de l'acide nucléique multibrin, et
(b) quand on laisse l'acide nucléique multibrin exister dans la condition définie en (a) ci-dessus, le composé change pour passer à un état sensiblement coloré, basé sur une interaction avec l'acide nucléique multibrin et exprime sensiblement une propriété fluorescente, basée sur ladite interaction, le composé capable d'interaction avec l'acide nucléique multibrin étant choisi dans le groupe constitué des composés représentés par la formule (V) suivante : 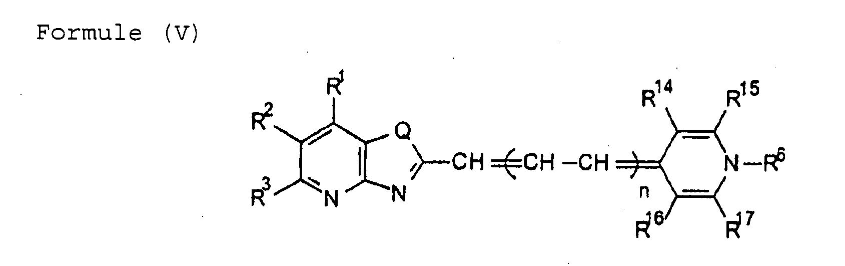 dans laquelle R¹, R² et R³ représentent chacun indépendamment un atome d'hydrogène ou un substituant choisi parmi un atome d'halogène, un groupe alkyle, un groupe alcényle, un groupe alcynyle, un groupe aryle, un groupe amino, un groupe hydroxyle, un groupe alcoxyle, un groupe aryloxy, un groupe alkylthio, un groupe arylthio, un groupe acylamino, un groupe sulfonylamino, un groupe hétérocyclique, un groupe cyano, un groupe sulfonyle, un groupe sulfinyle, un groupe nitro, un groupe sulfo, un groupe carboxyle, un groupe alcoxycarbonyle, un groupe aryloxycarbonyle, un groupe carbamoyle et un groupe sulfamoyle, ces substituants pouvant en outre être substitués de telle façon que le nombre atomique total est de 1 à 50, et R¹ et R², et R² et R³ peuvent se lier l'un à l'autre pour former un cycle ; Q représente un atome d'oxygène, un atome de soufre, -N (R²⁴) - ou -C (R²⁴) (R²⁵) - (R²⁴ et R²⁵ représentant chacun indépendamment un groupe alkyle, un groupe aryle ou un groupe hétérocyclique, et R²⁴ et R²⁵ pouvant se lier l'un à l'autre pour former un cycle de 3 à 8 chaînons), n représente un nombre entier valant 0, 1, 2, ou 3 ; R⁶ représente un groupe alkyle, un groupe aryle, ou un groupe hétérocyclique, ces substituants pouvant, en outre, être substitués de telle façon que le nombre atomique total est de 4 à 50, et il peut se lier à R¹⁵ ou R¹⁷ pour former un cycle ; R¹⁴, R¹⁵, R¹⁶, et R¹⁷ représentent chacun un atome d'hydrogène ou un substituant choisi dans le groupe constitué d'un atome d'halogène, d'un groupe alkyle, d'un groupe alcényle, d'un groupe alcynyle, d'un groupe aryle, d'un groupe amino, d'un groupe hydroxyle, d'un groupe alcoxyle, d'un groupe aryloxy, d'un groupe alkylthio, d'un groupe arylthio, d'un groupe acylamino, d'un groupe sulfonylamino, d'un groupe hétérocyclique, d'un groupe cyano, d'un groupe sulfonyle, d'un groupe sulfinyle, d'un groupe nitro, d'un groupe sulfo, d'un groupe carboxyle, d'un groupe alcoxycarbonyle, d'un groupe aryloxycarbonyle, d'un groupe carbamoyle, d'un groupe sulfamoyle, d'un .groupe acyle et d'un groupe mercapto, et R¹⁴ et R¹⁵, et R¹⁶ et R¹⁷ peuvent se lier l' un à l'autre pour former, un cycle de 5 à 8 chaînons ; les substituants R¹⁴, R¹⁵, R¹⁶ et R¹⁷ pouvant en outre être substitués, et dans laquelle le nombre atomique de chacun des R¹⁴, R¹⁵, R¹⁶ et R¹⁷ est de 1 à 50.

2. Procédé de mesure optique d'un acide nucléique multibrin qui comprend l'étape consistant à mettre un composé en contact avec un acide nucléique multibrin, dans lequel le composé est capable d'interaction avec l'acide nucléique multibrin, le composé ayant les propriétés suivantes:
(c) le composé est à l'état non protoné et est essentiellement non fluorescent en solution aqueuse en l'absence de l'acide nucléique multibrin,
(d) le composé peut exister à l'état essentiellement incolore et essentiellement non fluorescent à l'état protoné en solution aqueuse à au moins une condition, qui est l'absence de l'acide nucléique multibrin, et
(e) quand on laisse l'acide nucléique multibrin exister dans la condition définie en (d) ci-dessus, le composé change pour passer à un état sensiblement coloré, basé sur une interaction avec l'acide nucléique multibrin et exprime sensiblement une propriété fluorescente, basée sur ladite interaction, le composé capable d'interaction avec l'acide nucléique multibrin étant choisi dans le groupe constitué des composés représentés par la formule (V) suivante: 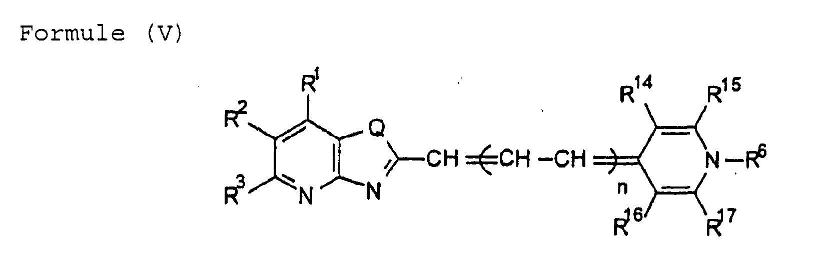
dans laquelle R¹, R² et R³ représentent chacun indépendamment un atome d'hydrogène ou un substituant choisi parmi un atome d'halogène, un groupe alkyle, un groupe alcényle, un groupe alcynyle, un groupe aryle, un groupe amino, un groupe hydroxyle, un groupe alcoxyle, un groupe aryloxy, un groupe alkylthio, un groupe arylthio, un groupe acylamino, un groupe sulfonylamino, un groupe hétérocyclique, un groupe cyano, un groupe sulfonyle, un groupe sulfinyle, un groupe nitro, un groupe sulfo, un groupe carboxyle, un groupe alcoxycarbonyle, un groupe aryloxycarbonyle, un groupe carbamoyle et un groupe sulfamoyle, ces substituants pouvant en outre être substitués de telle façon que le nombre atomique total est de 1 à 50, et R¹ et R² et R² et R³ peuvent se lier l'un à l'autre pour former un cycle ; Q représente un atome d'oxygène, un atome de soufre , -N(R²⁴) - ou -C(R²⁴) (R²⁵) - (R²⁴ et R²⁵ représentant chacun indépendamment un groupe alkyle, un groupe aryle ou un groupe hétérocyclique, et R²⁴ et R²⁵_{.} pouvant se lier l'un à l'autre pour former un cycle de 3 à 8 chaînons), n représente un nombre entier valant 0, 1, 2, ou 3 ; R⁶ représente un groupe alkyle, un groupe aryle, ou un groupe hétérocyclique, ces substituants pouvant, en outre, être substitués de telle façon que le nombre atomique total est de 4 à 50, et il peut se lier à R¹⁵ ou R¹⁷ pour former un cycle ; R¹⁴ , R¹⁵, R¹⁶, et R¹⁷ représentent chacun un atome d'hydrogène ou un substituant choisi dans le groupe constitué d'un atome d'halogène; d'un groupe alkyle, d'un groupe alcényle, d'un groupe alcynyle, d'un groupe aryle, d'un groupe amino, d'un groupe hydroxyle, d'un groupe alcoxyle, d'un groupe aryloxy, d'un groupe alkylthio, d'un groupe arylthio, d'un groupe acylamino, d'un groupe sulfonylamino, d'un groupe hétérocyclique, d'un groupe cyano, d'un groupe sulfonyle, d'un groupe sulfinyle, d'un groupe nitro, d'un groupe sulfo, d'un groupe carboxyle, d'un groupe alcoxycarbonyle, d'un groupe aryloxycarbonyle, d'un groupe carbamoyle, d'un groupe sulfamoyle, d'un groupe acyle et d'un groupe mercapto, et R¹⁴ et R¹⁵, et R¹⁶ et R¹⁷ peuvent se lier l'un à l'autre pour former un cycle de 5 à 8 chaînons; les substituants R¹⁴, R¹⁵, R¹⁶ et R¹⁷ pouvant en outre être substitués, et dans laquelle le nombre atomique de chacun des R^{14,} R¹⁵, R¹⁶ et R¹⁷ est de 1 à 50.

3. Procédé de mesure optique d'un acide nucléique multibrin comprenant :
(1) l'étape consistant à mettre le Composé (B) répondant à la formule générale suivante en contact avec un acide nucléique multibrin, et
(2) l'étape consistant à mesurer la fluorescence du Composé (C) produit par une interaction du Composé (B) et de l'acide nucléique multibrin : 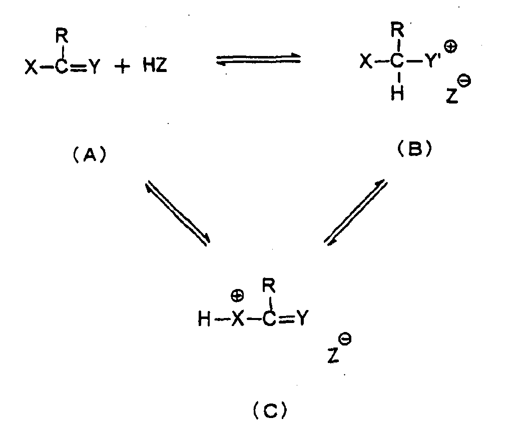
dans laquelle les flèches bidirectionnelles représentent l'équilibre chimique,
Composé (A) représente un composé essentiellement non fluorescent,
Composé (B) représente un composé essentiellement non fluorescent qui n'a pas une absorption maximale dans la plage de 400 à 1000 nm à au moins une condition,
Composé (C) représente un composé qui est produit par une interaction du Composé (B) et de l'acide nucléique multibrin à la condition mentionnée ci-dessus, et a une absorption maximale dans la plage de 400 à 1000 nm et exprime essentiellement une propriété fluorescente basée sur l'interaction ;
X représente un groupe d'atomes requis pour former une molécule colorante par liaison à -C(R)=Y ; Y représente un groupe d'atomes requis pour former une molécule colorante par liaison à X-C(R)= ; R représente un atome d'hydrogène ou un substituant et peut se lier à X et/ou Y pour former un cycle ; Y' représente un résidu subsistant à la suite de l'occurrence d'un transfert d'électrons de telle façon que la double liaison de C=Y dans le Composé (A) est protonée ; et Z représente une base conjuguée d'acide HZ et peut se lier à X, Y (ou Y') ou R, le Composé (A) étant choisi dans le groupe des composés représentés par la formule (V) selon la revendication 1.

4. Procédé selon la revendication 1, qui comprend l'étape consistant à permettre l'interaction avec l'acide nucléique multibrin à l'état de solution.

5. Procédé selon la revendication 1, qui comprend l'étape consistant à permettre l'interaction avec l'acide nucléique multibrin immobilisé sur un support en phase solide.

6. Composé représenté par la formule (V) selon la revendication 1 ou sel d'addition d'acide ou sel d'addition de base de celui-ci.

7. Composé selon la revendication 6, dans lequel Q est un atome d'oxygène ou un atome de soufre.


### SEQUENCE LISTING

<110> Fuji Photo Film Co. Ltd.
<120> Method for optically measuring multistrand nucleic acid
<130> FA2208M
<160> 4
<210> 1
<211> 60
<212> DNA
<213> Artificial
<400> 1
   GCTGCTGCTG GGCCAGTGGT TCCTCCATGT CCGGGGAGGA TCAGACACTT CAAGGTCTAG 60
<210> 2
<211> 80
<212> DNA
<213> Artificial
<400> 2
   TGGTGCAGCC TAGTGTTGGT GCTGCTGCTG GGCCAGTGGT TCCTCCATGT CCGGGGAGGA 60
   TCAGACACTT CAAGGTCTAG 80
<210> 3
<211> 80
<212> DNA
<213> Artificial
<400> 3
   TGGTGCAGCC TAGTGTTGGT GCTGCTGCTG GGCCAGTGGT TCCTCCATGT CCGGGGAGGA 60
   TCAGACACTT CAAGGTCTAG 80
<210> 4
<211> 100
<212> DNA
<213> Artificial
<400> 4
   TGGTGCAGCC TAGTGTTGGT GCTGCTGCTG GGCCAGTGGT TCCTCCATGT CCGGGGAGGA 60
   TCAGACACTT CAAGGTCTAG GCTAGACATG GCAGAGATGA 100